Europäisches Patentamt

European Patent Office

Office européen des brevets

(19)

(11) EP 0 952 228 A2

(12) **EUROPEAN PATENT APPLICATION**

(43) Date of publication:
**27.10.1999 Bulletin 1999/43**

(51) Int Cl.$^6$: **C12Q 1/68**

(21) Application number: **99107983.1**

(22) Date of filing: **24.09.1997**

(84) Designated Contracting States:
**AT BE CH DE DK ES FI FR GB GR IE IT LI LU MC NL PT SE**

(30) Priority: **24.09.1997 WOPCT/US97/17413**
**24.09.1996 US 26621 P**
**24.09.1996 US 719132**

(62) Document number(s) of the earlier application(s) in accordance with Art. 76 EPC:
**97944521.0**

(71) Applicant: **Rapigene, Inc.**
**Bothell, Washington 98021 (US)**

(72) Inventors:
- **Van Ness, Jeffrey**
  **Seattle, Washington 98125 (US)**
- **Tabone, John**
  **Bothell, Washington 98011 (US)**

(74) Representative: **Gowshall, Jonathan Vallance**
**FORRESTER & BOEHMERT**
**Franz-Joseph-Strasse 38**
**80801 München (DE)**

Remarks:
This application was filed on 22 - 04 - 1999 as a divisional application to the application mentioned under INID code 62.

(54) **Compositions and methods for enhancing hybridization specificity**

(57) Compositions and methods are provided for increasing the specificity of a probe nucleic acid for a target nucleic acid in a hybridization solution. An abasic residue, deoxyNebularine residue, or a hybotrope is used to increase specificity. A method is provided for identifying useful hybotropes, including salts, water miscible organic solvents, aprotic solvents and organic solvents, on the basis of enthalpy considerations. Hybotropic hybridization and modified oligonucleotides may be used in amplification reactions, such as PCR, sequence analysis methods, and genomic screening methods.

EP 0 952 228 A2

**Description**

TECHNICAL FIELD

The present invention relates generally to compositions and methods for hybridization of oligonucleotides, and more specifically to certain solutions and/or oligonucleotide analogues which may increase hybridization specificity.

BACKGROUND OF THE INVENTION

The detection of diseases is increasingly important in prevention and treatments. While multifactorial diseases are difficult to devise genetic tests for, more than 200 known human disorders are caused by a defect in a single gene, often a change of a single amino acid residue (Olsen, *Biotechnology: An industry comes of age,* National Academic Press, 1986). Many of these mutations result in an altered amino acid that causes a disease state.

Sensitive mutation detection techniques offer extraordinary possibilities for mutation screening. For example, analyses may be performed even before the implantation of a fertilized egg (Holding and Monk, *Lancet 3:*532, 1989). Increasingly efficient genetic tests may also enable screening for oncogenic mutations in cells exfoliated from the respiratory tract or the bladder in connection with health checkups (Sidransky et al., *Science 252:*706, 1991). Also, when an unknown gene causes a genetic disease, methods to monitor DNA sequence variants are useful to study the inheritance of disease through genetic linkage analysis. However, detecting and diagnosing mutations in individual genes poses technological and economic challenges. Several different approaches have been pursued, but none are both efficient and inexpensive enough for truly widescale application.

Mutations involving a single nucleotide can be identified in a sample by physical, chemical, or enzymatic means. Generally, methods for mutation detection may be divided into scanning techniques, which are suitable to identify previously unknown mutations, and techniques designed to detect, distinguish, or quantitate known sequence variants.

Several scanning techniques for detection of mutations have been developed on the observation that heteroduplexes of mismatched complementary DNA strands exhibit an abnormal behavior, especially when denatured. This phenomenon is exploited in denaturing and temperature gradient gel electrophoresis (DGGE and TGGE, respectively) methods. Duplexes mismatched in even a single nucleotide position can partially denature, resulting in retarded migration, when electrophoresed in an increasingly denaturing gradient gel (Myers et al., *Nature 313:*495, 1985; Abrams et al., *Genomics 7:*463, 1990; Henco et al., *Nucl. Acids Res. 18:*6733, 1990). Although mutations may be detected, no information is obtained regarding the precise location of a mutation. Mutant forms must be further isolated and subjected to DNA sequence analysis.

Alternatively, a heteroduplex of an RNA probe and a target strand may be cleaved by RNase A at a position where the two strands are not properly paired. The site of cleavage can then be determined by electrophoresis of the denatured probe. However, some mutations may escape detection because not all mismatches are efficiently cleaved by RNase A.

Mismatched bases in a duplex are also susceptible to chemical modification. Such modification can render the strands susceptible to cleavage at the site of the mismatch or cause a polymerase to stop in a subsequent extension reaction. The chemical cleavage technique allows identification of a mutation in target sequences of up to 2 kb and it provides information on the approximate location of mismatched nucleotide(s) (Cotton et al., *PNAS USA 85:*4397, 1988; Ganguly et al., *Nucl. Acids Res. 18:*3933, 1991). However, this technique is labor intensive and may not identify the precise location of the mutation.

An alternative strategy for detecting a mutation in a DNA strand is by substituting (during synthesis) one of the normal nucleotides with a modified nucleotide, thus altering the molecular weight or other physical parameter of the product. A strand with an increased or decreased number of this modified nucleotide relative to the wild-type sequence exhibits altered electrophoretic mobility (Naylor et al., *Lancet 337:*635, 1991). This technique detects the presence of a mutation, but does not provide the location.

Two other strategies visualize mutations in a DNA segment by altered gel migration. In the single-strand conformation polymorphism technique (SSCP), mutations cause denatured strands to adopt different secondary structures, thereby influencing mobility during native gel electrophoresis. Heteroduplex DNA molecules, containing internal mismatches, can also be separated from correctly matched molecules by electrophoresis (Orita, *Genomics 5:*874, 1989; Keen, *Trends Genet. 7:*5, 1991). As with the techniques discussed above, the presence of a mutation may be determined but not the location. As well, many of these techniques do not distinguish between a single and multiple mutations.

All of the above-mentioned techniques indicate the presence of a mutation in a limited segment of DNA and some of them allow approximate localization within the segment. However, sequence analysis is still required to unravel the effect of the mutation on the coding potential of the segment. Sequence analysis is a powerful tool, allowing, for example, screening for the same mutation in individuals of an affected family, monitoring disease progression in the case of malignant disease, or for detecting residual malignant cells in bone marrow before autologous transplantation. Despite these advantages, the procedure is unlikely to be adopted as a routine diagnostic method because of the high expense involved.

A large number of other techniques have been developed to analyze known sequence variants. Automation and economy are very important considerations for implementation of these types of analyses. In this regard, none of the alternative techniques discussed below combine economy and automation with the required specificity.

A number of strategies for nucleotide sequence distinction all depend on enzymes, some very costly, to identify sequence differences (Saiki, *PNAS USA 86*:6230, 1989; Zhang, *Nucl. Acids Res. 19*:3929, 1991).

For example, restriction enzymes recognize sequences of about 4-8 nucleotides. Based on an average G+C content, approximately half of the nucleotide positions in a DNA segment can be monitored with a panel of 100 restriction enzymes. As an alternative, artificial restriction enzyme recognition sequences may be created around a variable position by using partially mismatched PCR primers. With this technique, either the mutant or the wild-type sequence alone may be recognized and cleaved by a restriction enzyme after amplification (Chen et al., *Anal. Biochem. 195*:51, 1991; Levi et al., *Cancer Res. 51*:3497, 1991).

Another method exploits the property that an oligonucleotide primer that is mismatched to a target sequence at the 3' penultimate position exhibits a reduced capacity to serve as a primer in PCR. However, some 3' mismatches, notably G-T, are less inhibitory than others, thus limiting its usefulness. In attempts to improve this technique, additional mismatches are incorporated into the primer at the third position from the 3' end. This results in two mismatched positions in the three 3' nucleotides of the primer hybridizing with one allelic variant, and one mismatch in the third position in from the 3' end when the primer hybridizes to the other allelic variant (Newton et al., *Nucl. Acids Res. 17*: 2503, 1989). For this technique to be successful, it is necessary to define amplification conditions that significantly favor amplification of a l bp mismatch.

DNA polymerases have also been used to distinguish allelic sequence variants by determining which nucleotide is added to an oligonucleotide primer immediately upstream of a variable position in the target strand. Based on this approach, a ligation assay has been developed. In this method, two oligonucleotide probes hybridizing in immediate juxtaposition on a target strand are joined by a DNA ligase. Ligation is inhibited if there is a mismatch where the two oligonucleotide probes abut.

Mutations may be identified via their destabilizing effects on the hybridization of short oligonucleotide probes to a target sequence (*see* Wetmur, *Crit. Rev. Biochem. Mol. Biol. 26*:227, 1991). Generally, this technique, allele-specific oligonucleotide hybridization, involves amplification of target sequences and subsequent hybridization with short oligonucleotide probes. An amplified product can be scanned for many possible sequence variants by determining its hybridization pattern to an array of immobilized oligonucleotide probes. Many of these techniques, especially allele-specific oligonucleotide hybridization, require establishing conditions that favor the hybridization of an exact match over a mismatch. As is well known, such conditions are difficult to achieve. One approach to improving hybridization is the addition of a chaotrope.

Chaotropes decrease the melting temperature of an oligonucleotide duplex (*see* Van Ness and Chen, *Nucleic Acids Research 19*:5143, 1991). Oligonucleotide probes (12-50 mers) possess some functional properties that are not shared by long DNA probes. These parameters include different rates of duplex formation as a function of (a) the difference between the hybridization temperature and the $T_m$, (b) stringency requirements for maximal selectivity/specificity of hybridization, and (c) sequence-specific anomalous behavior.

Chaotropes are useful in DNA probe-based diagnostic assays, as they can simultaneously lyse the cells of organisms of interest, inhibit nucleases and proteases, and provide adequate hybridization stringency without chemically altering the target analyte. Chaotropic lysis and hybridization solutions eliminate the need to isolate nucleic acid prior to conducting the DNA probe assay, and facilitate the development of rapid and simple assay formats (*see* Van Ness and Chen. *Nucleic Acids Research 19*:5143. 1991, for review). However, the commonly used chaotropes do not substantially increase the differential hybridization of matched/mismatched sequences.

In addition, special problems arise when hybridization methods are employed that involve the use of mixed pools of oligonucleotide probes (12- to 50-mers) having differing base sequences and G+C content. Many applications utilize mixed pools of oligonucleotides and are frustrated by a host of problems. For example, many gene isolation strategies involve the reverse translation of a known polypeptide sequence into a set of all possible DNA sequences that can encode that protein (Jaye et al., *Nucl. Acids Res. 11*:2325-2335, 1983). A pool of oligonucleotide probes, homologous to the set of possible protein encoding DNA sequences, are then used to screen a genomic or cDNA library from the relevant organism or cell type in order to identify the desired gene sequence. While the length of all of the oligonucleotide probes is the same, the G+C content of each probe may vary significantly, making the selection of hybridization conditions that are suitable and specific for each oligonucleotide problematic. As a result, often many false positive clones will be selected when screening highly complex libraries for genes of low abundance.

This problem of simultaneously and accurately hybridizing many differing oligonucleotides of differing G+C content is even greater for sequence analysis of a specific region of DNA or identifying single base change mutations using large arrays of oligonucleotides (which may vary from 100% A+T to 100% G+C) bound to a fixed surface (Southern et al., *Genomics 13*:1008-1017, 1992; Maskos and Southern, *Nucl. Acids Res. 20*:1675-1678, 1992). These methods, while theoretically powerful, have been sorely limited by the inability to identify hybridization conditions that will facilitate accurate hybridization (*i.e.,* no mismatch hybrid duplexes formed) and allow all possible perfect hybrids to be stably formed.

One attempted solution has been to use a class of salts composed of small alkylammonium ions (most commonly tetramethylammonium (TMA+) and tetraethylammonium (TEA+)), that can greatly decrease the effect of base composition on DNA melting (Marky et al., *Biochemistry 20*:1427-1431, 1981; De Murcia et al., *Biophys. Chem. 8*: 377-383, 1978; Melchior and Von Hippel, *Proc. Nat. Acad. Sci. USA 70*:298-302, 1973). Of the tetraalkylammonium salts, only TMA+ and TEA+ are small enough to fit into the major groove of the B-form DNA double helix where they bind to the A+T base pairs of DNA (perhaps to the O-2 of thymine) (*see* De Murcia et al., *Biophysical Chemistry 8*:377 1978). The overall effect on stability is two-fold with the first being that the tetraalkylammonium salts increase the non-polar character of the solvent which destabilizes the base stacking interactions in native DNA (*see* Rees et al., *Biochemisrry 32*:137, 1993). The second effect is that the A+T base pairs are stabilized. Specifically, TMACl prevents DNA premelting by decreasing the transient openings between the base pairs from occurring below the melting temperature (*see* De Murcia et al.. *Biophysical Chemistry 8*:377 1978; Marky et al., *Biochemistry 20*:1427, 1981). The exact nature of TEACl stabilization is not known. Overall, the A+T pairing is stabilized resulting in a rise in the melting temperature for the A+T pairs (*see* Marky et al., *Biochemistry 20*:1427 1981; De Murcia et al., *Biophysical Chemistry 8*:377 1978). For 100% A+T oligonucleotide duplexes, the $T_m$ in TMACl is actually 6°C higher than that found in a sodium solution (*see* Marky et al., *Biochemistry 20*:1427, 1981).

When genomic DNA is melted in TMACl or TEACl at the specific concentrations of 3M and 2.4M, respectively, identical melting temperatures are exhibited for A+T and G+C pairs (*see* Melchior et al., *Proc. Natl. Acad. Sci. USA 70*:298, 1973). Usually what is observed is that synthetic DNA duplex stability in concentrated TMACl and TEACl stability is somewhat diminished and has little base compositional dependence (*see* Wood et al., *Proc. Natl. Acad. Sci. USA 82*:1585 1985; Marky et al., *Biochemistry 20*:1427 1981; Jacobs et al., *Nucleic Acids Res. 16*:4637, 1988). For example, a series of 19-mers ranging from 26% G+C to 79% G+C content had melting temperatures over a range of 18°C in 2X SSC, while in 3M TMACl the range narrowed to 5°C and in 2.4M TEACl, the temperatures were virtually unchanged negating all influence from G+C content (*see* Jacobs et al., *Nucleic Acids Res. 16*:4637, 1988). TEACl had the added benefit of reducing the melting temperature approximately 22°C over TMACl and SSC (*see* Jacobs et al., *Nucleic Acids Res. 16*:4637, 1988). When various lengths of hybridization probes are measured and the corresponding melting temperatures plotted versus length, the plot is a smooth curve even though the G+C content varied from 31-66% (*see* Wood et al., *Proc. Natl. Acad. Sci. USA 82*:1585 1985). In addition, the width of the melting curve, or the HCT, for natural DNA fragments is significantly reduced in TMACl (1°C) than in sodium solutions (5-10°C) (*see* Wood et al., *Proc. Natl. Acad. Sci. USA 82*:1585 1985). Narrowing of the HCT is indicative of the stabilization of the A+T pairing since the A+T pairs normally melt at a lower temperature than the G+C pairs creating broad melting curves.

In the context of gene isolation from complex libraries, the number of false positive clones isolated using a 17-mer mixed oligo pool (G+C range of 47% to 71%) was reduced 100-fold when performed in 3 M TMACl rather than using a NaCl hybridization solution (Wood et al., *Proc. Nat. Acad. Sci. USA 82*:1585-1588, 1985). However, even when using TMACl to eliminate the base composition effect on $T_m$, a significant number of false positive clones are still isolated due to formation of mismatched hybrids.

Using deoxyinosine at the third codon position (Honoré et al., *J. Biochem. Biophys. Methods 27*:39-48, 1993) of highly degenerate oligonucleotide pools from backtranslated protein sequences allows the oligonucleotide pool size to be significantly reduced. However, when screening a more complex genomic library for clones, the isolation of false positive clones may still be a significant problem (Jacobs et al., *Nucl. Acids Res. 16*:46374650, 1988). While the presence of tetramethylammonium and tetraethylammonium salts made oligonucleotide melting independent of base composition, there was no or little effect of mismatches on the thermal melting of oligonucleotides. That is, duplexes containing a mismatch had a similar $T_m$ to duplexes which were perfectly base-paired.

Another method used to enhance specificity in hybridization reactions creates base mismatches using base analogs to replace any of the A, G, C, or T nucleotides. Research has shown that some primers containing a base pair mismatch have increased specificity when the mismatch is placed in precise locations (*see* Wenham et al., *Clinical Chemistry 37*:241, 1991; Newton et al., *Nucleic Acids Research 17*:2503, 1989; Ishikawa et al., *Human Immunology 42*:315, 1995). However, differences of as little as 0.5°C in the melting temperatures are equally common between perfectly matched hybrids and the same hybrid with a single base mismatch introduced (*see* Tibanyenda et al., *European Journal of Biochemistry 139*:19, 1984; Werntges et al., *Nucleic Acids Research 14*:3773, 1986). Even better specificity has been noted between one and two base mismatched duplexes than has been observed between a perfectly matched duplex and the same duplex with a single mismatch (*see* Guo et al., *Nature Biotechnology 15*:331, 1997). Guo et al. found a ($T_m$ of 4°C between zero and one mismatches and a $\Delta Tm$ of 13°C between one and two adjacent mismatches for a 20-mer duplex. However, even with two mismatches, often there is still little destabilization of the duplex. This inability to consistently discriminate mismatches lends to the lack of specificity in PCR.

The use of more than one base pair mismatch per hybridization employing at least one nucleotide analog has been evaluated (see Guo et al., *Nature Biotechnology 15*:331. 1997). In this case, the analog compound consists of 3-nitropyrrole replacement of the purine or pyrimidine bases. 3-Nitropyrrole has the ability to minimally hydrogen bond with all four bases (see Nichols et al., *Nature 369*:492, 1994; Bergstrom et al., *Journal of the American Chemical Society 117*:1201. 1995). By introducing an artificial mismatch, large differences in the duplex melting temperatures occur ranging from approximately 5°C to 15°C with the largest difference occurring when the mismatch is located at the center of the 15-mer hybridizing oligo. Significant differences in $\Delta T_m$ occur when an artificial nucleotide is introduced into a duplex that already contains a base mismatch creating a two-mismatch duplex. The degree of destabilization depends upon the type of base mismatch (*e.g.,* G/T) and the separation between the two mismatches. In experimental examination, the base analog nucleotide ranged from 1 to 7 bases to the 3' side of the base mismatch, which was held in the center of the 15-mer. Differences in ATm for the three different base mismatched 15-mers ranged from a 2°C stabilization (in the C/T mismatch case only and when the mismatches are adjacent) to a 7°C further destabilization with the maximum destabilization consistently occurring at a 3 or 4 base mismatch separation (see Guo et al., *Nature Biotechnology 15*:331, 1997).

When two artificial mismatches are introduced, the proximity of the artificial bases greatly influences the degree of destabilization. The two artificial mismatches were centered on the middle of a 21-mer duplex beginning with a separation of 6 bp. The destabilization, or $\Delta Tm$, is minimally 12°C when compared to the perfectly matched duplex. The greatest difference of over 20°C occurs when the two artificial mismatches are 10 base pairs apart. This difference corresponds to one helical turn and indicates that some kind of interaction occurs between the two artificial bases that decreases the stability of the duplex.

Experimentally, when the PCR primer utilized contained one or two artificial mismatches between the primer and the DNA sample, the PCR gave results as would be expected for a perfectly matched primer (see Guo et al., *Nature Biotechnology* 15:331, 1997). However, when the primer contained both a true and an artificial mismatch, the PCR failed to produce any measurable results; while PCR with perfectly matched and true mismatches all produced measurable amounts of PCR product. The same study found similar results when using hybridization probes: those with perfect matches, true mismatches and artificial mismatches annealed while the probes containing artificial and true mismatches did not. These studies indicate greater specificity is created when artificial base mismatches are incorporated in hybridization reactions such that when naturally occurring mismatches occur, they are thermodynamically less stable than a perfectly matched hybridization reaction and thus less likely to produce a false positive in an assay or PCR. Interestingly, however. the difference in thermodynamic stability noted above for duplexes containing only artificial mismatches is not manifested in the experimental situation.

A further means of effecting hybridization discrimination is through differences in the stability between hybridization duplexes that contain nicks and gaps. In these reactions, duplexes are formed from tandemly stacked short oligomers hybridized to a longer strand that either align contiguously or non-contiguously leaving a few base pair gap. Hybridizations that result in a nick are subject to "stacking hybridization" where another DNA strand hybridizes across the nick site. Stacking hybridization does not occur where gaps are present in the non-contiguous oligomers. The stacking has the effect of increased discrimination as evidenced by decreased dissociation rates and greater thermodynamic stability than the non-contiguous counterparts (see Lane et al., *Nucleic Acids Res. 25*:611, 1997). Thermodynamic measurements show differences between the hybridization stacked duplexes standard free energy change ($\Delta G$) and the gapped duplexes is 1.4 to 2.4 kcal/mol. Therefore, discrimination in hybridization can be afforded through the use of multiple short probes.

Most of the base mimics in current use are the result of the pursuit for a universal base. Many utilize nitroazole base analogues and have demonstrated reduced discrimination in base pairing. A series of nitroazole nucleobase analogues have been studied in attempts to gain additional insight into the significance of electronic structure and heterocyclic size in base pairing for the development of more effective universal bases (see Bergstrom et al., *Nucleic Acids Res. 25*:1935, 1997). In this work, the thermodynamic properties of the deoxyribonucleosides of 3-nitropyrrole, 4-nitropyrazole, 4-nitroimidazole, and 5-nitroindole were measured. For comparison, thermodynamic measurements were also made on the deoxyribonucleosides of hypoxanthine and pyrazole as well an abasic spacer, 1,2-dideoxyribose. Four oligonucleotides were synthesized for each modified nucleoside in order to obtain duplexes in which each of the four natural bases was placed opposite the base mimic. All of the base mimics analyzed proved to be far less stable than the natural base pairings (A+T: $T_m$ = 65.7°C, C+G: $T_m$ = 70.5°C) with the $T_m$s ranging from 35-46°C for 5-nitroindole to 18-29°C for the other nitroazole bases analyzed. The only exception was 4-nitroimidazole paired with dGTP where the $T_m$ was 40.9°C. In analyzing the free energy for the duplex melting, the 3-nitropyrrole base mimic was found to have the least discrimination when pairing with any of the four naturally occurring bases with an overall $\Delta G$ of 0.4kcal/mol. The next least discriminating was 5-nitroindole with a $\Delta G$ of 0.8 kcal/mol. Both of these values are less than the $\Delta G$ of 1.1 kcal/mol found between the natural base pairings of A+T and G+C 4-Nitropyrazole showed a slight preference for pairing with A with a $\Delta G$ of 1 kcal/mol more stable than C. G, and T free energies. Finally, 4-nitroimidazole showed a high selectivity for pairing to G (as was evidenced by its high $T_m$ value) due to the ability of the imidazole N3 to hydrogen bond with the deoxyguanosine N1. It should be noted, however. that the above values are dependent upon the nearest base neighbors to the mimic. Further studies altered the nearest neighbors and found that 3-nitropyrrole and 5-nitroindole are quite non-discriminating base pairing partners.

Of interest, the enthalpy and entropy changes were found to track one another (*i.e.,* a large enthalpy change correlates to a large entropy change) regardless of the base mimic utilized implying that the correlation between AS and AH is independent of the mode of association of the bases. What was observed was that small enthalpy and entropy changes were found in the non-hydrogen bonding base mimics. The low values for entropy change reflect the greater degree of freedom of movement possible for bases that are not locked into the duplex by hydrogen bonding interactions. The small enthalpy changes reflect alterations in hydrogen bonding interactions as a result of the loss of hydrogen bonding interactions for the base opposite the base mimic. If a natural base remains stacked in the helix without an opposing hydrogen bonding partner then it has lost hydrogen bonding interactions with water without regaining a new donor/acceptor partner.

A similar study involved examining acyclic nucleoside analogues with carboxamido- or nitro-substituted heterocyclic bases (*see* Aerschot et al., *Nucleic Acids Res.* 23:4363, 1995). Utilization of acyclic nucleosides endows the constructs with enough flexibility to allow good base stacking as well as allow the base mimics to obtain an orientation to best base-pair with the corresponding base. The heterocyclic bases examined included: 4,5-imidazoledicarboxamide, 4-nitroimidazole, and 5-nitroindazole. These complexes were referenced against acyclic hypoxanthine, 1-(2(-deoxy-(-D-ribofuranosyl)-3-nitropyrrole, 5-nitroindole, and 2-deoxyinosine. All the new acyclic complexes had melting temperatures 7-20°C less than those observed for the natural bases. 5-Nitroindazole when paired against each of the four natural bases had the least spread in $\Delta T_m$ of only 2.2°C while the 4-nitroimidazole had a spread of 8.0°C with dG being significantly out of line with the other three bases as had similarly been observed above. Of the reference compounds, deoxyinosine had a $\Delta T_m$ of 5.6°C, 5-nitroindoles $\Delta T_m$ was 1.0°C, 1-(2-(deoxy-(-D-ribofuranosyl)-3-nitropyrrole had a $\Delta T_m$ of 5.1°C, and the $\Delta T_m$ of acyclic hypoxanthine was 4.8°C. However, all base mimics showed about the same destabilization ($\Delta T_m$ of 4-5°C) when placed in an oligo consisting almost exclusively of adenosines with exception of 4-nitroimidazole and acyclic deoxyinosine that had $\Delta T_m$s of 7.0°C and 8.9°C, respectively.

Aerschot and co-workers also examined the effect of incorporation of multiple base mimics into an oligo (*see* Aerschot et al., *Nucleic Acids Res. 23*:4363, 1995). Overall, melting temperatures dropped but most markedly with the incorporation of three base mimics. The nitroindoles, however, showed the least amount of temperature differential.

Another base mimic, 1-(2(-deoxy-(-D-ribofuranosyl) imidazole-4-carboxamide (Nucleoside 1), mimics preferentially dA as well as dC nucleosides *(see* Johnson et al., *Nucleic Acids Res. 25*:559, 1997). The ability to substitute for both dA and dC results from rotation about the carboxamide/imidazole bond as well as the bond between the imidazole and furanose ring. When the imidazole is anti to the furanose and the carboxamide group is anti to the imidazole, the lone pair on the oxygen and one of the amide NH hydrogens is in a position that mimics the $NH_2$ and N-1 of adenosine. Imidazole rotation about the glycosidic bond to the syn orientation places the amide group in a position that approximately matches the positions of the $NH_2$ and N-3 of cytosine.

When Nucleoside 1 is substituted for any naturally occurring nucleoside, the enthalpy increases with the greatest increase for a dG substitution for the 1-C pairing (from $\Delta H$ = 74.7 (kcal/mol)/$\Delta G$ = -16.5 (kcal/mol) for the G/C pairing to $\Delta H$ = -45.5 (kcal/mol)/$\Delta G$ = -5.8 (kcal/mol)). The smallest enthalpy change occurs for a dA substitution ($\Delta H$ = -72.9 (kcal/mol)/$\Delta G$ = -15.4 (kcal/mol) for A/T pairing to $\Delta H$ = -66.7 (kcal/mol)/$\Delta G$ = -11.7 (kcal/mol) for the 1-T pairing). Correspondingly, $T_m$ significantly decreases from 65.7°C and 70.5°C for the A-T and C-G couples, respectively, to 46.6°C for the 1-T pairing, 43.4°C for 1-G, 27.6°C for 1-A, and 14.6°C for 1-C.

When used in a PCR reaction, Nucleoside 1 and its N-propyl derivative are preferentially incorporated as dATP analogues (*see* Sala et al., *Nucleic Acids Res. 24*:3302, 1996). However, once incorporated into a DNA template, their ambiguous hydrogen bonding potential gave rise to misincorporation of any of the naturally occurring bases at frequencies of 3 x $10^{-2}$ per base per amplification. Most of the substitutions (primarily consisting of G) were a result of rotation about the carboxamide bond when part of the template. Between 11-15% of the substitutions were due to rotation of the imidazole moiety about the glycosidic bond. As part of a DNA template, the N-propyl derivative behaved in the same way as Nucleoside 1 despite its propyl moiety. This study indicates that while Nucleoside I preferentially behaves as dATP, it has the ability in a PCR type environment to behave as all four naturally occurring nucleotides as well. From this and the above studies, it is evident that a wide range of duplex stability can be obtained through variations in base mimics and their placement within an oligonucleotide.

Petrruska et al., *Proc. Natl. Acad. Sci. USA 85*:6252-6256, 1988, have reported on the correlation between the thermodynamic stability of mismatched primers and DNA polymerase fidelity. By analyzing the melting profiles of a perfectly based paired primer with a A/T correct match at the 3'-end compared to primers that had either the incorrect base pair G/T, C/T, or T/T it was noted that there was a shift in free energy changes upon dissociation ($\Delta\Delta G^0$) of 0.2, 0.3 and 0.4 kcal/mole for the terminal A/T compared to the G/T, C/T, or T/T mismatches. Interestingly, the A/T mismatch was extended (Drosphilia DNA polymerase) about 200 times faster than the G/T mismatch and about 1400 and 2500 times faster than the C/T and T/T mismatched respectively. The authors hypothesized that the binding cleft of the polymers excludes water and amplifies by amplifying free energy differences by increasing enthalpy differences in mismatched primers.

Livshits et al., *J. Bimolecular Structure and Dynamics 11*:783-795, Adenine Press, ISSN 0739-1102, has presented interesting data and theoretical considerations of dissociation curve of oligonucleotides immobilized in gel-based arrays. The gel introduces a complicating factor of having to account for the effect of diffusion on the dissociation process. However, there was agreement between the calculated and experimental dissociation temperatures due in part to enthalpy/entropy compensation.

Many DNA hybridization-based diagnostic tests are being developed to identify persons who might be suffering from (or be predisposed to) specific genetic diseases (*see* for example, Norari et al., *Gene 43*:23-28, 1986) or to determine a genetic histocompatibility profile, which is useful for tissue matching between donor and patient (*e.g.,* for a bone marrow transplant) (Sorg et al., *Fur. J Immunogen 19*:391-401, 1992). However, significant problems are encountered when trying to develop simple and reliable hybridization methods using allele-specific oligonucleotide probes that differ in sequence at one nucleotide position. Norari et al. solved the mismatch hybridization problem by the addition of 10-times more unlabeled complementary oligonucleotide than the mismatched labeled oligonucleotide. However, this is an impractical solution when multiplex hybridization methods are being used.

Diagnostic tests that rely on the polymerase chain reaction (PCR) technique also experience problems associated with the hybridization of oligonucleotides. Rychlik *(BioTechniques 18*:84-90, 1995) examined the effects on PCR of varying the G+C content of primers at either the 5' or 3' end of a priming oligonucleotide. Using standard PCR buffers and conditions, oligonucleotides having a high G+C content at the 3' end (the end used to extend DNA synthesis during PCR) results in high priming efficiency, but also promotes false priming due to greater tolerance for mismatches at the 5' end. Moreover, the effects of mismatches in PCR are variable; mismatches located in the middle of a primer-template duplex do not significantly affect the efficiency of PCR amplification, while 3'-terminal base mismatches sometimes strongly affects PCR product yield. As a further complication, the strength of the effect that the various base pair mismatches have on PCR amplification is not the same as that observed for oligonucleotide hybrid formation and stability (Ikuta et al., *Nucl. Acids. Res. 15*:797-811, 1987; Jacobs et al., *Nucl. Acids Res. 16*:4637-4650, 1988).

The present invention provides methods and compositions for detecting base changes by improving the specificity and accuracy of hybridization of an oligonucleotide with a target DNA sequence, and further provides other related advantages.

## SUMMARY OF THE INVENTION

This invention generally provides compositions and methods to increase the specificity of hybridization of nucleic acids.

In one aspect, the invention provides a composition comprising a nucleic acid and a salt, the salt comprising an anion and a cation, the anion selected from halogenated acetate, propionate and halogenated propionate, the cation selected from primary, secondary and tertiary ammonium comprising 1-36 carbon atoms, and quaternary ammonium comprising 448 carbon atoms.

In another aspect, the invention provides a composition which is non-flowing comprising a oligonucleotide of 6-100 nucleotides and a salt, the salt comprising an anion and a cation, the anion selected from acetate, halogenated acetate, propionate, and halogenated propionate, the cation selected from primary, secondary and tertiary ammonium comprising 1-36 carbon atoms, and quaternary ammonium comprising 4-48 carbon atoms. A "non-flowing" composition does not flow, as solutions flow during chromatography.

In another aspect, the invention provides a composition which is free from organic solvent, comprising a oligonucleotide of 6-100 nucleotides and a salt, the salt comprising an anion and a cation, the anion selected from acetate, halogenated acetate, propionate, and halogenated propionate, the cation selected from primary, secondary and tertiary ammonium comprising 1-36 carbon atoms, and quaternary ammonium comprising 4-48 carbon atoms.

In another aspect, the invention provides a composition which includes a nucleic acid and a salt, where the nucleic acid is immobilized on a solid support, and the salt is formed from an anion and a cation, the anion selected from acetate, halogenated acetate, propionate and halogenated propionate, the cation selected from primary, secondary and tertiary ammonium comprising 1-36 carbon atoms, and quaternary ammonium comprising 4-48 carbon atoms.

In another aspect, the invention provides a salt selected from the group:

(a) an acetate salt of a cation of the formula $HN(CH_3)_2R_a$ wherein $R_a$ is a $C_4$-$C_7$hydrocarbyl;
(b) a halogenated acetate salt of a cation of the formula $HN(CH_3)_2R_b$ wherein $R_b$ is a $C_7$-$C_{12}$hydrocarbyl;
(c) acetate and halogenated acetate salts of a cation of the formula $H_2N(C_5$-$C_7cycloalkyl)R_c$ where $R_c$ is a $C_1$-$C_{12}$hydrocarbyl; and

(d) acetate and halogenated acetate salts of N-substituted piperdine, wherein the nitrogen atom of piperidine is substituted with $C_1$-$C_{12}$hydrocarbyl. Such salts may be prepared by combining the respective acid and bases.

In another aspect, the invention provides an oligonucleotide in solution, where an oligonucleotides if formed, at least in part, from a plurality of fragments, each fragment shown schematically by structure (1) wherein,

represents a sequence of at least three nucleotides as found in wild-type DNA, where "B" represents a base independently selected at each location;

▬▬▬ represents represents a series of covalent chemical bonds termed a "specificity spacer," which separates and connects two bases $B_3$ and $B_5$;

the specificity spacer having steric and chemical properties such that

(a) it does not prevent hybridization between a fragment of structure (1) and an oligonucleotide fragment having a complementary base sequence, as shown schematically as structure (2)

In addition, the specificity spacer has at least one of the following properties: it cannot enter into hydrogen bonding with a base positioned opposite itself in a hybridized complementary base sequence of structure (2); it can enter into hydrogen bonding with a base positioned opposite itself in a hybridized complementary base sequence of structure (2), however it does not hydrogen-bond through any of adenine, guanine, cytosine, thymine or uracil according to standard Watson-Crick hydrogen bonding.

In another aspect, the invention provides an array, where the array includes a plurality of oligonucleotides immobilized in an array format to a solid support. Each oligonucleotide of the plurality is formed, at least in part, from a plurality of fragments, each fragment shown schematically by structure (1) wherein,

represents a sequence of at least three nucleotides as found in wild-type DNA, where "B" represents a base independently selected at each location;

▬▬▬▬ represents a series of covalent chemical bonds termed a "specificity spacer," which separates and connects two bases $B_3$ and $B_5$;

the specificity spacer having steric and chemical properties such that

(a) it does not prevent hybridization between a fragment of structure (1) and an oligonucleotide fragment having a complementary base sequence, as shown schematically as structure (2)

In addition, the specificity spacer has at least one of the following properties: it cannot enter into hydrogen bonding with a base positioned opposite itself in a hybridized complementary base sequence of structure (2); it can enter into hydrogen bonding with a base positioned opposite itself in a hybridized complementary base sequence of structure (2), however it does not hydrogen-bond through any of adenine, guanine, cytosine, thymine or uracil according to standard Watson-Crick hydrogen bonding.

In another aspect, the invention provides a composition which includes an oligonucleotide and a salt in solution, the oligonucleoditde being formed, at least in part of a plurality of fragments, each fragment shown schematically by structure (1) wherein,

represents a sequence of at least three nucleotides as found in wild-type DNA, where "B" represents a base independently selected at each location;

▬▬▬▬ represents a series of covalent chemical bonds termed a "specificity spacer," which separates and connects two bases $B_3$ and $B_5$;

the specificity spacer having steric and chemical properties such that

(a) it does not prevent hybridization between a fragment of structure (1) and an oligonucleotide fragment having a complementary base sequence, as shown schematically as structure (2) and

at least one of (b) and (c) where

(b) the specificity spacer cannot enter into hydrogen bonding with a base positioned opposite itself in a hybridized complementary base sequence of structure (2);

(c) the specificity spacer can enter into hydrogen bonding with a base positioned opposite itself in a hybridized comlementary sequence of structure (2) but the specificity spacer does not provide any base selected from adenine, guanine, thymine, uracil or cytosine for the hydrogen bonding;

and the salt is a hybotrope.

In another aspect, the invention provides an array composition formed, at least in part of a plurality of oligonucleotides immobilized in an array format to a solid support, each oligonucleotide of the plurality formed, at least in part of a plurality of fragments, each fragment shown schematically by structure (1) wherein,

represents a sequence of at least three nucleotides as found in wild-type DNA, where "B" represents a base independently selected at each location;

████████ represents a series of covalent chemical bonds termed a "specificity spacer," which separates and connects two bases $B_3$ and $B_5$;

the specificity spacer having steric and chemical properties such that

(a) it does not prevent hybridization between a fragment of structure (1) and an oligonucleotide fragment having a complementary base sequence, as shown schematically as structure (2) and

at least one of (b) and (c) where

(b) the specificity spacer cannot enter into hydrogen bonding with a base positioned opposite itself in a hybridized complementary base sequence of structure (2);

(c) the specificity spacer can enter into hydrogen bonding with a base positioned opposite itself in a hybridized comlementary sequence of structure (2) but the specificity spacer does not provide any base selected from adenine, guanine, thymine, uracil or cytosine for the hydrogen bonding; and the nucleic acid of formula (1) being in contact with a hybotrope.

In yet other aspects, methods are provided for distinguishing between hybridization of a complementary nucleic acid target and a nucleic acid probe in which the probe and target are either perfectly complementary or have one or more base mismatches, comprising the steps: (a) mixing the target and probe in a solution comprising a hybotrope; and (b) hybridizing at a discriminating temperature; and detecting the amount of probe hybridized to the target, thereby determining whether the duplex is perfectly complementary or mismatched. In preferred embodiments, the probe or target is from 6 to 40 bases. In another preferred embodiment, the probe is labeled. In a related aspect, the probe has one or more abasic residues and the solution does not contain a hybotrope. In a preferred embodiment, the hybridization reaction mixture comprises a hybotrope.

In yet another aspect, methods are provided for increasing discrimination in a nucleic acid synthesis procedure, such as polymerase chain reaction. In these methods, a single stranded nucleic acid target is mixed with an oligonucleotide primer or a solution comprising a hybotrope and a polymerase, the primer is annealed to the target at a discriminating temperature, and a complementary strand to the target is synthesized. The amount of duplex formed for a mismatched primer and target is less than for a perfectly matched primer and target.

These and other aspects of the present invention will become evident upon reference to the following detailed description and attached drawings. In addition, various references are identified below and are incorporated by reference in their entirety.

BRIEF DESCRIPTION OF THE DRAWINGS

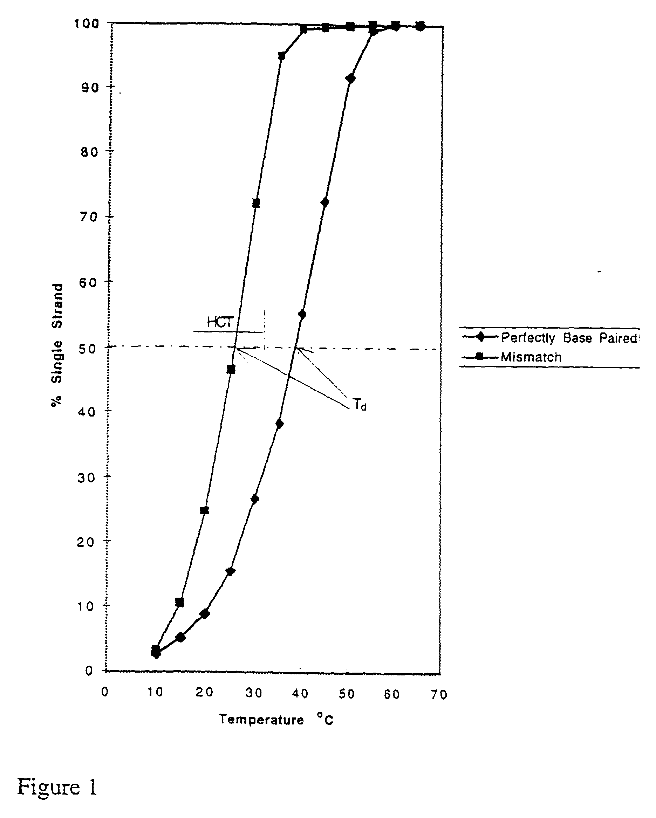

Figure 1 is a graph illustrating thermal melt profiles of oligonucleotide duplexes. Percentage single strand DNA ($\alpha$, y-axis) is plotted versus temperature (x-axis). The Td of the duplex is defined as the temperature at which 50% of the strands are in single strand form. The helical coil transition (HCT) is defined as the temperature difference between an $\alpha$ of 0.2 (or 20%) and 0.8 (or 80%). The melting curve denoted by the squares represents the behavior of a duplex in contact with a hybotrope (*e.g.*, LiTCA) and the melting curve denoted by the diamonds represents the behavior of an oligonucleotide duplex in a NaCl-based hybridization solution.

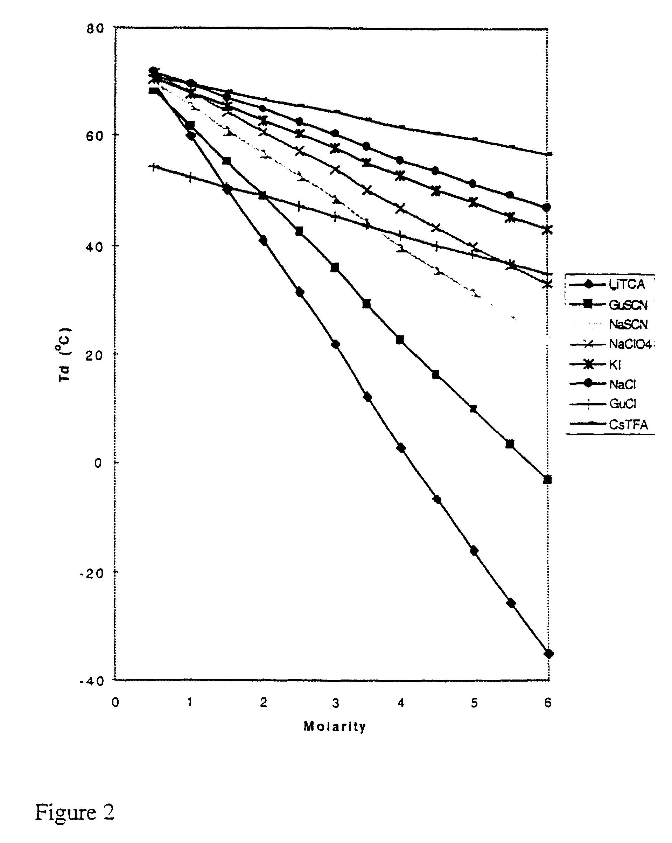

Figure 2 is a graph illustrating the relationship of the $T_d$ of an oligo duplex and salt concentration in hybridization solutions (LiTCA, GuSCN, NaSCN, NaClO$_4$, KI, NaCl, GuCl, CsTFA). The $T_d$ in degrees C is plotted versus molarity of the salt.

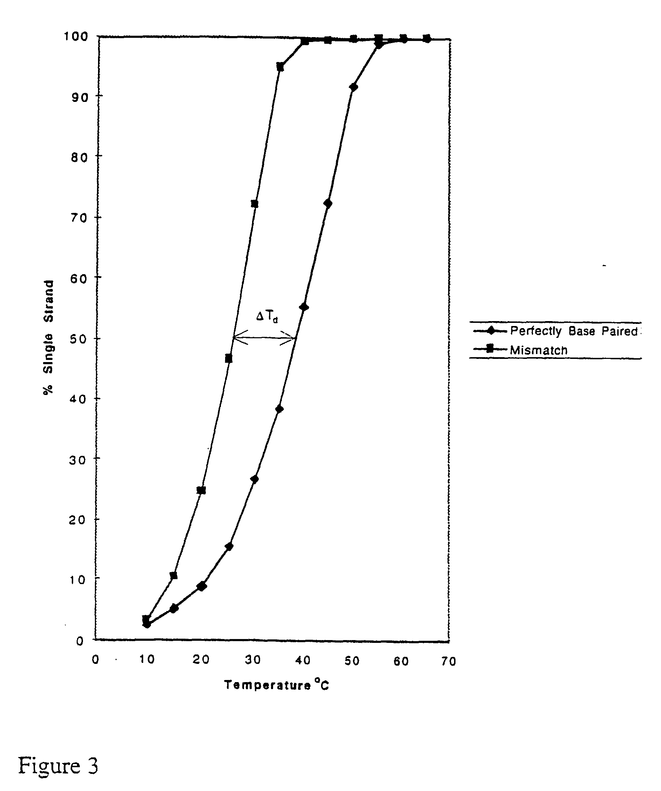

Figure 3 is a graph showing the difference in $T_d$ between two duplexes. one that is perfectly based-paired and the other that contains a single mismatch. The temperature difference between any two $T_d$s at $\alpha = 0.5$ is defined as the $\Delta T_d$. The percentage of single strand DNA (y-axis) is plotted versus temperature (°C; x-axis).

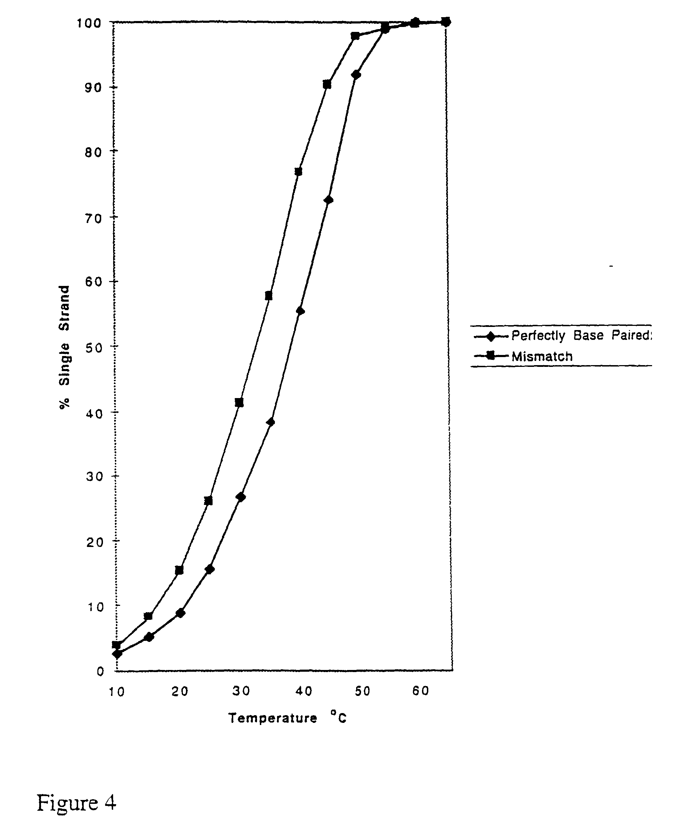

Figure 4 is a graph displaying melting profiles for an 18-mer oligonucleotide duplex that is perfectly based paired (diamonds) and the same oligonucleotide duplex that contains a central mismatch (squares A/A, position 9). The $\Delta T_d$ is 6°C. The melting profiles were determined in 2.0 M LiTCA. The percentage single strand (y-axis) is plotted versus temperature (°C; x-axis).

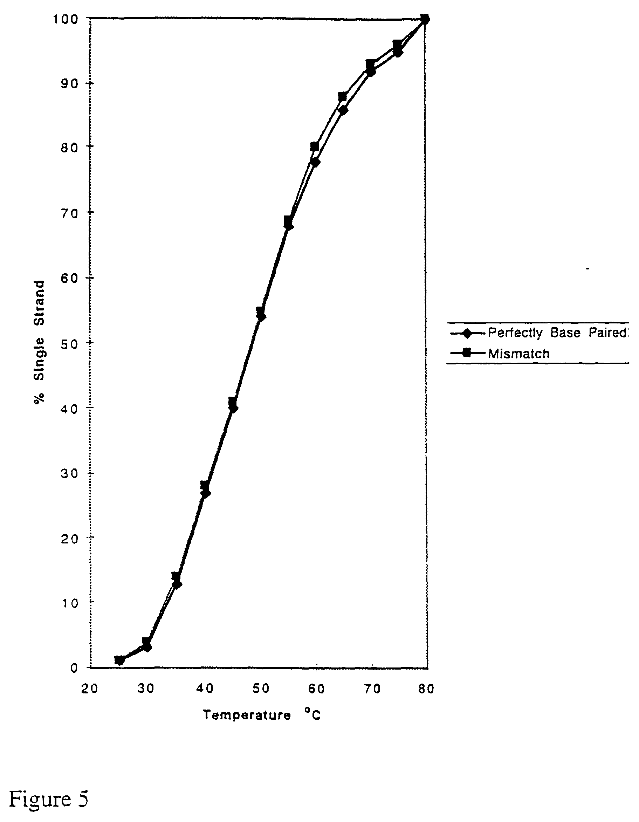

Figure 5 is a graph illustrating melting profiles for an 18-mer oligonucleotide duplex that is perfectly based-paired (diamonds) and the same oligonucleotide duplex that contains a central mismatch (squares; A/A, position 9). The melting curves are determined in QY low stringency hybridization buffer (Promega, Madison, WI). The percentage single strand (y-axis) is plotted versus temperature (°C; x-axis).

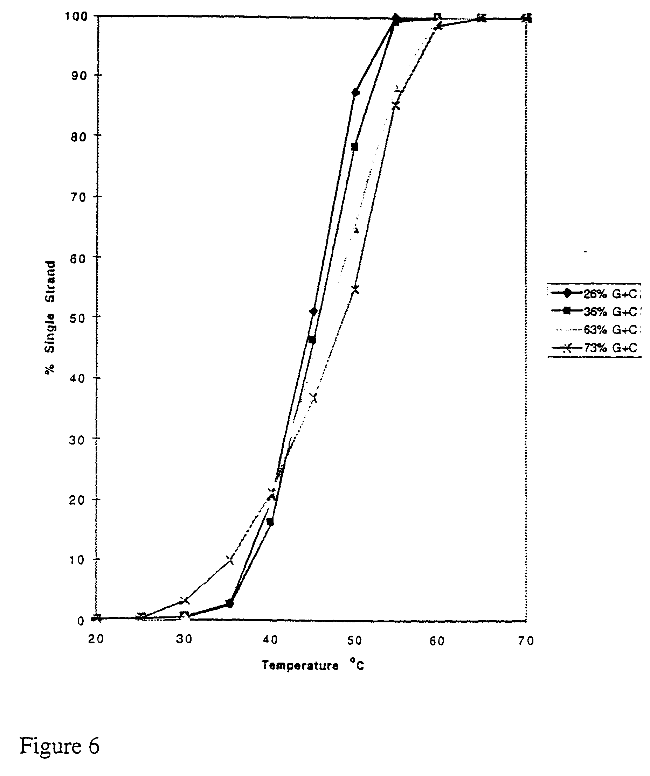

Figure 6 is a graph showing melting profiles for a set of 19-mer oligonucleotides duplexes that vary in G+C composition from 26% to 73%. All of the duplexes are perfectly based paired. The $\Delta T_d$ is 5°C across the entire G+C range. The melting profiles are determined in 3M TMATCA. The % single strand (y-axis) is plotted versus temperature (°C; x-axis).

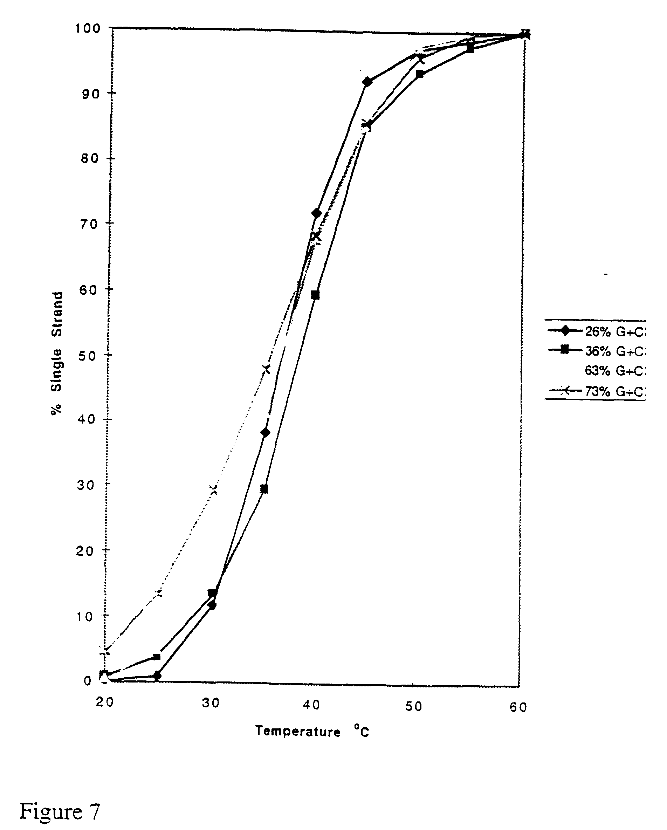

Figure 7 is a graph displaying melting profiles for a set of 19-mer oligonucleotides duplexes that vary in G+C composition from 26% to 73%. All of the duplexes are perfectly based paired. The $\Delta T_d$ is 4°C across the entire G+C range. The melting profiles are determined in 3M TEATCA. The % single strand (y-axis) is plotted versus temperature (°C; x-axis).

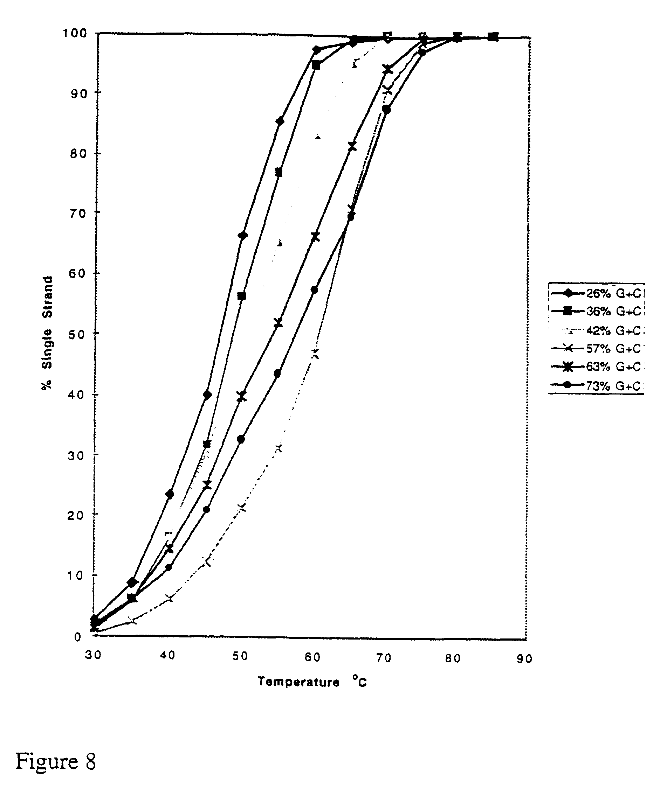

Figure 8 is a graph illustrating melting profiles for a set of 19-mer oligonucleotide duplexes that vary in G+C composition from 26% to 73%. All of the duplexes are perfectly base-paired. The $\Delta$-$T_m$ is 16°C across the entire G+C range. The melting profiles are determined in 0.165M NaCl. The % single strand (y-axis) is plotted versus temperature (°C; x-axis).

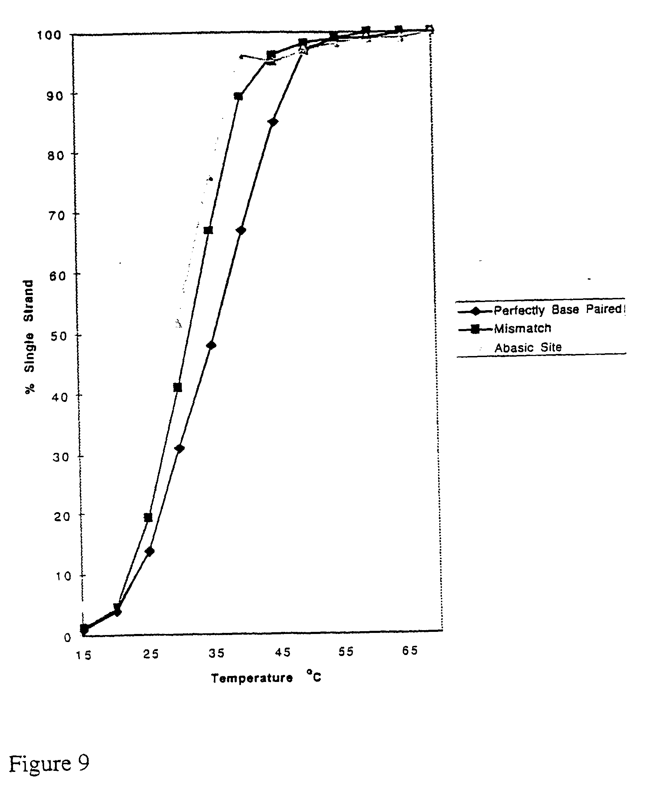

Figure 9 is a graph illustrating melting profiles for an 18-mer oligonucleotide duplex that is perfectly based paired and the same oligonucleotide duplex that contains either a central mismatch (A/A) or abasic substitution at position 9. The melting profiles are determined in GuSCN. The % single strand (y-axis) is plotted versus temperature (°C; x-axis).

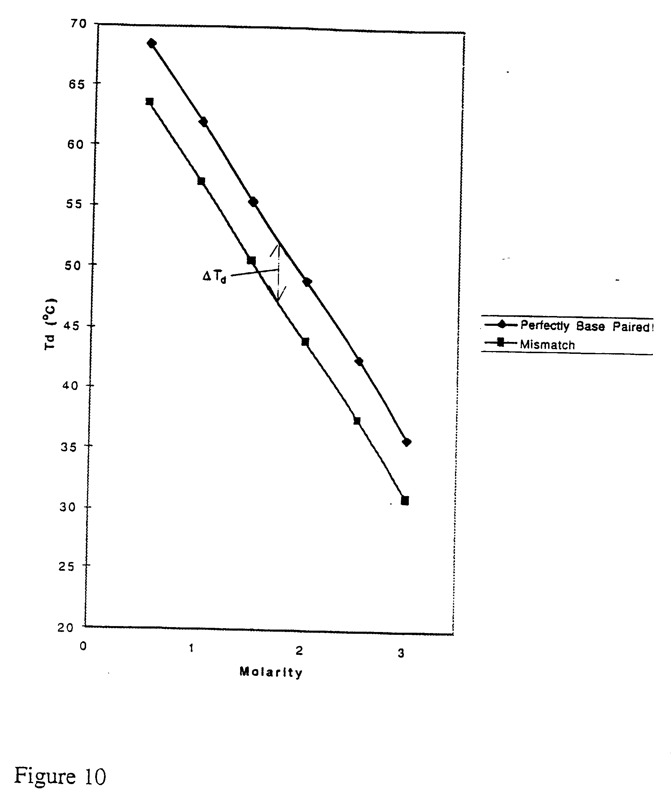

Figure 10 is a graph showing the relationship between molarity and $T_d$ of the data obtained from the melting curves described in Figure 9. The $T_d$ on the y-axis is plotted versus the molarity of GuSCN on the x-axis.

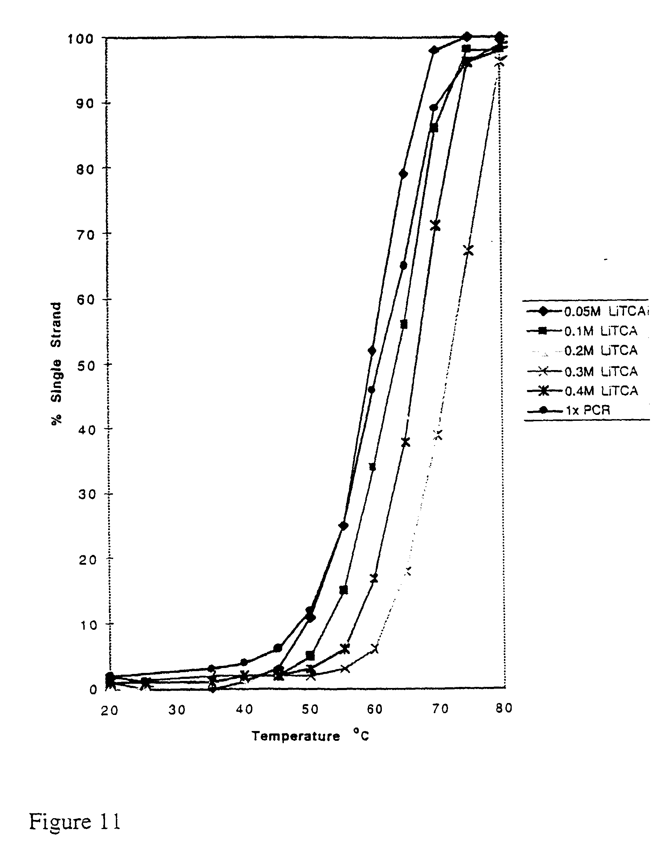

Figure 11 is a graph illustrating melting profiles for an 18-mer oligonucleotide duplex that is perfectly based paired in 1 x PCR buffer or LiTCA over a concentration range of 0.05 M to 0.4 M. The % single strand (y-axis) is plotted versus temperature (x-axis).

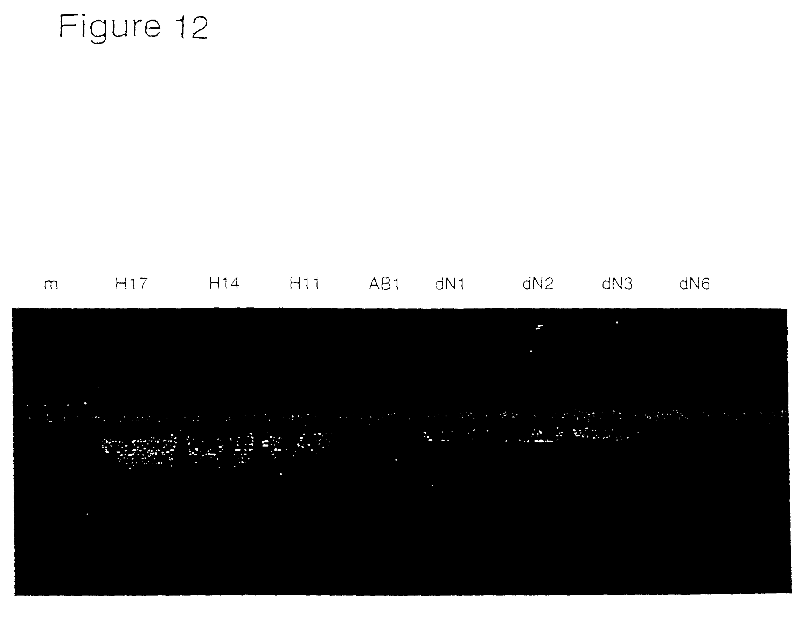

Figure 12 is a photograph of a 2% agarose gel that shows the presence or absence of an amplicon 381 bp in length. "m", marker; and H17, H14, H11, AB1, dN1, dN2, dN3 and dN6 are the 5' primers used in amplification.

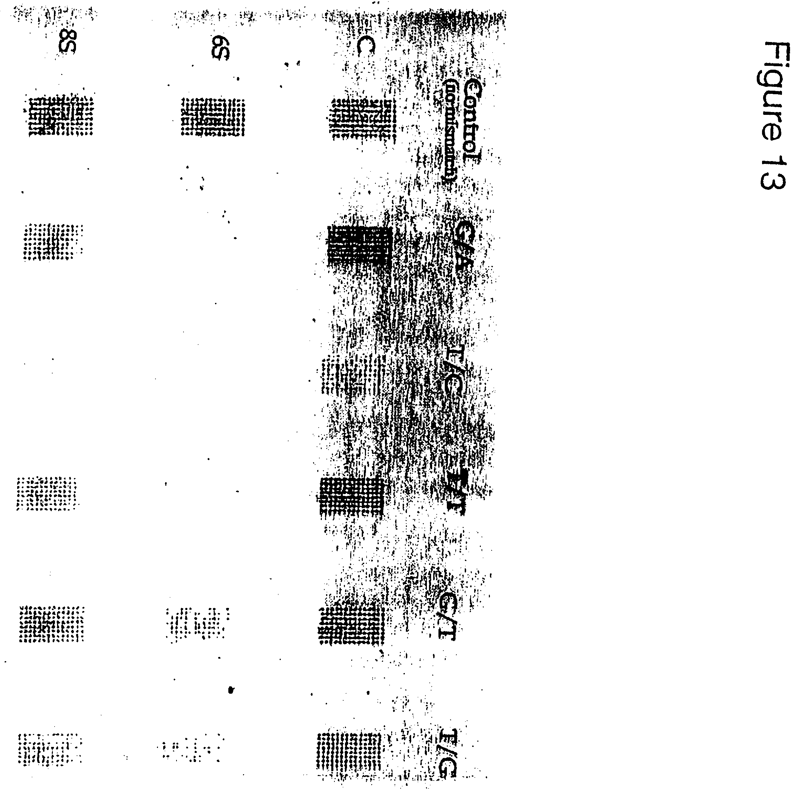

Figure 13 is the text scan of a set of arrayed oligonucleotides that when duplexed with probe contain the mismatch indicated in the top row. "C" indicates control probe, "6S" indicates the 6S abasic substituted probe and "8S" indicates the 8S abasic substituted probe. The figure is a compilation of 3 separate filters.

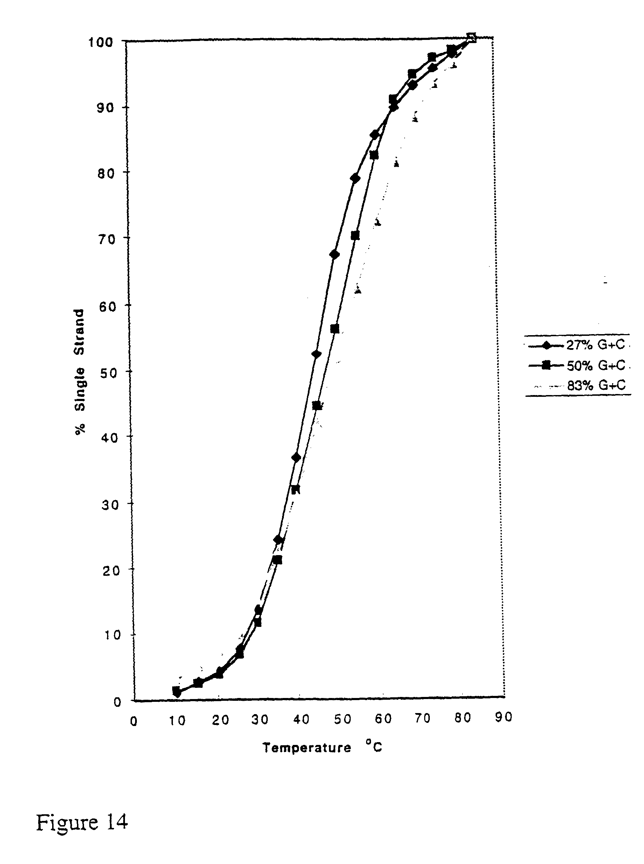

Figure 14 is a graph showing the difference in $T_d$ between three duplexes, that vary in G+C content from 27% to 83%. The percentage of single strand DNA (y-axis) is plotted versus temperature (°C; x-axis). The melting solution was 100 mM 2-methoxyethylamine trifluoroacetate.

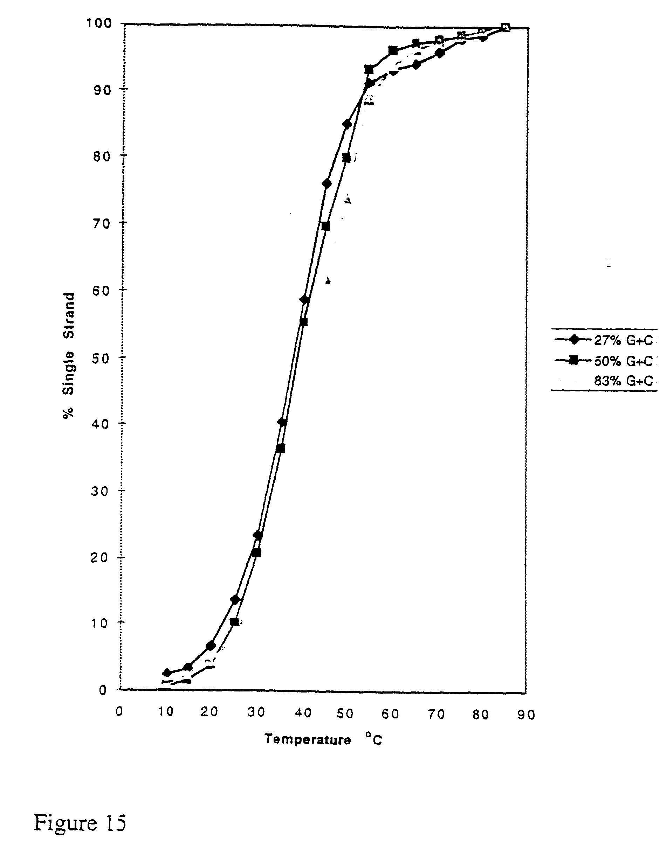

Figure 15 is a graph showing the difference in $T_d$ between three duplexes, that vary in G+C content from 27% to 83%. The percentage of single strand DNA (y-axis) is plotted versus temperature (°C; x-axis). The melting solution was 100 mM diisobutylamine acetate.

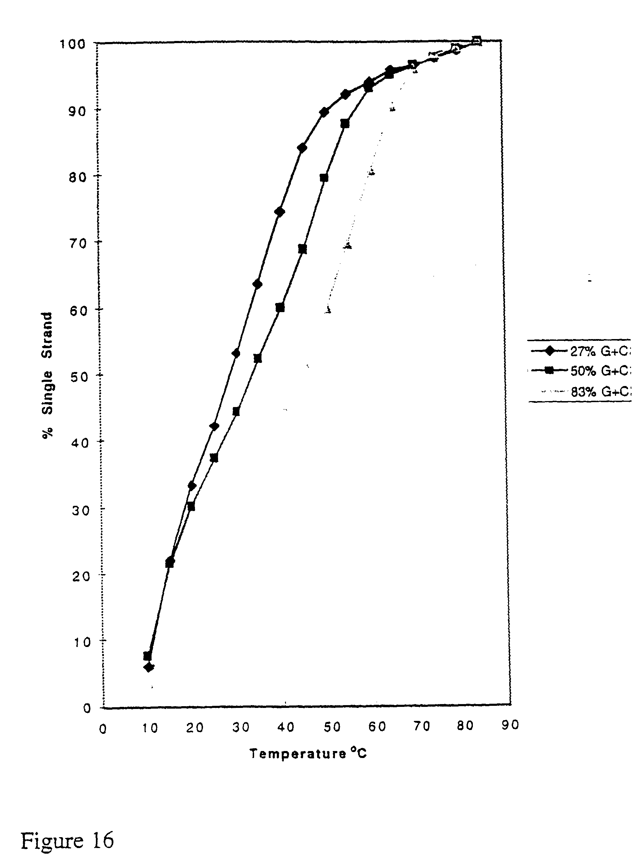

Figure 16 is a graph showing the difference in $T_d$ between three duplexes, that vary in G+C content from 27% to 83%. The percentage of single strand DNA (y-axis) is plotted versus temperature (°C; x-axis). The melting solution was 2 M Guanidinium thiocyanate.

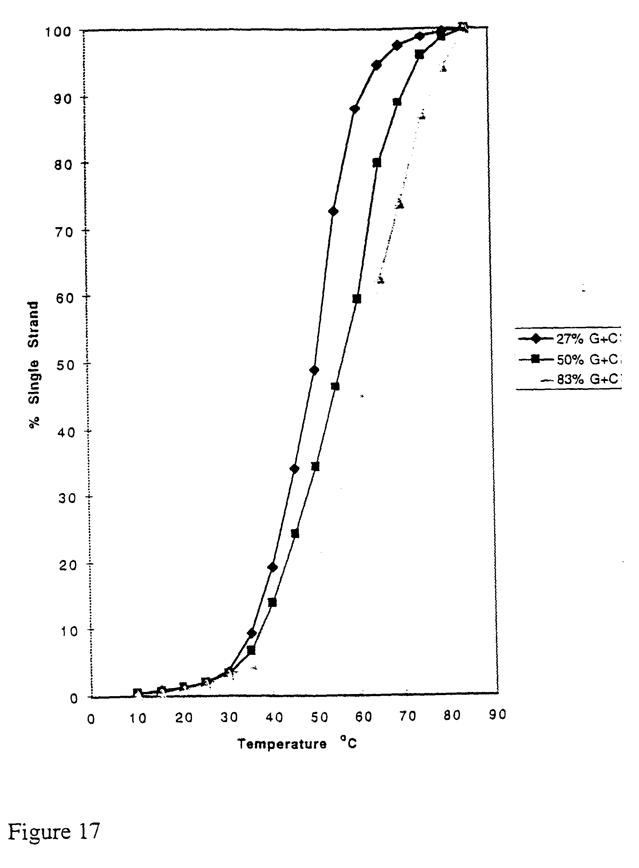

Figure 17 is a graph showing the difference in $T_d$ between three duplexes, that vary in G+C content from 27% to 83%. The percentage of single strand DNA (y-axis) is plotted versus temperature (°C; x-axis). The melting solution was 1x PCR buffer.

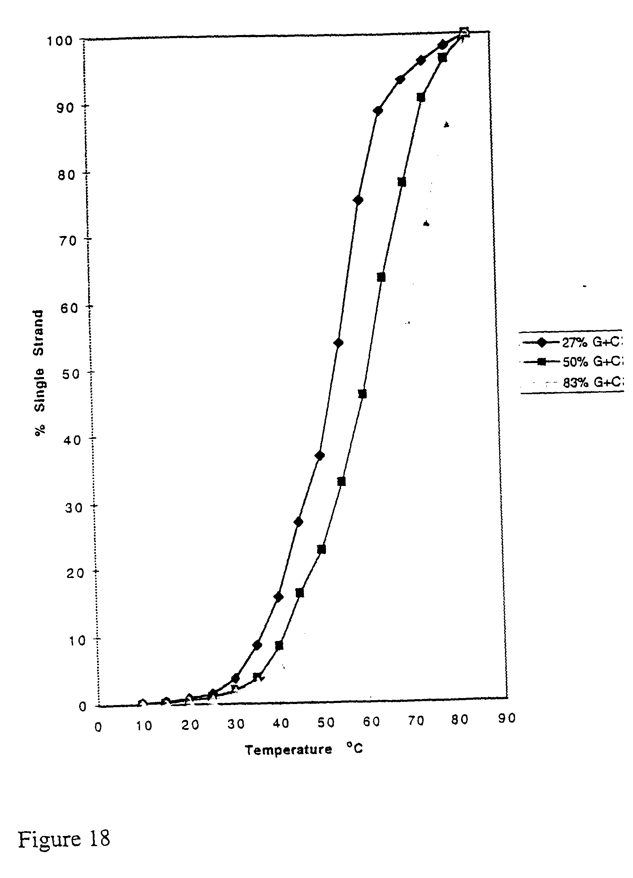

Figure 18 is a graph showing the difference in $T_d$ between three duplexes, that vary in G+C content from 27% to 83%. The percentage of single strand DNA (y-axis) is plotted versus temperature (°C; x-axis). The melting solution was 1x SSC.

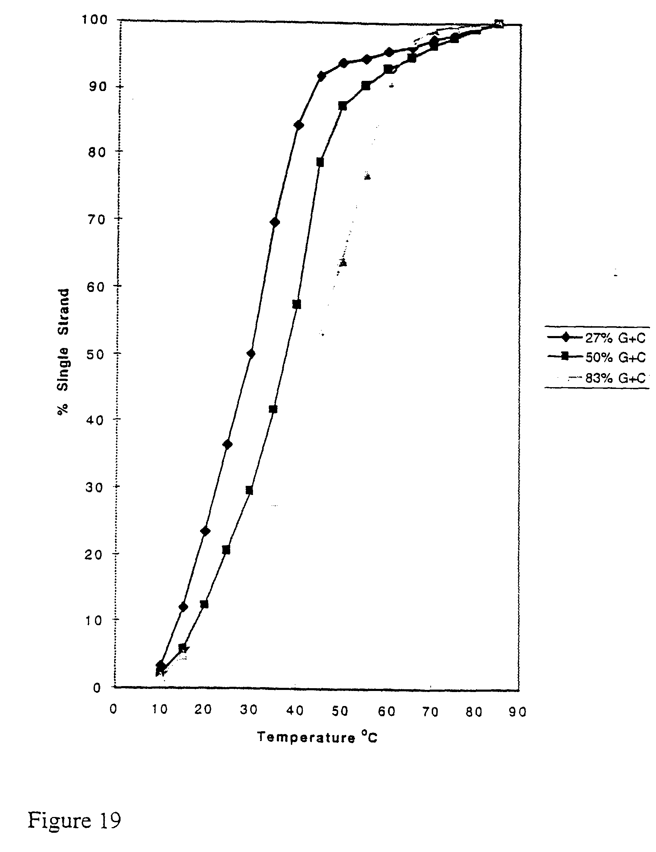

Figure 19 is a graph showing the difference in $T_d$ between three duplexes, that vary in G+C content from 27% to 83%. The percentage of single strand DNA (y-axis) is plotted versus temperature (°C; x-axis). The melting solution was 20% formamide, 10 mM Tris pH 7.6, and 5 mM EDTA with 0.1 % sarkosyl.

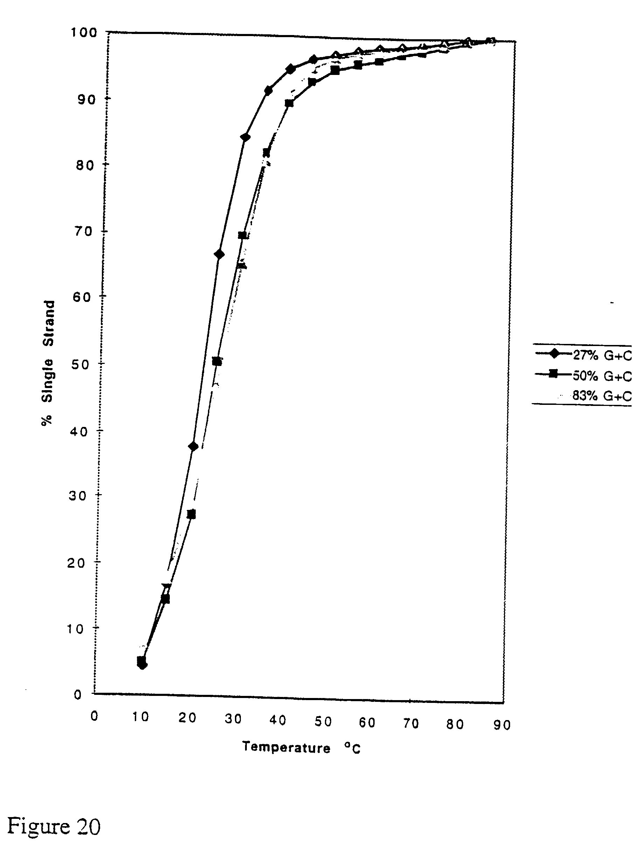

Figure 20 is a graph showing the difference in $T_d$ between three duplexes, that vary in G+C content from 27% to 83%. The percentage of single strand DNA (y-axis) is plotted versus temperature (°C; x-axis). The melting solution was 1 M dicyclohexylammonium acetate.

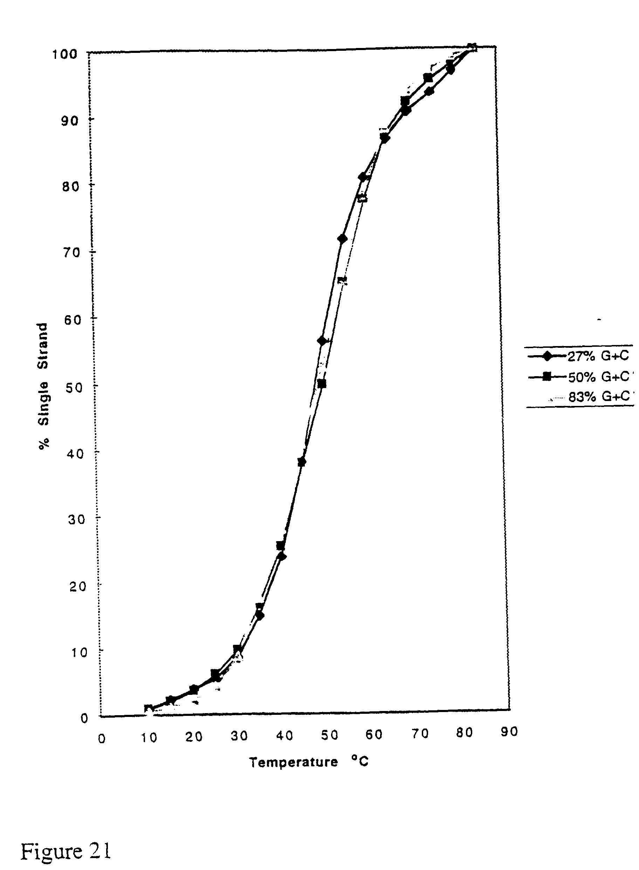

Figure 21 is a graph showing the difference in $T_d$ between three duplexes, that vary in G+C content from 27% to 83%. The percentage of single strand DNA (y-axis) is plotted versus temperature (°C; x-axis). The melting solution was 500 mM n-ethylbutylammmonium acetate.

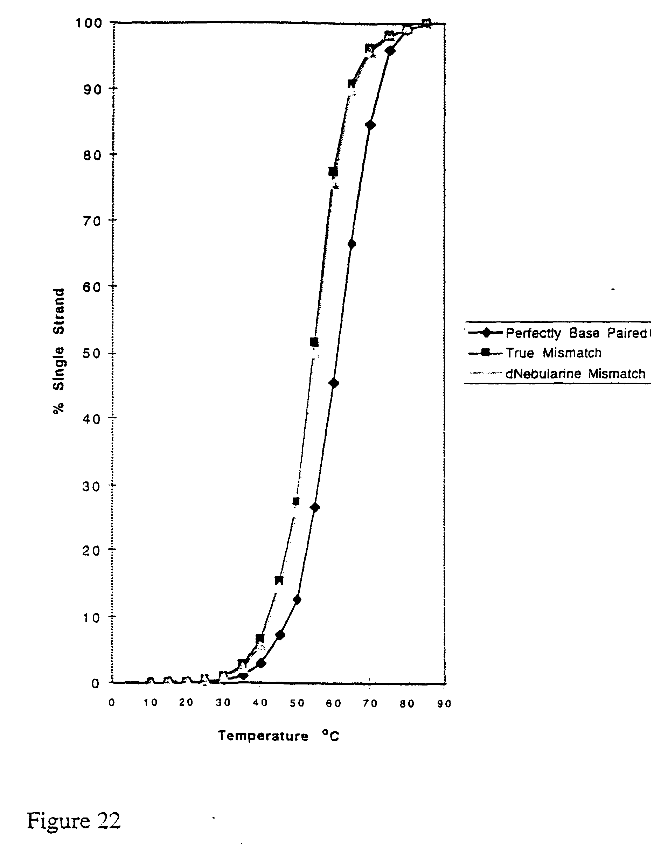

Figure 22 is a graph showing the difference in $T_d$ between three duplexes, one that is perfectly based-paired and the other two that contains a mismatch or a deoxynebularine substitution. The percentage of single strand DNA (y-axis) is plotted versus temperature (°C; x-axis). DMO-2060: 5'-hexylamine-GTC/ATA/CTC/CTG/CTT/GCT/GAT/CCA/CAT/CTG-3' (oligonucleotide immobilized on the nylon bead.; DMO-2055: 5'-TEXAS RED-TGT/GGA/TCA/GCA/AGC/AGG/AGT/ATG-3' (perfect complement); DMO-2058; 5'-TEXAS RED- TGT/GGA/TCA/GGA/AGC/AGG/AGT/ATG-3' (mismatch complement); and DMO-2058-dN: 5'-TEXAS RED-TGT/GGA/TCA/G(deoxynebularine)A/AGC/AGG/AGT/ATG-3' (deoxynebularine mismatch complement). The melting solution was 1 M diisopropylamine acetate. The maximum difference between the 3 melting curves in the Td or Tm is 6 C. The helical coil transition (HCT) of the true mismatch was 14 C; the HCT for the deoxynebularine mismatch duplex was 14 C and the HCT for the perfectly based paired duplex was 16 C.

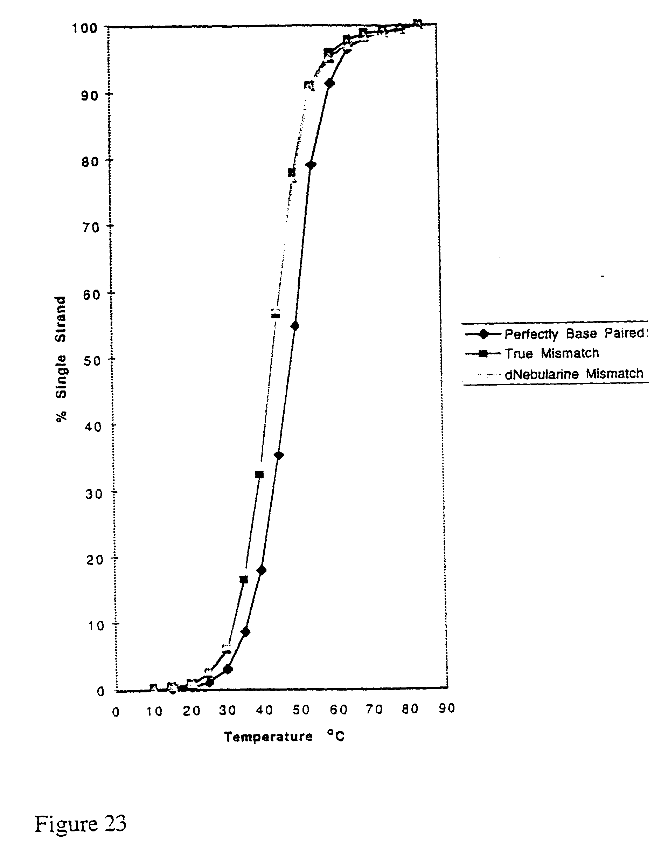

Figure 23 is a graph showing the difference in $T_d$ between three duplexes. one that is perfectly based-paired and the other two that contains a mismatch or a deoxynebularine substitution. The temperature difference between any two $T_d$s at $\alpha = 0.5$ is defined as the $\Delta T_d$. The percentage of single strand DNA (y-axis) is plotted versus temperature (°C; x-axis). DMO-2060: 5'-hexylamine-GTC/ATA/CTC/CTG/CTT/GCT/GAT/C CA/CAT/CTG-3' (oligonucleotide immobilized on the nylon bead.; DMO-2055: 5'-TEXAS RED-TGT/GGA/TCA/GCA/AGC/AGG/AGT/ATG-3' (perfect complement); DMO-2058; 5'-TEKAS RED- TGT/GGA/TCA/GGA/AGC/AGG/AGT/ATG-3' (mismatch complement): and DMO-2058-dN: 5'-TEXAS RED-TGT/GGA/TCA/G(deoxynebularine)A/AGC/AGG/AGT/ATG-3' (deoxynebularine mismatch complement). The melting solution was 1 M n.n-dicyclohexylamine acetate. The maximum difference between the 3 melting curves in the $T_d$ was 4 C. The helical coil transition (HCT) of the true mismatch was 15 C; the HCT for the deoxynebularine mismatch duplex was 15 C and the HCT for the perfectly based paired duplex was 15 C.

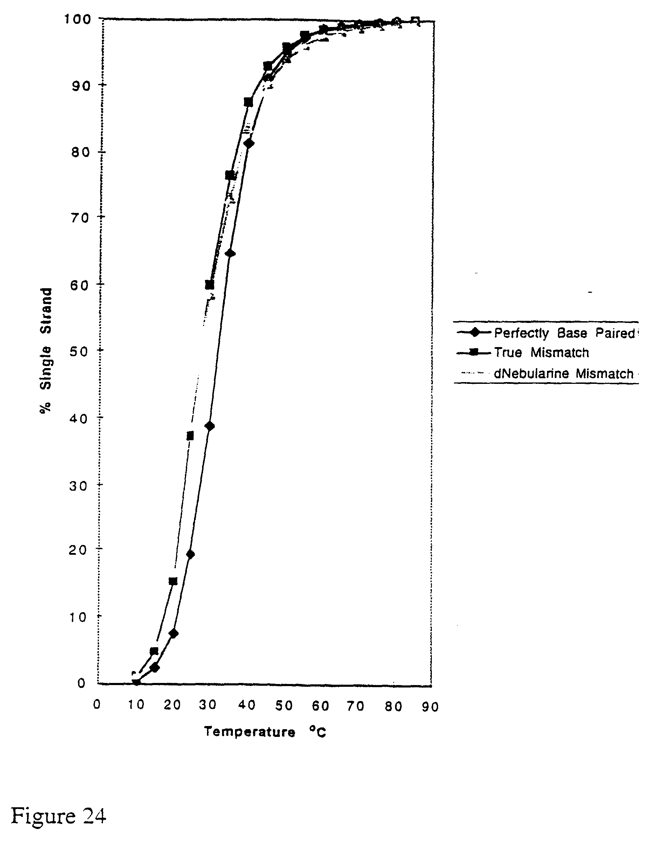

Figure 24 is a graph showing the difference in $T_d$ between three duplexes, one that is perfectly based-paired and the other two that contains a mismatch or a deoxynebularine substitution. The temperature difference between any two $T_d$s at $\alpha = 0.5$ is defined as the $\Delta T_d$. The percentage of single strand DNA (y-axis) is plotted versus temperature (°C; x-axis). DMO-2060: 5'-hexylamine-GTC/ATA/CTC/CTG/CTT/GCT/GAT/CCA/CAT/CTG-3' (oligonucleotide immobilized on the nylon bead.; DMO-2055: 5'-TEXAS RED-TGT/GGA/TCA/GCA/AGC/AGG/AGT/ATG-3' (perfect complement); DMO-2058; 5'-TEXAS RED- TGT/GGA/TCA/GGA/AGC/AGG/AGT/ATG-3' (mismatch complement); and DMO-2058-dN: 5'-TEXAS RED-TGT/GGA/TCA/G(deoxynebularine)A/AGC/AGG/AGT/ATG-3' (deoxynebularine mismatch complement). The melting solution was 1 M n,n-dicyclohexylamine acetate. The maximum difference between the 3 melting curves in the $T_d$ was 4 C. The helical coil transition (HCT) of the true mismatch was 17 C; the HCT for the deoxynebularine mismatch duplex was 17 C and the HCT for the perfectly based paired duplex was 15 C.

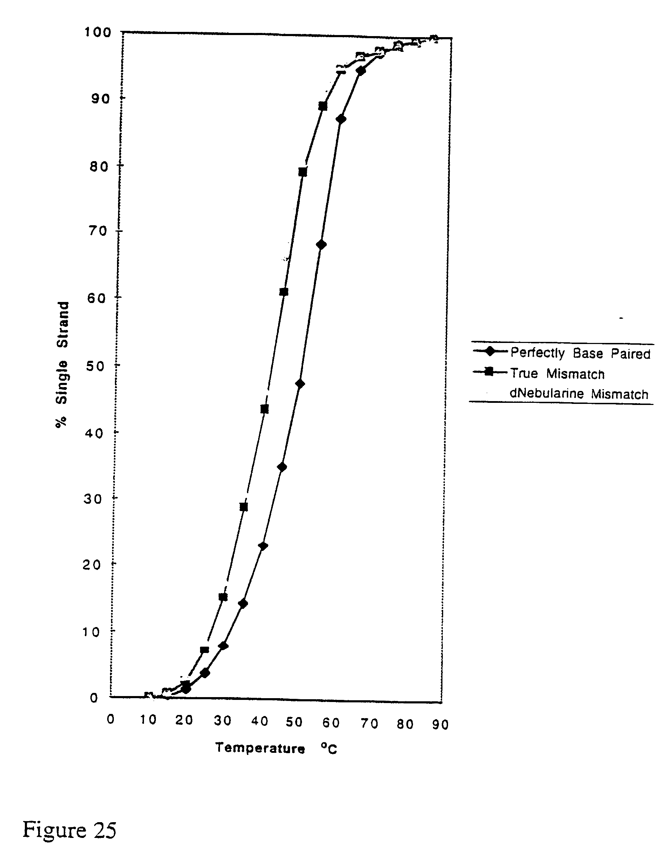

Figure 25 is a graph showing the difference in $T_d$ between three duplexes, one that is perfectly based-paired and the other two that contains a mismatch or a deoxynebularine substitution. The temperature difference between any two $T_d$s at a = 0.5 is defined as the $\Delta$-$T_d$. The percentage of single strand DNA (y-axis) is plotted versus temperature (°C; x-axis). DMO-2060: 5'-hexylamine-GTC/ATA/CTC/CTG/CTT/GCT/GAT/CCA/CAT/CTG-3' (oligonucleotide immobilized on the nylon bead.; DMO-2055: 5'-TEXAS RED-TGT/GGA/TCA/GCA/AGC/AGG/AGT/ATG-3' (perfect complement); DMO-2058; 5'-TEXAS RED- TGT/GGA/TCA/GGA/AGC/AGG/AGT/ATG-3' (mismatch complement); and DMO-2058-dN: 5'-TEXAS RED-TGT/GGA/TCA/G(deoxynebularine)A/AGC/AGG/AGT/ATG-3' (deoxynebularine mismatch complement). The melting solution was 100 mM n,n-dimethylhexylamine acetate. The minimum difference between the 3 melting curves in the $T_d$ was 9 C. The helical coil transition (HCT) of the true mismatch was 15 C; the HCT for the deoxynebularine mismatch duplex was 15 C and the HCT for the perfectly based paired duplex was 15 C.

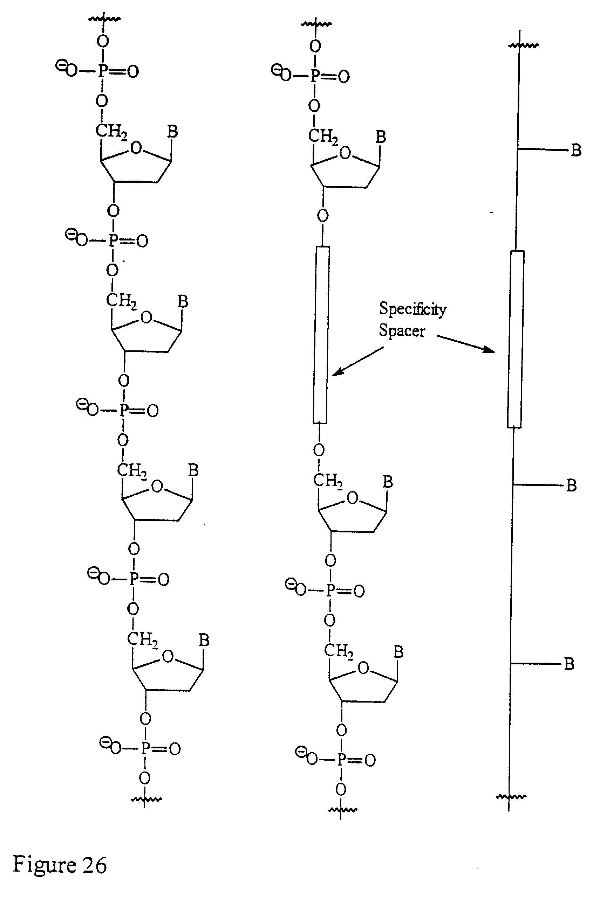

Figure 26 explains a convention used herein to denote oligonucleotides having a specificity spacer.

DETAILED DESCRIPTION OF THE INVENTION

Prior to setting forth the invention, it may be helpful to an understanding thereof to define certain terms used herein.

As used herein, "hybotrope" refers to any chemical or any mixture of a chemical in an aqueous or organic environment with buffers, chelators, salts and/or detergents that changes the enthalpy of a nucleic acid duplex by at least 20% when referenced to a standard salt solution (0.165 M NaCl, 0.01 M Tris pH 7.2, 5 mM EDTA and 0.1% SDS). That is, the energy content of the nucleic acid duples is decreased. The reference oligonucleotide is 5'-GTC/ATA/CTC/CTG/CTT/GCT/GAT/CCA/CAT/CTG-3' as the immobilized oligonucleotide and 5'-TGT/GGA/TCA/GCA/AGC/AGG/AGT/ATG-3' as the solution nucleotide which is typically labeled at the 5'-end with a fluorochrome such as Texas Red. The oligonucleotide duplex (24 nucleotides in length) has a helical to coil transition (HCT) of 25°C or less. The HCT is the difference between the temperatures at which 80% and 20% of the duplex is single stranded. The average minimum slope for a solution to be defined as a hybotrope is the first derivative of the HCT and is equal to 2.4 in units of 1/temperature in degrees C ((80% single strand - 20% single-strand)/25°C).

As used herein, "stringency" is the percentage of mismatched base pairs that are tolerated for hybridization under a given condition.

As used herein, "discrimination" is the difference in $T_d$ between a perfectly base-paired duplex and a duplex containing a mismatch.

As used herein, a "discrimination temperature" is a temperature at which a hybridization reaction is performed that allows detectable discrimination between a mismatched duplex and a perfectly matched duplex. As shown herein. a range of temperatures satisfy criteria of a discrimination temperature.

As used herein, an "abasic" residue in an oligonucleotide refers to a compound that approximates the length of a ribofuranose sugar, is covalently attached to neighboring bases (e.g., via phosphodiester or equivalent linkages), and is substituted at the beta anomeric position with a group that does not interact with the base on the opposite strand of a duplex. An abasic residue may be an apurine or apyrimidine structure, an anucleoside structure. or an analogue of a phosphate backbone. The abasic substitution may also consist of a backbone of N-(2-aminoethyl)-glycine linked.

As used herein, a "base analog" in an oligonucleotide refers to a compound that has a ribofuranase sugar and is substituted at the beta anomeric position with a group that has a similar 3-D shape as an A, C, G, T, or U base, but does not hydrogen bond to the base on the opposite strand of a duplex.

As used herein, "deoxyNebularine" refers to a 2'-deoxynubularine, which is 9-(beta-D-2'-deoxyribofuranosyl) purine (Eritja et al., *Nucl. Acids Res. 14*:8135, 1986). The molecular formula is $C_{10}H_{12}N_4O_4$.

As used herein, "nucleic acid" or "nucleic acid molecule" refers to any of deoxyribonucleic acids (DNAs), ribonucleic acids (RNAs), oligonucleotides, fragments generated by the polymerase chain reaction, and fragments generated by any of ligation, scission, endonuclease action, and exonuclease action. Nucleic acids can be composed of naturally occurring bases and analogs of naturally occurring bases, or a combination of both. Nucleic acids can be either single stranded or double stranded.

As used herein, "$T_m$" is the temperature at which half the molecules of a nucleic acid duplex are single strand-

ed. $T_m$ is measured in solution, while $T_d$ is measured for the duplex affixed to a solid support, both terms indicate the temperature at which half of a duplex are single stranded.

## A. HYBOTROPES

As noted above, the present invention provides compositions, including hybotropes, that can change the enthalpy of a nucleic acid duplex (*i.e.,* that can decrease the energy content of the oligonucleotide duplex, so that the cooperativity of the melting processes is increased, as discussed in more detail below). Generally, enthalpy of a duplex in a solution containing a hybotrope is increased at least 20%, and preferably, 30-100% over a duplex in a reference solution comprising 0.165M NaCl.

Several consequences flow from increased enthalpy. Importantly. the temperature range over which a duplex melts is decreased, likely due to increased cooperativity of melting. The difference between a hybrotropic solution and a hybridization solution used in most molecular biology protocols is illustrated in Figure 4 and Figure 5. In Figure 4, the difference in $T_d$ between a duplex containing a mismatch and duplex which is perfectly base-paired is about 5°C and is clearly distinguished. The hybotrope in Figure 4 is LiTCA. In Figure 5 the difference in $T_d$ between a duplex containing a mismatch and duplex which is perfectly base-paired is less than 2°C and is not distinct. Also, the HCT of the hybotrope in Figure 4 is less than 25°C and the HCT of the SSC-based solution is greater than 25°C.

Because the temperature range of a melt is smaller in a hybotropic solution, there is a greater difference in the $T_m$ of a perfectly complementary duplex and a duplex containing one or more mismatched base pairs (*e.g.,* base pairing other than A:T, G:C, A:U). This property is illustrated in Figure 3 in which an 18 mer duplex perfectly complement or containing a 1 bp mismatch is melted in a solution comprising a hybotrope. As shown, the difference in $T_d$ between the two duplexes is substantial. In general, a hybotrope causes an increase in $\Delta T_d$ of $\geq$°C (*e.g.,* $\geq$ 2°C, $\geq$ 2.5°C, $\geq$ 3°C, $\geq$ 3.5°C, $\geq$ 4°C) over the $\Delta T_d$ of the matched and mismatched duplexes in a reference solution (*e.g.,* 0.18M Na+). For a 6 to 18 base pair duplex (50% G+C) a hybotrope induces a $\Delta T_d$ of $\geq$ 2°C (e.g., $\geq$ 2°C, $\geq$ 2.5°C, $\geq$ 3°C, $\geq$ 3.5°C, $\geq$ 4°C, $\geq$ 4.5°C, $\geq$ 5°C), for a 19 to 24 base pair duplex, a hybotrope induces a $\Delta T_d$ of $\geq$ 1°C (*e.g.,* $\geq$ 1°C. $\geq$ 1.5°C, $\geq$ 2°C, $\geq$ 2.5°C, $\geq$ 3°C, $\geq$ 3.5°C, $\geq$ 4°C, $\geq$ 4.5°C, $\geq$ 5°C) and for a 25 to 36 base pair duplex, a hybotrope induces a $\Delta T_d$ of $\geq$ 0.5°C (*e.g.,* $\geq$ 0.5°C, $\geq$ 1°C, $\geq$ 1.5°C, $\geq$ 2°C, $\geq$ 2.5°C, $\geq$ 3°C, $\geq$ 3.5°C, $\geq$ 4°C, $\geq$ 4.5°C, $\geq$ 5°C).

The melting of a duplex causes a transition from a helical state (duplex) to a coil state (single stranded). The transition, called HCT (helical to coil transition) is readily measured and is expressed in units of temperature. As used herein, HCT is the temperature difference between which a duplex is 80% ($\alpha = 0.8$) and 20% ($\alpha = 0.2$) single-stranded.

A hybotrope may be identified as any chemical or any mixture of a chemical in an aqueous or organic environment with buffers, chelators, salts and/or detergents that decreases the enthalpy of a nucleic acid duplex by 20% when referenced to a standard salt solution (0.165 M NaCl, 0.01 M Tris pH 7.2, 5 mM EDTA and 0.1% SDS). The reference oligonucleotide is 5'-GTC/ATA/CTC/CTG/CTT/GCT/GAT/CCA/CAT/CTG-3' as the immobilized oligonucleotide and 5'-TGT/GGA/TCA/GCA/AGC/AGG/AGT/ATG-3' as the solution nucleotide which is typically labeled at the 5'-end with a fluorochrome such as Texas Red. The oligonucleotide duplex (24 nucleotides in length) has a helical to coil transition (HCT) of 25°C or less. The HCT is the difference between the temperatures at which 80% and 20% of the duplex is single stranded.

## 1. Relationship of Hybotrope to HCT

In Figure 1, the characteristic parameters of a thermal melting profile (helical coil transition) of an oligonucleotide duplex in two different hybridization solutions are presented. The squares represent the melting profile of an oligonucleotide duplex in NaCl based hybridization solution (*e.g.,* SSPE, SSC). 20xSSPE is 173.5g NaCl, 27.6g NaHPO4, and 7.4g EDTA at pH7.4 in 1L water. 20xSSC is 175.3g NaCl, 88.2g NaCitrate at pH 7 in 1L water. The diamonds represent the melting profile of the same oligonucleotide duplex in a hybotrope-based hybridization solution, in this case LiTCA (lithium trichloroacetate). $T_d$ is the temperature (°C) at which half of the molecules in a population are single-strand and half of the molecules are double-stranded. The HCT (helical coil transition) is the width of the melting curve from a value of 20% single-strand to 80% single-strand and possesses the unit of temperature (*e.g.,* °C, °K). The stringency factor is the value of the slope (partial derivative) of the helical coil transition at the $T_d$. Either stringency factor or HCT may be used to identify a hybotrope.

In Table 1, the slope (k) of the linear equation that relates concentration of solute to $T_d$, the helical coil transition, and the $\Delta T_d$ for 9 different hybotropic and hybridization solutions is presented. An 18 bp oligonucleotide duplex was melted in the respective solutions and the values are obtained as described in the examples.

Table 1

| Hybridization Solution Type | Slope (k) | HCT(°C) | Stringency Factor |
|---|---|---|---|
| LiTCA | 19 | 8 | 7.5 |
| GuSCN | 13 | 10 | 6.0 |
| NaSCN | 8.5 | 11 | 5.4 |
| NaClO$_4$ | 7 | 12 | 5.0 |
| KI | 5 | 15 | 4.0 |
| NaCl | 4.5 | 17.5 | 3.4 |
| GuCl | 3.5 | 18 | 3.3 |
| CsTFA | 2.5 | 18 | 3.3 |
| 30% formamide | ND* | 20 | 3.0 |

* = not determined

Thus, from these data, HCT is inversely proportional to the stringency factor for a given hybridization solution type; the lower the value of HCT, the higher the stringency factor. The HCT increases as the slope of the linear function that relates salt concentration to $T_d$ decreases (($T_d$[salt] = $T_d$[0] - k[Cx⁻]), where $T_d$[0] is the extrapolated $T_d$ at zero salt concentration, k is the salt specific constant and Cx⁻ is the concentration of the salt or hybotrope; *see* Figure 2).

A hybotrope may be identified as any chemical or any mixture of a chemical in an aqueous or organic environment with buffers, chelators, salts and/or detergents that decreases the enthalpy of a nucleic acid duplex by 20% when referenced to a standard salt solution (0.165 M NaCl, 0.01 M Tris pH 7.2, 5 mM EDTA and 0.1% SDS). The reference oligonucleotide is 5'-GTC/ATA/CTC/CTG/CTT/GCT/GAT/CCA/CAT/CTG-3' as the immobilized oligonucleotide and 5'-TGT/GGA/TCA/GCA/AGC/AGG/AGT/ATG-3' as the solution nucleotide which is typically labelled at the 5'-end with a fluorochrome such as Texas Red. The oligonucleotide duplex (24 nucleotides in length) has a helical to coil transition (HCT) of 25°C or less..

2. Relationship of HCT to Discrimination

Either stringency factor or HCT is related directly to another readily measurable parameter of oligonucleotide duplexes. This parameter, $\Delta T_d$, is the temperature difference between the $T_d$ of an oligonucleotide duplex that is perfectly base paired and the $T_d$ of the same oligonucleotide duplex that contains a mismatch at some position in the duplex (*see* Figure 3). As shown herein, the temperature difference between a perfectly base paired duplex and a duplex containing a mismatch is a function of the stringency factor (or HCT) of a given hybridization solution or hybotrope. The relationship is expressed as: $\Delta T_d$ increases as the stringency factor of a solution increases. In Table 2, this relationship is presented for 18 bp oligonucleotide duplexes. The duplex is melted in the respective hybridization solution and HCT and $\Delta T_d$ is determined as described herein.

Table 2

| Hybridization Solution Type | Slope (k) | HCT (°C) | $\Delta T_d$ (°C) |
|---|---|---|---|
| LiTCA | 19 | 8 | 7.5 |
| GuSCN | 13 | 10 | 6.0 |
| NaSCN | 8.5 | 11 | 5.5 |
| NaClO$_4$ | 7 | 12 | 4.5 |
| KI | 5 | 15 | 3.0 |
| NaCl | 4.5 | 17.5 | 1.5 |
| GuCl | 3.5 | 18 | 1.2 |
| CsTFA | 2.5 | 18 | 1.2 |

15

Table 2   (continued)

| Hybridization Solution Type | Slope (k) | HCT (°C) | $\Delta T_d$ (°C) |
|---|---|---|---|
| 30% formamide | ND* | 20 | 1.5 |

\* = not determined

The data presented in Table 2 show that HCT is inversely proportional to the $\Delta T_d$ between a perfectly base paired duplex and a duplex containing a mismatch. That is, either stringency factor or HCT predicts the ability of given hybridization solution to discriminate mismatched duplexes. This aspect of hybotrope-based hybridization is further illustrated in Figures 4 and 5. Figure 4 is a graph showing melting profiles in 2.0 M LiTCA for an 18-mer oligonucleotide duplex that is perfectly based paired (diamonds) and the same oligonucleotide duplex that contains a central mismatch (A/A, position 9). The $\Delta T_d$ is 6°C. Figure 5 is a graph showing melting profiles for an 18-mer oligonucleotide duplex in QY low stringency hybridization buffer (Promega, Madison, WI) that is perfectly based paired (squares) and the same oligonucleotide duplex that contains a central mismatch (A/A, position 9). The $\Delta T_d$ is 0°C. Therefore, the $\Delta T_d$ value relates to the ability of a chemical to discriminate between perfectly base paired duplexes and duplexes that contain a mismatch. The practical utility of this result is discussed below.

In addition, transition enthalpies between a fully base-paired and base stacked double helix to two unpaired and unstacked single strands can be calculated. (Breslauer, K.J.. Chapter 15, "Methods for Obtaining Thermodynamic Data on Oligonucleotide Transitions," in *Thermodynamic Data for Biochemistry and Biotechnology,* ed. H. Hinz, Academic Press, New York, NY, 1986.) The difference between a non-cooperative and cooperative transition is expressed in terms of $\Delta H_{vH}$ (van't Hoff enthalpy). In a cooperative transition, the value of $(d\alpha/dT)T_d$ is high. and therefore, the $\Delta H_{vH}$ is also high. In a non-cooperative transition, the value of $(d\alpha/dT)T_d$ is low, and therefore, the $\Delta H_{vH}$ is also low. (The term $(d\alpha/dT)T_d$ is the derivative of the slope of the melting curve at the $T_d$, $\alpha$ is defined as the % single strand on the ordinate axis.)

In this regard, thermodynamic parameters for two different sets of oligonucleotides (42% G+C; 63% G+C) in three types of hybridization solution are shown in Table 3. The data show that the enthalpy values are inversely related to the values obtained for the temperature range of the thermal coil transition of the duplex (HCT).

Table 3

| Solution Type | % G+C | Length (nt) | $T_d$(°C) | HCT(°C) | $\Delta H_{vH}$ (kcal/mol) |
|---|---|---|---|---|---|
| 2 M LiTCA | 42 | 19 nt | 35.5 | 12 | -52.8 |
| 2 M TMATCA | 42 | 19 | 55.4 | 18 | -47.0 |
| 3 M TMATCA | 42 | 19 | 43.0 | 11.5 | -60.7 |
| 3 M TMACl | 42 | 19 | 60.0 | 15.5 | -46.2 |
| 2 M LiTCA | 63 | 19 | 42.0 | 15 | -42.0 |
| 2 M TMATCA | 63 | 19 | 48.0 | 19.5 | -38.6 |
| 3 M TMATCA | 63 | 19 | 47.0 | 13 | -61.8 |
| 3 M TMACl | 63 | 19 | 59.0 | 17.5 | -39.7 |

3. Characterization of a Hybotrope

a. Characteristics of a hybotrope.

As noted herein, a hybotrope is useful within the context of the present invention if it is a solution or is miscible from about 0.05 M to about 10 M in water, other protic, or aprotic solvent. In certain preferred embodiments, the hybotrope does not inactivate polymerases. In other preferred embodiments, the anion part of a hybotrope has a $pK_1$ of less than 2.2.

The chaotrope is a chemical that increases the enthalpy of an oligonucleotide or nucleic acid duplex by at least 20% when referenced to a standard salt solution (*i.e.,* 0.165 M NaCl). Enthalpy is measured by plotting the slope of the thermal transition, $\alpha$, versus temperature (see Figure 1) and applying the following:

The van't Hoff enthalpy can be obtained from the differentiated equilibrium melting curve (Marky and Breslauer. 1987) by plotting $d\alpha$ versus temperature. Briefly, thermodynamic data provide a basis for predicting the stability ($\Delta G'$) and temperature- dependent melting behavior (also described here as the helical coil transition (HCT), ($\Delta H°$)) from the

primary sequence of bases in the duplex. We use a thermally induced helical coil transition (from double strand to single strand) to obtain values for the $\Delta H_{vH}$. The analysis of the shape of the helical coil transition is used to calculate the van't Hoff transition enthalpy. As described by Marky and Breslauer, (1987), $\alpha$ is equal to the fraction of single strands in the duplex state. If $\alpha$ is plotted versus temperature the temperature at which $\alpha$ takes the value of 0.5 is defined as the $T_d$. The equilibrium constant K for any transition can be expressed in the form of $\alpha$, the van't Hoff enthalpy can be expressed as:

$$\Delta - H_{vH} = RT2[d\ln K/dT] \text{ or } \Delta - H_{vH} = -R[d\ln K/d(1/T)]$$

To solve the general expression when $\alpha$ takes the value of 0.5 in terms of $\alpha$ the foregoing equation is differentiated and solved for $\alpha$ at the $T_d$:

$\Delta - H_{vH} = (2 + 2n)RT2(\partial\alpha/\partial T)_{T-Td}$ which can also be written:
$\Delta - H_{vH} = (2 + 2n)R(\partial\alpha/\partial(1/T))_{T-Td}$

In this series of experiments it is assumed that a bi-molecularity exists where n=2 for the preceding equations and therefore the corresponding coefficient is equal to 6. Another assumption employed is that there is no dependence of $T_d$ on concentration since at every temperature increment the concentration of single strands is zero (recall that all unhybridized material is washed away from the solid support prior to the melting process and that at each 5°C temperature increment, the solid support is placed in a fresh solution). For any process at equilibrium, $\Delta G = -RT(\ln Keq)$ and $\Delta G = \Delta H - T\Delta S$ it is possible to write $-RT (\ln K) = \Delta H - T\Delta S$.
As has been shown by Gralla and Crothers (Gralla, J., and Crothers, D.M., *J Mol. Biol. 73*:497-511, 1973) for bimolecular transitions, the full width or half-width of a differentiated melt curve at the half-height is inversely proportional to the van't Hoff transition enthalpy. As suggested, for an equilibrium of the form $nA \Leftrightarrow A_n$ the general forms of the van't Hoff equation are:

$\Delta - H_{vH} = B/((1/T_1)-(1/T_2))$ (for the full width at half-height)
$\Delta - H_{vH} = B'/((1/T_{max})-(1/T_2))$ (for the upper half-width at half-height)

where $T_{max}$ is the temperature at the maximum, and $T_1$ and $T_2$ correspond to the upper and lower temperatures at which value the change in the plotted temperature is equal to one-half of $[(\partial\alpha/\partial(1/T))_{max}]$. For a molecularity of 2, $-B = 10.14$ and $-B'= 4.38$. The detailed derivations are given in Marky and Breslauer, (1987). This approach of measuring the van't Hoff enthalpies is particularly amenable to melting duplexes off solid supports as all problems associated with baselines and background are completely eliminated.
The equilibrium constant K for a helical transition of a molecularity of 2 can be expressed as the extent of a (the fraction of single strand molecules in a duplex). The value of K is usually determined at the $T_m$ of the helical coil transition where $\alpha = 0.5$. This value of the $T_m$ is then extrapolated to some reference temperature (*e.g.*, 298K) using the empirically determined $T_m$ (or $T_d$) and the calculated van't Hoff enthalpy (assumed to be temperature independent) and the integrated form of the van't Hoff equation:

$$\ln[K(Tm)/K(T_{ref})] = \Delta H^0/R(1/T-1/Tm)$$

From the empirically determined value of $K(T_{ref})$, it is possible to determine $\Delta G^0$ for the helical coil transition using the relation $\Delta G^0 = \Delta H^0 - T\Delta S^0$. Since the melting curves described here are concentration independent, the ln $(K_{Tm}) = 0$ since K = 1 at the $T_m$. Therefore the van't Hoff equation reduces to:

$$-\ln K(T) = \Delta H^0/R(1/T-1/T_m),$$

which upon multiplying both sides by RT, provides

$$-RT \ln K(T) = \Delta H^0(1-T/T_m) = \Delta G^0$$

This expression can be used to calculate the transition free energy $\Delta G^0$ at any temperature of interest (T) from the experimentally measured values of $T_m$ and $\Delta H_{vH}$. The corresponding $\Delta S^0$ can be calculated from relation $\Delta G^0$

$= \Delta H^0 - T\Delta S^0$.

As a result of reducing the HCT, a hybotrope increases the stringency factor of a hybridization solution or solvent, where the stringency factor is the value of the slope (partial derivative) of the helical coil transition at the value of the $T_m$. As discussed above, the stringency factor can be used to identify a hybotrope.

A hybotrope is generally soluble or miscible in water, polar, apolar or organic solvent from about 0.05 to 10 M, or a hybotrope can be composed solely of a polar, apolar or organic solvent.

b. Structure of hybotropes.

The term "hybotrope" refers to any chemical or any mixture of a chemical in an aqueous or organic environment with buffers, chelators, salts and/or detergents that changes the enthalpy of a nucleic acid duplex by at least 20% when referenced to a standard salt solution (0.165 M NaCl, 0.01 M Tris pH 7.2, 5 mM EDTA and 0.1% SDS). That is. the energy content of the nucleic acid duples is decreased. The reference oligonucleotide is 5'-GTC/ATA/CTC/CTG/CTT/GCT/GAT/CCA/CAT/CTG-3' as the immobilized oligonucleotide and 5'-TGT/GGA/TCA/GCA/AGC/AGG/AGT/ATG-3' as the solution nucleotide which is typically labeled at the 5'-end with a fluorochrome such as Texas Red. The oligonucleotide duplex (24 nucleotides in length) has a helical to coil transition (HCT) of 25°C or less. The HCT is the difference between the temperatures at which 80% and 20% of the duplex is single stranded. The average minimum slope for a solution to be defined as a hybotrope is the first derivative of the HCT and is equal to 2.4 in units of 1/temperature in degrees C ((80% single strand - 20% single-strand)/25°C).

The hybotrope may be a salt selected from LiTCA, RbTCA, GuSCN, NaSCN, $NaClO_4$, KI, TMATCA TEATCA, TMATBA, TMTCA, TMTBA, TBATCA or TBATBA.

Preferred hybotropes are a salt formed of an anion and a cation, where the anion is selected from acetate, propionate and halogenated versions thereof. The halogen of the halogenated anion is selected from fluorine, chlorine, bromine and iodine, but is preferably fluorine and/or chlorine. The halogenated anion may contain as few as one and as many as three halogen atoms for halogenated acetate. The halogenated propionate may contain as few as one or as many as five halogen atoms. Trichloroacetate and trifluoroacetate are two prefered anions.

The cation is preferably an ammonium ion, not including $NH_4$. Thus, the cation is a primary, secondary or tertiary ammonium comprising 1-36 carbon atoms, or a quaternary ammonium comprising 4-48 carbon atoms. Preferably, the cation is formed from atoms selected from 2-20 carbon atoms, 0-5 oxygen atoms and 1-5 nitrogen atoms. Thus, the cation substituents, where the groups bonded to the central nitrogen of the ammonium ion are called the "cation substituents" may contain ester, ether, hydroxyl, amine and amide functionality. Preferably, the cation substiuents are hydrocarbyl groups, i.e., groups formed entirely of carbon and hydrogen. where hydrocarbyl groups may be saturated or unsaturated, and the carbon atoms of a hydrocarbyl group may be linear, branched or arranged in a cyclic fashion.

A preferred ammonium ion is a quaternary ion of the structure $N(R)_4$ wherein R is a $C_1$-$C_{12}$hydrocarbyl and any two R groups may join together to form a cyclic structure with the nitrogen atom. The phrase "any two R groups may join together to form a cyclic structure with the nitrogen atom" means that the ammonium ion may be heterocyclic in that the central nitrogen atom is part of a cyclic structure. For example, the central nitrogen atom may be the nitrogen atom in piperidine, where this nitrogen atom is also bonded to other R groups. Preferred R groups for the quaternary ammonium ion are independently selected from $C_1$-$C_{12}$alkyl, $C_3$-$C_{12}$cycloalkyl and $C_7$-$C_{12}$arylalkyl.

Another preferred ammonium ion is a tertiary ion of the structure $HN(R)_3$ wherein R is a $C_1$-$C_{12}$hydrocarbyl and any two R groups may join together to form a cyclic structure with the nitrogen atom. Again, preferred R groups for the tertiary ammonim are are independently selected from $C_1$-$C_{12}$alkyl, $C_3$-$C_{12}$cycloalkyl and $C_7$-$C_{12}$arylalkyl.

Yet another preferred ammonium ion is a secondary ion of the structure $N(H)_2(R)_2$ wherein R is a $C_1$-$C_{12}$hydrocarbyl and the two R groups may join together to form a cyclic structure with the nitrogen atom. Again, preferred R groups for the tertiary ammonim are are independently selected from $C_1$-$C_{12}$alkyl, $C_3$-$C_{12}$cycloalkyl and $C_7$-$C_{12}$arylalkyl.

Suitable salts include, without limitation, those containing an ammonium cations selected from ethylbutylammonium, 1-methylimidizole, 1-methylpiperidine, 1-methylpyrrolidine, 3-methoxypropylamine, triethylamine, bis (2-methoxyethyl)amine, diallylamine, dibutylamine, diisobutylamine, N,N-dimethylaminobutane, N,N-dimethylclyclohexylamine, N,N-dimethylheptylamine, N,N-dimethylhexylamine, triethanolamine, 1-ethylpiperidine, dicyclohexylamine, diisopropylamine, dipropylamine, N,N-dimethylisopropylamine, N-ethylbutylamine, tetraethylamonium, tripropylamine, 2-methoxyethylamine, and N,N-dimethyloctylamine, and the anion is selected from acetate, trichloroacetate and trifluoroacetate.

As used herein, the following terms have the indicated meanings.

Alkyl refers to an aliphatic hydrocarbon radical, $-(CH_2)_nCH_3$, either branched or unbranched such as methyl, ethyl, N-propyl, *iso*-propyl, *N*-butyl, *iso*-butyl, *sec*-butyl, *tert*-butyl, dodecyl or the like.

Aryl refers to a radical derived from an aromatic hydrocarbon by removal of one hydrogen atom such as

phenyl, $\alpha$-naphthoyl, $\beta$-naphthyl, biphenyl, anthryl and the like.

Arylalkyl, -$(CN_2)_n$-Ar, refers to an alkyl radical as defined above joined to an aryl radical.

Hydroxyalkyl refers to a radical -$(CH_2)_nOH$.

Cycloalkane or cycloalkyl refers to a radical of a saturated hydrocarbon in a ring structure such as cyclopropyl, cyclobutyl, cyclopentyl. cyclohexyl, cycloheptyl, adamantyl and the like.

Unless otherwise stated, all number ranges are inclusive of the stated range (e.g., 1 to 5 carbons, includes to and 5 carbons).

Halogen refers to chlorine, bromine, iodine or fluorine.

c. Novel hybotropes.

Some of the hybotropes disclosed herein form novel hybridization solutions that improve the specificity of oligonucleotide probes. For example, tetramethylammonium trichloroacetate (TMATCA) and tetraethylammonium trichloroacetate (TEATCA) confer a high level of hybridization stringency. Moreover, these hybotropes neutralize G+C content influence $T_d$. In the Examples, random oligonucleotide probes (all 19-mers) differing in G+C content from 25% to 73% are shown to possess a $T_d$ within 5°C of each other in the presence of TMATCA (see Figure 6); the average $T_d$ in 3 M TMATCA was 45°C. Similar results are obtained with TEATCA (see Figure 7). As a control, the $T_d$s of these 19 mers were determined in 3 M TEACl. The resulting differences in $T_d$ was 6°C and the average $T_d$ of the 6 oligonucleotides was about 62°C. Furthermore, in 30% formamide, the 6 oligonucleotide probes differed in $T_d$ by 15°C; in 0.165 M NaCl, the range in $T_d$ values was 15°C (see Figure 8); and in 2 M LiTCA, the difference in $T_d$ was about 10°C. Most significantly, however, the HCT in TMATCA ranges from 8°C for the 25% G+C content oligo to 14°C for the 73% G+C oligonucleotide. In contrast, the HCT in TMACl ranges from 12.5°C for the 25% G+C content oligo to 17.5°C for the 73% G+C oligonucleotide. This 4°C to 5°C shift in the HCT of the oligos in the presence of TMATCA results in a significant improvement in the stringency factor of TMATCA compared to TMACl. Thus, TMATCA is a significantly better salt than any previously described solution for conducting oligonucleotide-based assays.

Novel hybridization solutions have also been identified which neutralize the effects of G+C content on the melting behaviour of nucleic acid duplexes. These solutions are in some cases hybotropes and in other cases can be used as PCR buffers or as hubridization solutions which minimize the effects of G+C content on nucleic acid duplexes. These new hybridization solutions, their properties, and their preparation are described in Example 12. Figure 14 is a graph showing the difference in $T_d$ between three duplexes. that vary in G+C content from 27% to 83%. The capture oligonucleotide is a 36-mer (DMO-GC36cap: 5'- hexylamine-GCA/GCC/TCG/CGG/AGG/CGGI/ATG/ATC/GTC/ATT/AGT/ATT-3') and three complementary oligos which are labelled with the fluorochrome are DMO-83GC: 5°-Texas Red-CCG/CCT/CCG/CGA/GGC/TGC-3'; DMO-50GC: 5'-Texas Red-AAT/GAC/GAT/CAT/CCG/CCT-3'; DMO-27GC: -Texas Red-AAT/ACT/AAT/GAC/GAT/CAT-3'. The temperature difference between any two $T_d$s at $\alpha = 0.5$ is defined as the $\Delta T_d$. The percentage of single strand DNA (y-axis) is plotted versus temperature (°C; x-axis). The melting solution was 100 mM 2-methoxyethylamine trifluoroacetate. The maximum difference between the 3 melting curves in the $T_d$ was 6 C. The helical coil transition of the 27% G+C content was 21 C, 50% G+C was 33 C and for the 83% G+C duplex was 29 C. Note that the helical coil transitions (HCTs) of the 3 different G+C content oligonucleotides is different. This is in contrast to the case with diisobutylamine as shown in Figure 15. Figure 15 is a graph showing the difference in $T_d$ between three duplexes, that vary in G+C content from 27% to 83% (the same system as described in Figure 14. The temperature difference between any two $T_d$s at a = 0.5 is defined as the $\Delta T_d$. The percentage of single strand DNA (y-axis) is plotted versus temperature (°C; x-axis). The melting solution was 100 mM diisobutylamine acetate. The maximum difference between the 3 melting curves in the $T_d$ was 5 C. The helical coil transition of the 27% G+C content was 22 C, 50% G+C was 26 C and for the 83% G+C duplex was 25 C. The helical coil transitions for the three oligonucletide duplexes are very similar. This is the behaviour that is preferred for use in array hybridizations or polymerase chain reactions.

In Figure 16 the inability of GuSCN to neutralize G+C content is shown. Figure 16 is a graph showing the difference in $T_d$ between three duplexes, that vary in G+C content from 27% to 83% (the same capture and probe oligonucleotides as described in figure 14). The temperature difference between any two $T_d$s at a = 0.5 is defined as the ATd. The percentage of single strand DNA (y-axis) is plotted versus temperature (°C; x-axis). The melting solution was 2 M Guanidinium thiocyanate. The maximum difference between the 3 melting curves in the $T_d$ was 16 C. The helical coil transition of the 27% G+C content was 28 C, for the 50% G+C duplex was 30 C and for the 83% G+C duplex was 32 C. Similar results were obtained with lx PCR buffer (Figure 17) and 1x SSC buffer (Figure 18). There was also no neutralization of G+C content with 20% formamide (Figure 19).

In contrast to the situation in Figures 17, 18 and 19, Figure 20 shows the melting behaviour of the 3 different G+C oligonucleotide duplexes in 1 M dicyclohexylamine acetate. Figure 20 is a graph showing the difference in $T_d$ between three duplexes, that vary in G+C content from 27% to 83% (same duplexes as described in Figure 14). The temperature difference between any two $T_d$s at $\alpha = 0.5$ is defined as the $\Delta T_d$. The percentage of single strand DNA (y-axis) is plotted versus temperature (°C; x-axis). The melting solution was 1 M dicyclohexylamine acetate. The maximum difference between the 3 melting curves in the $T_d$ or $T_m$ is 3 C. The helical coil transition of the 27% G+C content was 13 C. for the 50% G+C duplex was 17 C and for the 83% G+C duplex was 19 C. This is an ideal profile for a hybrotrope. In contrast the the narrow helical coil transition observed in Figure 20, a much wider HCT is observed with 500 mM n-ethylbutylamine acetate. Figure 21 is a graph showing the difference in $T_d$ between three duplexes, that vary in G+C content from 27% to 83% (the identical duplex system as described in Figure 14). The temperature difference between any two $T_d$s at a = 0.5 is defined as the $\Delta T_d$. The percentage of single strand DNA (y-axis) is plotted versus temperature (°C; x-axis). The melting solution was 500 mM n-ethylbutylamine acetate. The maximum difference between the 3 melting curves in the $T_d$ is 1 C. The helical coil transition of the 27% G+C content was 22 C, for the 50% G+C duplex was 22 C and for the 83% G+C duplex was 26 C.

The ability of some of the G+C neutralizing buffer to act as hybrotropes is illustrated in Figure 22. Figure 22 is a graph showing the difference in $T_d$ between three duplexes, one that is perfectly based-paired and the other two that contains a mismatch or a deoxynebularine substitution. The temperature difference between any two $T_d$s at a = 0.5 is defined as the $\Delta T_d$. The percentage of single strand DNA (y-axis) is plotted versus temperature (°C; x-axis). DMO-2060: 5'-hexylamine-GTC/ATA/CTC/CTG/CTT/GCT/GAT/CCA/CAT/CTG-3' (oligonucleotide immobilized on the nylon bead.; DMO-2055: 5'-TEXAS RED-TGT/GGA/TCA/GCA/AGC/AGG/AGT/ATG-3' (perfect complement); DMO-2058; 5'-TEXAS RED- TGT/GGA/TCA/GGA/AGC/AGG/AGT/ATG-3' (mismatch complement); and DMO-2058-dN: 5'-TEXAS RED-TGT/GGA/TCA/G(deoxynebularine)A/AGC/AGG/AGT/ATG-3' (deoxynebularine mismatch complement). The melting solution was 1 M diisopropylamine acetate. The maximum difference between the 3 melting curves in the $T_d$ is 6 C. The helical coil transition (HCT) of the true mismatch was 14 C; the HCT for the deoxynebularine mismatch duplex was 14 C and the HCT for the perfectly based paired duplex was 16 C. The same situation was observed for 1 M diisopropylamine acetate (Figure 22), 1 M n,n-dimethylcyclohexylamine acetate (Figure 23) and 1 M dicyclohexylamine acetate (Figure 24) and N,N-dimethylhexylamine acetate (Figure 25).

Preferred hybotropes of the present invention include, without limitation, bis(2-methoxyethyl)amine acetate. 1-ethylpiperidine acetate, 1-ethylpiperidine trichloroacetate, 1-ethylpiperidine trifluoroacetate, 1-methylimidizole acetate, 1-methylpiperidine acetate, 1-methylpiperidine trichloroacetate, 1-methylpyrrolidine acetate, 1-methylpyrrolidine trichloroacetate, 1-methylpyrrolidine trifluoroacetate, 2-methoxyethylamine acetate, N,N-dimethylcydohexylmnine acetate, N,N-dimethylcyclohexylarnine trifluoroacetate, N,N-dimethylcyclohexylamine, N,N-dimethylheptylamine acetate, N,N-dimethylheptylamine acetate, N,N-dimethylhexylamine acetate, N,N-dimethylhexylamine acetate, N,N-dimethylisopropylamine acetate, N-ethylbutylamine acetate, N-ethylbutylamine trifluoroacetate, N,N-dimethylardnobutane trichloroacetate, N,N-dimethylisopropyiamine trichloroacetate, triethanolamine acetate, triethylamine acetate, triethylamine trichloroacetate, tripropylamine acetate, tetraethylammonium acetate. These compounds or chemicals can be combined with buffers, chelating agents and/or detergents.

d. Effect of hybotrope concentration on discrimination.

As shown herein, the discrimination between mismatched oligonucleotides (mutant abbreviated as "mt") and perfectly based-paired oligonucleotides (abbreviated as "wt") is not a function of concentration of a particular hybotrope but rather a function of hybotrope type. Surprisingly, the HCT for the hybotropes LiTCA, GuSCN, GuHCl, and NaClO$_4$ does not change over about the range of about 0.5 M to about 6.0 M. Moreover, the slope of the mt duplex is always observed to be greater than for wt duplexes (see Figure 9). Furthermore, the difference between the $T_m$ of the wt duplex and the mutant duplex ($\Delta T_d$) is not affected by the concentration of the hybotrope. However, the $T_d$ is directly proportional to concentration (see Figure 10). Because $\Delta T_d$ does not change over a wide concentration range for the hybotropic solutions, a wide temperature range can be employed for conducting oligonucleotide-based assays (e.g., 20°C to 80°C). Second, relatively low concentrations (e.g., 0.5 M) of hybotrope may be employed in hybridization assays. including polymerase catalyzed reactions.

The approximate concentration range at which a solution of a compound (such as LiTCA) exhibits the characteristics of a hybotropic solution is approximately 0.2 to 0.4 M (Figure 11; Examples).

e. Effect of length of duplex.

The length of an oligonucleotide probe (i.e., resultant duplex) has the effect of increasing the $T_m$ as length increases. Due to this relationship, discrimination using a hybotrope is effectively limited to hybridization lengths of 6-40 bases and preferably 6-30 bases.

f. Assays for determining if a compound is hybotropic.

As discussed above, a hybotrope is a chemical that can increase the enthalpy of a nucleic acid duplex by 20% or more when referenced to a standard salt solution. A convenient assay for measuring this increased enthalpy is a thermal transition assay. A hybotrope may be identified as any chemical or any mixture of a chemical in an aqueous or organic environment with buffers, chelators, salts and/or detergents that decrease the enthalpy of a nucleic acid duplex by 20% when referenced to a standard salt solution (0.165 M NaCl, 0.01 M Tris pH 7.2, 5 mM EDTA and 0.1% SDS). The reference oligonucleotide is 5'-GTC/ATA/CTC/CTG/CTT/GCT/GAT/CCA/CAT/CTG-3' as the immobilized oligonucleotide and 5'-TGT/GGA/TCA/GCA/AGC/AGG/AGT/ATG-3' as the solution nucleotide which is typically labelled at the 5'-end with a fluorochrome such as Texas Red. The oligonucleotide duplex (24 nucleotides in length) has a helical to coil transition (HCT) of 25°C or less." Moreover, a suitable hybotrope is soluble in water, other protic solvent or aprotic solvent. Although not required, a hybotrope preferably does not inactivate polymerases when in they are with polymerases and the like in PCR reactions (and the like). Assays for these properties are briefly discussed below.

HCT of an 18-24 mer with a 50% G+C content are readily measured for a given solution. Briefly, an 18-24 mer oligonucleotide and its complement with a 50% G+C are synthesized. The oligonucleotides are dissolved to 2 μM in the candidate hybotrope solution. The mixture is heated to 85°C (at 0.5°C/min) and then cooled to 10-15°C to allow hybridization. Absorbance versus time is recorded at 260 nm by a UV-VIS spectrophotometer equipped with a thermal programmer. The HCT is determined from a plot of normalized absorbance values (fully annealed = 0% single strand; fully denatured = 100% single strand) versus temperature. A solution in which the temperature difference between 80% and 20% single stranded (HCT) is ≤ 25°C is a suitable hybotropic solution within the context of this invention.

Solubility may be measured by making a saturated solution with the respective salt, filtering off undissolved salt, removing the liquid or aqueous material and then determining the weight of the remaining salt.

pK values are measured using standard titration methods.

Polymerase activity in a hybotropic solution may be measured according to the use of the polymerase. For example, in amplification reactions, duplicate reactions with and without the hybotrope are run. The hybotrope does not inactivate the enzyme if 10% of activity is retained.

## B. ABASIC, ANUCLEOSIDIC ANALOGS AND DEOXY NEBULARINE RESIDUES

As described herein, an increase in specificity of priming or probing when using synthetic oligonucleotides is accomplished by minimizing the helical coil transition of the respective primer duplex, thereby increasing the stringency factor of the respective sequence. An increased stringency factor of an oligonucleotide decreases the stability of a mismatch and therefore promotes a high fidelity hybridization. Furthermore, increasing the stringency factor or decreasing HCT may also result in an increase in the specificity of priming. One way to increase stringency is to introduce one or more abasic anucleosidic or deoxynebularine residues into one strand of a duplex. Thus, introducing one of these residues leads to a "base pair" that is not hydrogen bonded. In effect, this is analogous to a mismatch and will decrease the $T_d$ and HCT of the respective derived oligonucleotide compared to a perfectly base-paired oligonucleotide that has the same sequence. Although, for the sake of simplicity the oligonucleotides in the examples below incorporate only one type of these residues at a time, combinations such as an abasic and an anucleosidic residue may be utilized.

As noted above, an abasic residue is a compound that approximates the length of a ribofuranose sugar, is covalently attached to neighboring bases and is substituted at the beta anomeric with a group that does not interact [i.e., hydrogen bond] with the base on the opposite strand of a duplex. Abasic residues in oligonucleotides can be introduced by the chemical or enzymatic hydrolysis of the glycosidic bond. The resulting structure is apurinic or apyrimidinic, lacks coding information, and fails to base pair. One abasic residue, the CE phosphoramidite of the tetrahydrofuran derivative, is commercially available (dSPACER, Glenn Research, Sterling, Virginia) along with other "spacer" phosphoramidites (Glenn Research, Sterling, Virginia). Alternatively, an abasic substitution may comprise a backbone of N-(2-aminoethyl)-glycine linked by amide bonds. Unlike native DNA or RNA backbone, this structure has no deoxyribose or phosphate groups.

The typical placement of the abasic site is approximately in the middle of the oligonucleotide. A typical primer has the following configuration: 5'-N$_{10}$-spacer-N$_{10}$-3'. However, multiple abasic sites may be placed in the oligonucleotide(s) at regular or irregular intervals, depending on the value of HCT to be achieved. Generally, a primer ranges in length from 6 to 40 or from 16 to 30 nt in length and contains from 1 to 5 abasic sites. Thus, abasic sites can be incorporated at a spacing of 3. 4, 5, 6. or 8 nucleotides or incorporated in any combination of nucleotides (or analogues) that base-pair with abasic sites. For example, a 6-mer may have one 1 abasic site, an 18-mer, 2 abasic sites, a 24-mer, 3 basic sites, etc. As a general guideline when an oligonucleotide is used to detect a mutation, the abasic site is preferably not located at the site of the mutation. However, abasic sites may be placed at the site of mutations that are not of interest (e.g., a polymorphism that does not result in a phenotype).

As shown in the table below, introduction of an abasic residue into 5'-hexylamine-TGTGGATCAGCA-spacer-GCAGGAGTATG-3', wherein the spacer is either the C3-spacer or dSPACER from Glenn Research (Sterling, VA, where these two chemicals have the same effect but are chemically distinct), lowers the HCT from 2.5°C to 6°C compared to a normal oligonucleotide, and depending on the solution used.

Table 4

| Buffer Type Factor | Oligo Type | HCT (°C) | $T_d$(°C) | Stringency factor |
|---|---|---|---|---|
| IX PCR buffer | normal | 18 | | |
| 1X PCR buffer | abasic (dSPACER) | 12 | | |
| 1X PCR buffer | abasic (C3 spacer) | 12 | | |
| 0.5 M TMATCA | normal | 14 | | |
| 0.5 M TMATCA | abasic (dSPACER) | 8 | | |
| 0.5 M TMATCA | abasic (C3 spacer) | 8 | | |
| 2.0 M LiTCA | normal | 12.5 | 44.5 | 4.97 |
| 2.0 M LiTCA | abasic (dSPACER) | 10 | 39 | 6.37 |
| 2.0 M LiTCA | abasic (C3 spacer) | 10 | 39 | 6.25 |
| 3.0 M GuSCN | normal | 16 | 35.5 | 3.85 |
| 3.0 M GuSCN | abasic (dSPACER) | 12.5 | 32 | 5.24 |
| 3.0 M GuSCN | abasic (C3 spacer) | 12.5 | 31 | 5.31 |

DeoxyNebularine (dN) can also be used to increase the enthalpy of an oligonucleotide duplex. Preferably, deoxyNebularine replaces a G, C, or T base in a probe or primer. Multiple deoxyNebularine sites may be placed in the oligonucleotide(s) at regular or irregular intervals, depending on the value of HCT to be achieved. Generally, a primer ranges in length from 6 to 40, preferably from 16 to 30 bases and contains from 1 to 5 deoxyNebularine sites. A typical primer has the following configuration: 5'-$N_{10}$- deoxyNebularine -$N_{10}$-3'. As shown in the table below, introduction of a deoxyNebularine residue into 5'-hexylamine-TGTGGATCAGCA-dN-GCAGGAGTATG-3' lowers the HCT from 2.5°C to 6°C, depending on the hybridization solution or hybotrope used.

Table 4B

| Buffer Type | Oligo Type | HCT* | $T_d$(°C) | Stringency Factor |
|---|---|---|---|---|
| 1X PCR buffer | normal | 18 | | |
| 1X PCR buffer | deoxyNebularine | 12 | | |
| 1X PCR buffer | deoxyNebularine | 12 | | |
| 0.5 M TMATCA | normal | 14 | | |
| 0.5 M TMATCA | deoxyNebularine | 8 | | |
| 0.5 M TMATCA | deoxyNebularine | 8 | | |
| 2.0 M LiTCA | normal | 12 | 44 | 5.0 |
| 2.0 M LiTCA | deoxyNebularine | 10 | 39 | 6.3 |
| 2.0 M LiTCA | deoxyNebularine | 10 | 39 | 6.3 |
| 3.0 M GuSCN | normal | 16 | 35 | 3.9 |
| 3.0 M GuSCN | deoxyNebularine | 12.5 | 32 | 5.2 |
| 3.0 M GuSCN | deoxyNebularine | 12.5 | 31 | 5.3 |

The invention thus provides an oligonucleotide comprising a plurality of fragments. each fragment shown schematically by structure (1)

(1)

$$B_1 \quad B_2 \quad B_3 \qquad B_5$$

wherein,

$$B_1 \quad B_2 \quad B_3$$

represents a sequence of at least three nucleotides (and preferably 4-12) as found in wild-type DNA, where "B" represents a base independently selected at each location:

▬▬▬▬ represents a series of covalent chemical bonds termed a "specificity spacer," which separates and connects two bases $B_3$ and $B_5$.

The meaning of strucuture (1) can be understood by reference to Figure 26, where it can be seen that structure (1) corresponds to a sequence of nucleotides wherein at least the base and perhaps more of the nucleotide is missing in the region termed the "specificity spacer". Each specificity spacer occupies no more that a single nucleotide site.

The specificity spacer has steric and chemical properties such that it does not prevent hybridization between a fragment of structure (1) and an oligonucleotide fragment having a complementary base sequence, as shown schematically as structure (2)

(1)

$$B_1 \quad B_2 \quad B_3 \qquad B_5$$

$$B_1' \quad B_2' \quad B_3' \quad B_4' \quad B_5'$$

(2)

Thus, in the structure above, it can be seen that the specificity spacer occupies a single nucleotide site, and does not prevent the "wild-type" nucleotides, i.e., the nucleotides having the standard phosphate-sugar-base group found in naturally occuring oligonucleotides (*e.g.*, DNA, cDNA, RNA) from base-pairing. The wild-type nucleotides are represented by the straight lines terminating in a "B", where "B" represents a standard base selected from adenine, guanine, cytosine, uracil and thymine.

The specificity spacer may or may not hydrogen bond with the nucleotide in the complementary chain (2) with which the chain having structure (1) forms a duplex. Preferably, the specificity spacer cannot hydrogen bond with anything. However, in another preferred embodiment, the specificity spacer can hydrogen bond with the "opposite base" (shown as "$B_4$- n the duplex of (1) and (2)), but not in the conventional Watson-Crick manner. In fact, if the specificity spacer can hydrogen bond with the base in the complementary chain, then that hydrogen bonding is preferably much weaker than the hydrogen bonding that would occur if the specificity spacer were to bond to the opposite base by standard Watson-Crick base pairing.

A preferred specificity spacer has the formula

$$\begin{array}{ccc} X & & X \\ \| & & \| \\ -P-O-\boxed{\quad SSC \quad}-O-P- \\ | & & | \\ Y & & Y \end{array}$$

wherein

Y is selected from oxygen, sulfur, methyl and amino when X is oxygen, or Y is selected from oxygen and sulfur when X is sulfur; and

SSC represents a specificity spacer component having a chain of 2-5 carbon atoms shown in the formula

$$\xi\!-\!C\!-\!(C)_n\!-\!C\!-\!\xi$$

wherein n is 0, 1, 2 or 3. The SSC should not have less than two carbon atoms because that would cause the nucleotides which neighbor the specificity spacer to be too close together to effective hydrogen bond with a complentary oligonucleotide. Likewise, the SSC should not have more than 5 carbons because again that would disrupt the ability of nucleotides in the specificity spacer-containing sequence to hydrogen bond with a complementary sequence. Preferably, the specificity spacer component has a total of 3 or 4 carbons directly separating the two flanking -O-P groups.

The 2-5 carbon atoms of the SSC may be substituted with essentially any atoms, so long as the arrangement of those atoms is not such that the specificity spacer completely stops a complementary oligonucleotide chain from hybridizing with the specificity spacer-containing oligonucleotide. Preferred SSCs are either unsubstituted (i.e., are alkylene chains) or are alkylene chains substituted with sterically non-demanding substituents such as halogen, C1-C10hydrocarboxyloxy (a hydrocarbyl group joined to the "2-5 carbon atoms" through an ether oxygen atom), hydroxyl, C1-C5hydrocarbyl and like-sized or smaller groups.

The specificity spacer component may contain a five- or six-memembered carbocyclic or heterocyclic ring. For instance, the SSC may be a ribose or deoxyribose group as found in a standard nucleotide, however this ribose or deoxyribose is "abasic" in that the purine or pyrimidine base is absent, and is preferably replaced with a hydrogen. A specificity spacer component of this structure may be represented by the formula (2)

$$\xi\!-\!C\!-\!C\!-\!C\!\sim \qquad (2)$$
$$X\quad\Big(\!-\!\Big)_{1-2}$$

wherein n is 1 and X is selected from carbon, oxygen and sulfur, such that any carbon shown in formula (2), including X when it is carbon, may be substituted with hydrogen, $C_1$-$C_5$hydrocarbyl, $C_1$-$C_5$hydrocarbyloxy, a non-hydrogen bonding purine base analog or a non-hydrogen bonding pyrimidine base.

The invention provides oligonucleotides having specificity spacers which may be in solution or may be bound to a solid support. Especially when bound to a solid support, the invention provides compositions in an array form, having a plurality of oligonucleotide sequences, each having specificity spacers.

Each oligonucleotide containing a specificity spacer contains a plurality of such spacers. Thus, specificity spacers preferably constitute 15-60% of the nucleotide positions in an oligonucleotide. However, the specificity spacers are not adjacent to one another, *i.e,* there is at least one "wild-type" nucleotide located between any two specificity spacers. In fact, all of the specificity spacers in an oligonucleotide preferably are separated by 4-12 "wild-type" nucleotides, and are more preferably separated by 5-8 or 8-12 wild-type nucleotides. The specificity spacers are preferably arranged in a repeating pattern, such that there would be 5 wild-type nucleotides followed by a specificity spacer, followed by 5 more wild-type nucleotides, followed by a specificity spacer, followed by 5 more wild-type nucleotides, etc. At each occurrence in an oligonucleotide chain, the chemical structure of the specificity spacer is independently selected.

Methods of synthesizing oligonucleotides are known to those of skill in the art. In paticular, methods of synthesizing oligonucleotides with natural and non-nucleotidic monomers are found in: Oligonucleotide Synthesis: A Practical Approach, Gait, ed., IRL Press, Oxford (1984). The 5'-Dimethoxytrityl-2'-deoxynucleoside-3'-(N,N-diisopropyl-2-cyanoethyl)phosphoramidites for the synthesis of standard oligonucleotides are available from Glen Research (Herndon, VA); Beckman Instruments (Brea, CA) or Applied Biosystems (Foster City, CA).

The specificity spacers suitable for direct incorporation into oligonucleotides for use in this invention are commercially as cynanoethyl phosphoramidites from manufacturers like Glen Research, Midland Certified Reagents (Midland, TX), and Clonetech (Palo Alto, CA). Alternatively, other specificity spacers can be prepared from compounds that contain the required diol by a three stage process familiar to those skilled in the art and described in Gait. Stage 1 involves protecting any amine as benzamides or other suitable protecting group. Stage 2 involves protecting one of the hydroxyls, preferably a primary hydroxyl, as a dimethoxytrityl ether using dimethoxytrityl chloride in pyridine. In the third stage the second hydroxyl is converted into a N.N-diisopropyl-2-cyanoethyl)phosphoramidite by phosphitylation with N,N,N,N-tetraisopropylphosphoramidite and diisopropylammonium tetrazolide. These phosphoramidites can be used on automated DNA synthesizers availible from Beckman, ABI or Perseptive Biosystems.

## C. METHODS OF USING HYBOTROPES AND OLIGONUCLEOTIDES CONTAINING AN ABASIC OR ANUCLEOSIDIC RESIDUE

As noted herein, a hybotrope may be used in essentially any reaction involving hybridization of a duplex in which the annealed region is from about 6 to about 40 base pairs long. Such reactions include screening for one or few base changes (*e.g.*, genetic screen), DNA sequence analysis by random oligonucleotide hybridization, amplification reactions, RTase polymerization, such as synthesis of cDNA, differential amplification.

As used herein, a "discrimination temperature" is a temperature at which a hybridization reaction is performed that allows discrimination between a mismatched duplex and a perfectly matched duplex. As shown herein, a range of temperatures satisfy criteria of a discrimination temperature. The discrimination temperature ranges from the temperature at which an $\alpha$ value (fraction of single stranded nucleic acid) is 0.2 for a given oligonucleotide duplex (or nucleic acid duplex) containing a mismatch at any place in the duplex, to the temperature at which a value for $\alpha$ equals 0.8 for the same given oligonucleotide duplex (or nucleic acid duplex), but which does not contain a mismatch at any place in the duplex. An $\alpha$ value is the fraction of single stranded nucleic acid at any given temperature generated during the thermal transition of a DNA strand from a double-stranded to a single stranded form. In determining $\alpha$, the mismatch can be due to any type of modified nucleotide, nucleoside, or derivative thereof. A discrimination temperature is applicable to any given duplex 6 nt to 250 nt in length, of any given G+C content, containing modified or substituted nucleotides or nucleosides, and in which the duplex is composed of deoxyribonucleotides, ribonucleotides, or mixtures of different types of strands. As an example, for an oligonucleotide duplex of 18 nucleotides in length, the critical discrimination temperature (range) would be from 10 to 15°C. The lowest temperature of the discrimination temperature range is dependent on the concentration and type of hybotrope used and can range from 0 to 80°C, preferably from 20 to 50°C.

### 1. Detection of a Mutation.

Mutations are a single-base pair change in genomic DNA. Within the context of this invention, most such changes are readily detected by hybridization with oligonucleotides that are complementary to the sequence in question. In the system described here, two oligonucleotides are employed to detect a mutation. One oligonucleotide possesses the wild-type sequence and the other oligonucleotide possesses the mutant sequence. When the two oligonucleotides are used as probes on a wild-type target genomic sequence, the wild-type oligonucleotide will form a perfectly based paired structure and the mutant oligonucleotide sequence will form a duplex with a single base pair mismatch. As shown herein, the resulting two types of duplexes (wild-type and mutant) have different $T_d$s as a result of a single base pair mismatch in one of the duplexes. For example, a 6 to 7°C difference between the $T_d$ of a wild-type (wt) duplex (perfectly based paired) and duplex containing a mismatch (described above as $\Delta T_d$) is obtained in a hybotrope (LiTCA), but not in standard salt-based hybridization solutions (*e.g.*, SSC). The $\Delta T_d$ value for a 30-mer was 6°C, 6.4°C for a 24-mer, and 7°C for a 18-mer.

A "long" oligonucleotide probe (> 18nt) may thus be used for mutation detection in a long polynucleotide target nucleic acid. For this application, a probe that hybridizes only to a single copy portion of the human genome is preferable. A 30-mer can be used to search a collection of $1.15 \times 10^{18}$ 30 mers (complexity = $3.45 \times 10^{19}$ nucleotides), a 24-mer can be used to search a collection of $2.81 \times 10^{24}$ 24 mers (complexity = $6.75 \times 10^{15}$ nucleotides) and a 18-mer can be used to search a collection of $6.87 \times 10^{10}$ 10-mers (complexity = $1.23 \times 10^{12}$ nucleotides). In addition, from a empirical point of view, oligonucleotides work best as probes or primers of eucaryotic DNA or RNA when greater than 23 nt in length.

As discussed above, a 6 to 7°C difference in the $T_d$ of a wild type versus mismatched duplex permits the ready identification or discrimination of the two types of duplexes. To effect this discrimination, hybridization is performed at the $T_d$ of the mismatched duplex in the respective hybotropic solution. The extent of hybridization is then measured for the set of oligonucleotide probes. When the ratio of the extent of hybridization of the wild-type probe to the mismatched probe is measured, a value to 10/1 to greater than 20/1 is obtained. These types of results permit the development of robust assays for mutation detection.

For exemplary purposes, one assay format for mutation detection utilizes target nucleic acid (*e.g.*, genomic DNA) and oligonucleotide probes that span the area of interest. The oligonucleotide probes are greater or equal to 24 nt in length (with a maximum of about 36 nt) and labeled with a fluorochrome at the 3' or 5' end of the oligonucleotide probe. The target nucleic acid is obtained via the lysis of tissue culture cells, tissues, organisms, etc., in the respective hybridization solution. The lysed solution is then heated to a temperature which denatures the target nucleic acid (15-25°C above the $T_d$ of the target nucleic acid duplex). The oligonucleotide probes are added at the denaturation temperature, and hybridization is conducted at the $T_d$ of the mismatched duplex for 0.5 to 24 hours. The genomic DNA is then collected and by passage through a GF/C (GF/B, and the like) glass fiber filter. The filter is then washed with the respective hybridization solution to remove any non-hybridized oligonucleotide probes (RNA, short oligos and nucleic acid does not bind to glass fiber filters under these conditions). The hybridization oligo probe can then be thermally eluted from the target DNA and measured (by fluorescence for example). For assays requiring very high levels of sensitivity, the probes are concentrated and measured.

Other highly sensitive hybridization protocols may be used. The methods of the present invention enable one to readily assay for a nucleic acid containing a mutation suspected of being present in cells, samples, etc., *i.e.,* a target nucleic acid. The "target nucleic acid" contains the nucleotide sequence of deoxyribonucleic acid (DNA) or ribonucleic acid (RNA) whose presence is of interest, and whose presence or absence is to be detected for in the hybridization assay. The hybridization methods of the present invention may also be applied to a complex biological mixture of nucleic acid (RNA and/or DNA). Such a complex biological mixture includes a wide range of eucaryotic and procaryotic cells, including protoplasts; and/or other biological materials which harbor polynucleotide nucleic acid. The method is thus applicable to tissue culture cells, animal cells, animal tissue, blood cells (*e.g.,* reticulocytes, lymphocytes), plant cells, bacteria, yeasts, viruses, mycoplasmas, protozoa, fungi and the like. By detecting a specific hybridization between nucleic acid probes of a known source, the specific presence of a target nucleic acid can be established.

A typical hybridization assay protocol for detecting a target nucleic acid in a complex population of nucleic acids is described as follows: Target nucleic acids are separated by size on a gel matrix (electrophoresis), cloned and isolated, sub-divided into pools, or left as a complex population. The target nucleic acids are transferred, spotted, or immobilized onto a solid support such as a nylon membrane or nitrocellulose membrane. (This "immobilization" is also referred to as "arraying"). The immobilized nucleic acids are then subjected to a heating step or UV radiation, which irreversibly immobilizes the nucleic acid. The membranes are then immersed in "blocking agents" which include Dendhart's reagent (Dendhart, *Biochem. Biophys. Res. Comm. 23*:641, 1966). heparin (Singh and Jones, *Nucleic Acids Res. 12*:5627, 1984), and non-fat dried milk (Jones et al., *Gene Anal. Tech. 1*:3, 1984). Blocking agents are generally included in both the prehybridization step and hybridization steps when nitrocellulose is used. The target nucleic acids are then probed with labeled oligonucleotide probes under conditions described above in hybotrope-based solutions. Probes may be detected by a conjugated enzyme. Unbound enzyme is then washed away and the membrane is immersed in a substrate solution. Signal is then detected by colorimetric means, by fluorescence or by chemiluminescence, depending on substrate type. Alternatively, the probe is directly labeled (*e.g.,* radioactive isotope, fluorescent molecule, mass-spectrometry tags; *see* U.S. Application No. 08/589,250, filed January 23, 1996, chemiluminescent tags and the like).

2. <u>DNA sequence analysis.</u>

DNA sequence analysis is conventionally performed by hybridizing a primer to target DNA and performing chain extensions using a polymerase. Specific stops are controlled by the inclusion of a dideoxynucleotide. The specificity of priming in this type of analysis can be increased by including a hybotrope in the annealing buffer and/or incorporating an abasic residue in the primer and annealing at a discriminating temperature.

Other sequence analysis methods involve hybridization of the target with an assortment of random, short oligonucleotides. The sequence is constructed by overlap hybridization analysis. In this technique, precise hybridization is essential. Use of hybotropes or abasic residues and annealing at a discriminating temperature is beneficial for this technique to reduce or eliminate mismatched hybridization. The goal is to develop automated hybridization methods in order to probe large arrays of oligonucleotide probes or large arrays of nucleic acid samples. Application of such technologies include gene mapping, clone characterization, medical genetics and gene discovery, DNA sequence analysis by hybridization, and finally, sequencing verification.

Many parameters must be controlled in order to automate or multiplex oligonucleotide probes. The stability of the respective probes must be similar, the degree of mismatch with the target nucleic acid, the temperature, ionic strength. the A+T content of the probe (or target), as well as other parameters when the probe is short (*i.e.,* 6 to 50 nucleotides) should be similar. Usually, the conditions of the experiment and the sequence of the probe are adjusted until the formation of the perfectly based paired probe is thermodynamically favored over the any duplex which contains a mismatch. Very large scale applications of probes such as sequencing by hybridization (SBH), or testing highly polymorphic loci such as the cystic fibrosis trans-membrane protein locus require a more stringent level of control of multiplexed probes. William Bains (*GATA 11*:49, 1994), has ascertained that the ability to use multiplexed oligonucleotide probes is generally much more difficult to implement than is suggested by theory. Hybotropes and abasic residues will essentially overcome the limitations in the use of multiplexed probes as presented by Bains.

The actual length of an oligonucleotide probe to uniquely prime any natural nucleic acid target is far longer than is predicted by theory. In general, the probability that a given probe is unique is related to the length. Theoretically, the length is 12 to 15 nucleotides when the target is 520 kilobases in length. However, it is shown that a probe needs to be 24 nucleotides in order to possess a 90% probability of being unique. Therefore, using longer "short" probes (i. e., 24-36 nucleotide lengths) in hybridization assays that need to be specific is highly desirable. The methods and compositions presented here substantially aid in the use of long oligonucleotide probes (*i.e.,* 24-36 nucleotide lengths) in terms of discrimination.

## 3. Amplification reactions.

The observation that $\Delta T_d$ does not change as a function of concentration of hybotrope has substantial utility for use in DNA, RNA or nucleic acid amplifications based on primer extension by a polymerase (*e.g.,* polymerase chain reaction, *see* U.S. Patent Nos. 4,683,195; 4,683,202; and 4,800,159, cycling probe technology, NASBA), ligation (LCR, ligation chain reaction), and RNA amplification (*see* Lizardi et al., *Bio/Technology 6:*1197, 1988; Kramer et al., *Nature 339:*401, 1989; Lomeli et al., *Clin. Chem. 35:*1826, 1989; U.S. Patent No. 3,786,600). The observation that wt and mt 30-mer oligonucleotides can be distinguished on the basis of thermal melting in 0.5 M LiTCA permits the possibility of a substantial improvement in priming efficiency in PCR. In its current configuration, the PCR buffer is optimized for the polymerase rather for specific priming. That is, conditions have evolved since the introduction of the technique that favor performance of the polymerase over the performance of specificity of priming with oligonucleotides. Thus, PCR buffer as currently commercially available does not provide or support a high level of stringency of hybridization of PCR primers.

Commercially available PCR buffers are examined with respect to the melting behavior of 24-mer oligonucleotides in both the wild-type (wt) and mutant (mt) forms. In Table 5, the level of discrimination achieved in PCR buffer versus a low molarity concentration of hybotrope is shown.

Table 5

| $\Delta T_d$ for PCR buffers and low molarity hybotropes | | | | | |
|---|---|---|---|---|---|
| Solution | Conc. | Oligo Length | HCT* | $\Delta T_d$* | $T_d$* |
| PCR buffer | 1x | 24-mer wt | 15 | | 61 |
| PCR buffer | 1x | 24-mer mt | 14 | 1 | 60 |
| LiTCA | 0.1 M | 24-mer wt | 12 | | 65.5 |
| LiTCA | 0.1 M | 24-mer mt | 8 | 4 | 61.5 |

\* = °C

As shown, the HCT for standard PCR buffer is about 15°C, whereas the HCT for 0.1 M LiTCA is about 12°C. The $\Delta T_d$ for the lx PCR buffer is only 1°C for the 24-mer, whereas the $\Delta T_d$ for 0.1 M LiTCA is 4°C. Therefore, priming specificity is significantly improved in 0.1 M LiTCA versus 1X PCR buffer. Higher concentrations of hybridization solutions may also be used (0.1 M LiTCA to 3.0 M LiTCA or 0.1 to 3.0 TMATCA).

Alternatively, priming is performed in a hybotrope solution and chain extension is performed in a separate buffer that supports the polymerase. For example, a solid phase PCR could be employed where the solid phase is moved through two solutions. Priming would occur in some appropriate concentration of LiTCA or TMATCA and then the polymerase chain reaction would take place in a different PCR buffer containing the polymerase. It is also possible to conduct the first few rounds in the amplification in a hybotrope based hybridization solution and conducting the remaining rounds on normal PCR buffer (generally, only the first few rounds are important for specificity).

The use of deoxyNebularine modified oligonucleotides also increases the specificity of priming in the PCR. One deoxyNebularine substitution incorporated into an oligonucleotide reduces the HCT by 2.5°C. Two oligonucleotides probes containing 3 deoxyNebularine sites per 24-mer decrease HCT by 8°C relative to the unsubstituted control. This decrease in the HCT dramatically increases the level of specificity of priming in an amplification reaction (*e.g.,* polymerase chain reaction). This is likely due to the reduction of false or mis-priming during the first few (*e.g.,* 10) cycles of PCR. That is, the enthalpy of the deoxyNebularine substituted oligonucleotide increases relative to the unsubstituted primer, thus increasing the specificity of priming. Within the context of this invention, the primer is preferably 6 to 36 bases in length and contains 1 to 6 deoxyNebularine sites. The sites are preferably separated by 4, 5, 6, 7 or 8 nucleotides and may be separated by up to 12 to 24 nucleotides. The substitutions are also preferably clustered at the 3' end of the primer to ensure specificity of primer extension by nucleic acid polymerases, which may be, for example, DNA or RNA primers. Moreover, the temperature range over which priming occurs is dramatically reduced when deoxyNebularine-substituted primers are used.

As shown in the examples (see Example 8), the temperature range in which amplifications are observed is decreased from about 25°C - 65°C to about 25°C - 35°C. In addition, this decrease is observed for two different DNA polymerases.

The results also indicate that the dSpacer substitution prevents the polymerase from "reading through" the abasic site. That is, when the polymerase encounters an abasic residue, chain extension is terminated. However,- unlike abasic residues, a deoxyNebularine residue does not terminate chain extension. Although, as noted above, the temperature range over which the amplification range is much reduced compared to non-substituted oligonucleotides. Therefore, deoxyNebularine substituted primers can substantially increase the specificity of a DNA polymerase chain reaction.

Furthermore, the combination of an deoxyNebularine site in an amplification PCR primer and a hybotrope salt solution, which promotes a high enthalpy value for the primer duplex, significantly lowers the HCT of the primer duplex. As discussed above, when the HCT decreases, the stringency factor increases and high-discrimination priming of the polymerase chain reaction can take place. These are conditions required for favorable multiplexing PCRs. The term multiplexing refers to the ability to use more than one set of primers in a PCR reaction and generate multiple products or the ability to use more than one target nucleic acid per set of PCR primers.

The use of the hybotrope tetramethylammonium trichloroacetate is of particular utility because the dependence of G+C content on $T_d$ (stability) is neutralized. However, other hybotropes of the present invention which may be used in the polymerase chain reaction include, without limitation, bis(2-methoxyethyl)amine acetate, 1-ethylpiperidine acetate, 1-ethylpiperidine trichloroacetate, 1-ethylpiperidine trifluoroacetate, 1-methylimidizole acetate, 1-methylpiperidine acetate, 1-methylpiperidine trichloroacetate, 1-methylpyrrolidine acetate, 1-methylpyrrolidine trichloroacetate, 1-methylpyrrolidine trifluoroacetate, 2-methoxyethylamine acetate. N,N-dimethylcyclohexylamine acetate, N,N-dimethylcyclohexylamine trifluoroacetate. N,N-dimethylcyclohexylarnine, N,N-dimethylheptylamine acetate, N,N-dimethylheptylamine acetate, N,N-dimethylhexylamine acetate, N,N-dimethylhexylamine acetate, N,N-dimethylisopropylamine acetate, N-ethylbutylamine acetate, N-ethylbutylamine tritluoroacetate, N,N-dimethylaminobutane trichloroacetate, N,N-dimethylisopropylamine trichloroacetate, triethanolamine acetate, triethylamine acetate, triethylamine trichloroacetate, tripropylamine acetate, tetraethylammonium acetate. These compounds or chemicals can be combined in amplification reaction with divalent cations such as Mg++, buffers, detergents, co-factors, nucleotides and their analogs, polymerases and/or ligases. The compounds listed above can be used in concentration ranging from 5 mM to 6 M, preferably from 100 mM to 2.5 M.

## D. HYBOTROPES AND ABASIC NUCLEIC ACIDS IN ARRAYS

In the fields of molecular biology and microbiology, it has long been common to employ solid supports having biomolecules immobilized thereon. Immobilization provides various advantages, such as, allowing for multiplexing of samples and ready measurements of tags employed in a large number of signal systems.

Recently, intense attention has focused on creating arrays of biomolecules, and particularly polynucleotides, on a flat solid support. The following publications (and the references cited therein), which are exemplary only, provide general and specific overviews of various utilities for these biomolecular arrays, as well as methods of preparing such arrays: M.D. Eggers et al., *Advances in DNA Sequencing Technology, SPIE 1891*:113-126, 1993; A.B. Chetverin et al., *Bio/Technology 12*:1093-1099, 1994; E.M. Southern, *Nucleic Acids Research 22*:1368-1373, 1994; R.J. Lipshutz et al., *BioTechniques 19*:442-447, 1995; M. Schena, *BioEssays 18*:427-431, 1996; A.P. Blanchard et al., *Biosensors & Bioelectronics 11*:687-690, 1996; M.J. O'Donnell-Maloney et al., *Genetic Analysis: Biomolecular Engineering 13*: 151-157, 1996; A. Regalado, *Start-Up* 24-30, Oct. 1996; and D. Stipp, *Fortune* 30-41, March 31, 1997.

The advent of large scale genomic projects and the increasing medical use of molecular diagnostics, has prompted the development of large volume throughput methods for screening recombinant DNA libraries representing entire genomes, the performance of large scale DNA sequencing projects, and executing replicative immunological assays, nucleic acid hybridization assays, or polymerase chain reaction assays. The following publications (and the references cited therein). which are exemplary only, provide general and specific overviews of large throughput methods that rely on biomolecular arrays, as well as methods of preparing such arrays: M.D. Eggers et *al., Advances in DNA Sequencing Technology, SPIE 1891*:113-126. 1993; A.B. Chetverin et al., *Bio/Technology 12*:1093-1099, 1994; E.M. Southern, *Nucleic Acids Research 22*:1368-1373, 1994; R.J. Lipshutz et al., *BioTechniques 19*:442-447, 1995; M. Schena, *BioEssays 18*:427-431, 1996; A.P. Blanchard et al., *Biosensors & Bioelectronics 11*:687-690, 1996: M.J. O'Donnell-Maloney et al., *Genetic Analysis: Biomolecular Engineering 13*:151-157, 1996; A. Regalado, *Start-Up* 24-30, Oct. 1996; and D. Stipp, *Fortune* 30-41, March 31, 1997.

The need for high throughput methodology has led, in some cases, to a change from a 96-well microtiter dish format, to a 384-well (Maier et al., *J. Biotechnology 35*:191, 1994) or 864-well (Drmanac et al., *Electrophoresis 13*:120, 1992) format, which can also be used in conjunction with robotic devises (*see,* e.g., Belgrader et al., *BioTechniques 19*:426, 1995; Wilke et al., *Diagnostic Microbiology and Infect. Disease 21*:181, 1995). However, all of these automated techniques require the use of a robotic pin-tool devise that is capable of reproducibly transferring equal volumes of liquid from one arrayed configuration (*i.e.,* 96-well microtiter plate) to another (*i.e.,* 96-spot array on a hybridization filter membrane).

Recently, methods have also been developed to synthesize large arrays of short oligodeoxynucleotides (ODNs) bound to a glass surface that represent all, or a subset of all, possible nucleotide sequences (Maskos and Southern, *Nucl. Acids Res. 20:*1675, 1992). Once such an ODN array has been made may be used to perform DNA sequencing by hybridization (Southern et al., *Genomics 13:*1008, 1992; Drmanac et al., *Science 260:*1649, 1993). The utility of this method of DNA sequencing would be greatly improved if better methods existed for the transfer and arraying of the precise amounts of the biochemical reagents required for the synthesis of large sets ODNs bound to hybridizable surfaces. This would enable greater equality of ODN yield at each position within the array and also increase the nucleotide chain length it is possible to synthesize.

The polymerase chain reaction (PCR) has found wide application to many different biological problems. Two major limitations to the commercial utilization of PCR are the high cost of the reagents and the inability to automate the performance of the process. Reagent costs can be lowered if the total volume of each reaction can be decreased, allowing a concomitant decrease in DNA polymerase and nucleotides. An accurate and reliable means to array small volumes of reagents using a robotically controlled pin tool could help solve both of these PCR problems.

The combination of hybotropes and modified oligonucleotide probes described in this application will permit the useful multiplexing of probes and "capture" oligonucleotides in the array format. Hybotropes that neutralize the G+C ccntent effect on $T_m$ or $T_d$ are especially useful in the application and use of array technology. In traditional hybridization solutions the difference in $T_m$ or $T_d$ when the G+C content is varied from 20% to 80% is generally 12 to 16°C. Therefore is it impossible to maintain the ideal hybridization temperature which is 1 to 8 degrees below the $T_m$ of the respective oligonucleotide duplex as the G+C content is varied. Solutions (hybotropes) which neutralize the effect of G+C on $T_m$ or $T_d$ permit the useful multiplexing of probes. Hybotropes such as bis(2-methoxyethyl)amine acetate, l-ethylpiperidine acetate, 1-ethylpiperidine trichloroacetate, 1-ethylpiperidine trifluoroacetate, 1-methylimidizole acetate, 1-methylpiperidine acetate, 1-methylpiperidine trichloroacetate, 1-methylpyrrolidine acetate, 1-methylpyrrolidine trichloroacetate, 1-methylpyrrolidine trifluoroacetate, 2-methoxyethylamine acetate, N,N-dimethylcyclohexylamine acetate, N,N-dimethylcyclohexylamine trifluoroacetate, N,N-dimethylcyclohexylamine, N,N-dimethylheptylamine acetate, N,N-dimethylheptylmnine acetate, N,N-dimethylhexylamine acetate, N,N-dimethylhexylamine acetate, N,N-dimethylisopropylamine acetate, N-ethylbutylamine acetate, N-ethylbutylamine trifluoroacetate, N,N-dimethylaminobutane trichloroacetate, N,N-dimethylisopropylamine trichloroacetate, triethanolamine acetate, triethylamine acetate, triethylamine trichloroacetate, tripropylamine acetate, tetraethylammonium acetate are useful in array formats.

A number of genetic diseases are caused by single, or a limited set, of mutations due to founder effects or advantages to heterozygous carriers. There is an increasing clinical interest in monitoring sequence variants associated with, for example, altered metabolism of drugs or serving as genetic markers in forensic medicine, and in the diagnosis of infectious disease, identification of drug-resistant variant strains may require distinction between similar sequence variants.

The solutions described herein are used to increase the specificity of priming in the PCR. There are several options in terms of a mechanism in which the specificity of the priming step can be improved. The first is a through the use of a solid support to which one of the PCR primers is (covalently) attached. The solid support can take many forms such as beads, membranes, etc. The priming step can take place in the hybotrope and then the solid support can be washed and moved into a solution that supports the polymerase chain extension. The solid support is then moved back into the nesstrope for the priming reaction and the cycle is repeated. The cycling of the solid support between the two different solutions only has to occur to a limited number of times (1-15 cycles) after which time the traditional amplification cycle in a standardized PCR buffer can be allowed proceed. Alternatively, the target nucleic acids of interest are moved between the priming solution and the polymerase extension reaction solution using electric fields (*i.e.,* electrophoresis).

The use of hybotropes and/or abasic or anucleosidic oligonucleotide probes can be used to increase the specificity and efficiency of isothermal applications of polymerases to the amplification of nucleic acid sequences. Applications of isothermal conditions for using nucleic acid polymerases include nucleic acid sequencing, genotyping, mutation detection, oligonucleotide ligation assays, mutation detection, and the like.

The following examples are offered by way of illustration, and not by way of limitation.

EXAMPLES

EXAMPLE 1

PREPARATION, PROPERTIES, AND USES OF NOVEL HYBOTROPES

A novel hybotrope is synthesized which demonstrates properties not previously described for a salt solution. Tetramethyl ammonium- and tetraethyl ammonium-trichloroacetate are synthesized by neutralizing tetramethyl ammonium-and tetraethyl ammonium-hydroxide with trichloroacetate to pH 7.0 to pH 8.5, depending upon the application. The resulting salt solution is then dried under vacuum to complete dryness and the mass is determined. The salt is then dissolved in water to a final concentration of 0.5 to 3.0 M. The resulting salt solution is then buffered with a buffer such as Tris-HCl, pH 7.0-8.5, and detergents, such as sarkosyl, are added to about 0.1%, and optionally EDTA is added to 0.5 to 5 mM.

These hybotropes possess the property of neutralizing the differences in G+C and A+T base-pairing strength while simultaneously lowering the $T_d$ and $\Delta T_d$, increasing $\Delta T_d$. In the table below the characteristics of the novel TEAT-CA and TMATCA hybotropes.

25% G+C content: 5'-AAATAATTCAGGGTCAAAA-3'
36% G+C content: 5'-CTGTCGTAGGTAAATAACT-3'
42% G+C content: 5'-AAAAAGTGGGGAAGTGAGT-3'
57% G+C content: 5'-GTGTTAACTTCCGCTCCTC-3'
63% G+C content: 5'-GGCGTAGGTCTGTCGTGCT-3'
73% G+C content: 5'-GGTGTGGGTCCGTCGTGCC-3'

The following $T_d$s are obtained in the hybridizations described below:

Table 6

| Solution Type | Length of Probe | G+C Content | $T_d$ | HCT |
|---|---|---|---|---|
| 3 M TEATCA | 19-mer | 25% | 38.0 | 7.5 |
| 3 M TEATCA | 19-mer | 36% | 38.5 | 10 |
| 3 M TEATCA | 19-mer | 42% | 39 | 11.5 |
| 3 M TEATCA | 19-mer | 57% | 40 | 12 |
| 3 M TEATCA | 19-mer | 63% | 41 | 13 |
| 3 M TEATCA | 19-mer | 73% | 42 | 14 |
| 3 M TMACl | 19-mer | 25% | 61 | 12.5 |
| 3 M TMACl | 19-mer | 36% | 62 | 14 |
| 3 M TMACl | 19-mer | 42% | 60 | 15.5 |
| 3 M TMACl | 19-mer | 57% | 65 | 17 |
| 3 M TMACl | 19-mer | 63% | 59 | 17.5 |
| 3 M TMACl | 19-mer | 73% | 59 | 17.5 |
| 3 M TMATCA | 19-mer | 25% | 44.5 | 8 |
| 3 M TMATCA | 19-mer | 36% | 45.5 | 10 |
| 3 M TMATCA | 19-mer | 42% | 43 | 11.5 |
| 3 M TMATCA | 19-mer | 57% | 48.5 | 12 |
| 3 M TMATCA | 19-mer | 63% | 47 | 13 |

Table 6   (continued)

| Solution Type | Length of Probe | G+C Content | $T_d$ | HCT |
|---|---|---|---|---|
| 3 M TMATCA | 19-mer | 73% | 48.5 | 14 |
| 2 M TMATCA | 19-mer | 25% | 43 | 15 |
| 2 M TMATCA | 19-mer | 36% | 44.5 | 17 |
| 2 M TMATCA | 19-mer | 42% | 44.5 | 18 |
| 2 M TMATCA | 19-mer | 57% | 53 | 19.5 |
| 2 M TMATCA | 19-mer | 63% | 48 | 19.5 |
| 2 M TMATCA | 19-mer | 73% | 52 | 19 |
| 30% formamide | 19-mer | 25% | 25 | 20 |
| 30% formamide | 19-mer | 36% | 27.5 | 20 |
| 30% formamide | 19-mer | 42% | 29 | 20 |
| 30% formamide | 19-mer | 57% | 40 | 21 |
| 30% formamide | 19-mer | 63% | 37.5 | 22 |
| 30% formamide | 19-mer | 73% | 40 | 23 |

The data in Table 6 clearly indicate a decrease in the helical coil transition in solutions containing 3 M TMATCA or 3 M TEACl compared to the control solution which is TMACL. An average decrease of 3.5°C is observed for solutions containing 3 M TMATCA or 3 M TEACl compared to the control solution which is TMACL. Also, formamide has a surprisingly high value for the helical coil transition, which ranges from 20 to 23°C depending on the G+C value. Also shown is the concentration dependence of the ability of a TMATCA solution to neutralize G+C content. At 2 M, TMATCA is neither able to neutralize G+C content or reduce the HCT. This property is unique as no other hybotrope displays a concentration dependence.

EXAMPLE 2

DETERMINATION OF THE MELTING TEMPERATURE OF OLIGONUCLEOTIDE DUPLEXES IN VARIOUS HYBOTROPE AND NON-HYBOTROPE BASED HYBRIDIZATION SOLUTIONS.

This example describes the determination of the $T_d$ of wild type and mutant oligonucleotides when hybridized to a target nucleic acid. It is shown that hybotrope based hybridization solutions allow the detection of single base pair mutations in a nucleic acid target with a probe up to a 30 nucleotides in length.

Solutions and Reagents

Filter wash (FW) is 0.09 M NaCl, 540 mM Tris pH 7.6, 25 mM EDTA. SDS/FW is FW with 0.1% sodium dodecyl sulfate (SDS). Hybridization solutions contain the text specified concentration of hybotrope 2% N-lauroylsarcosine (sarcosyl), 50 mM Tris pH 7.6 and 25 mM EDTA. Formamide hybridization solution contains 30% formamide, 0.09 M NaCl, 40 mM Tris-HCl pH 7.6, 5 mM EDTA and 0.1% SDS. GuSCN is purchased from Kodak (Rochester, NY). GuCl, lithium hydroxide, trichloroacetic acid, NaSCN, $NaClO_4$ and KI, are purchased from Sigma (St. Louis, MO). Rubidium hydroxide is purchased from CFS Chemicals (Columbus, OH). CsTFA is purchased from Pharmacia (Piscataway, NJ).

Preparation of LiTCA, TMATCA and TEATCA

LiTCA and TMATCA, and TEATCA are prepared by the dropwise titration of a 3 N solution of LiOH, TEAOH and TMAOH respectively, with trichloracetic acid (100% w/v, 6.1 N) to pH 7.0 on ice with constant stirring. The salt is evaporated to dryness under vacuum, washed once with ether and dried.

Oligonucleotide Synthesis

Oligonucleotides are synthesized on a commercial synthesizer using standard cyanoethyl-N,N-diisorpropylamino-phosphoramidite (CED-phosphoramidite) chemistry. Amine tails are incorporated onto the 5'-end using the commercially available N-monomethoxytritylaminohex-6-yloxy-CED-phosphoramidite. Alternatively, oligonucleotides are commercially purchased.

Preparation of Nylon Bead Supports (ODN-Bead)

ODN (oligonucleotide)-beads (3/32nd inch diameter) are prepared as previously described (Van Ness et al., *Nucl. Acids Res. 19*:3345, 1991). The ODN-beads contain 0.01 to 1.2 mg/bead of covalently immobilized ODN.

Determination of $T_d$ and $T_{opt}$ Values Using ODN-Beads in Various Hybridization Solution Containing Hybotropic Salts

To label the probe oligonucleotides, amine ODNs are reacted with amine-reactive fluorochromes. The derived ODN preparation is divided into 3 portions and each portion is reacted with either (a) 20-fold molar excess of Texas Red sulfonyl chloride (Molecular Probes, Eugene, OR), with (b) 20-fold molar excess of Lissamine sulfonyl chloride (Molecular Probes, Eugene, OR), or (c) 20-fold molar excess of fluorescein isothiocyanate. The final reaction conditions consist of 0.15 M sodium borate at pH 8.3 for 1 hour at room temperature. The unreacted fluorochromes are removed by size exclusion chromatography on a G-50 Sephadex column.

For the determination of ODN/ODN $T_d$ from the ODN-bead. fluorescently-labeled ODN is incubated in various hybridization solutions with a complementary ODN immobilized on ODN-beads. From 5 to 5000 ng of ODN are hybridized in 300-400 $\mu$l volumes at various temperatures (19-65°C) for 5-30 minutes with constant agitation. The beads are washed with 3x 1 ml of the respective hybridization solution, and then once with the respective melting solution at the starting temperature of the melting process. The beads in 300-400 $\mu$l of the respective melting solution are then placed in a 0-15°C water bath. At 5 minute intervals, the temperature is raised 5°C, the solution decanted into a well of a microtiter plate, and fresh solution (5°C below the next increment) is added to the beads. The "melting" or duplex dissociation is conducted over a temperature range of 15°C to 95°C. Fluorescence is measured with a commercial fluorescence plate reader.

To calculate the $T_d$, cumulative counts eluted at each temperature are plotted against temperature. The temperature at which 50% of the material is dissociated from the bead is the $T_d$.

For the determination of RNA/ODN or DNA/ODN $T_d$ mems from nylon membranes (Schleicher & Schuell, Keene, N.H.), $^{32}$P-labeled ODN (3'-labeled with terminal transferase) is incubated with 0.5 cm$^2$ pieces of membrane, in text-specified hybridization solutions. For the (non-covalent) immobilization of genomic DNA onto nylon membranes, purified DNA is denatured in 0.3 M NaOH at 20°C for 10 minutes. An equal volume of 2 M ammonium acetate is added and the sample was applied to Nytran membranes assembled in a slot blot apparatus. RNA was denatured in 4.6 M formaldehyde-6 x SSC (0.9 M NaCl, 90 mM sodium citrate) for 15 min. at 60°C and applied to the membranes as above. After immobilization of the nucleic acids, the filters were baked at 80°C for 2 hours, then stored dry at ambient temperature. The hybridizations and dissociations were then performed as described above for the nylon bead solid supports.

To determine the $T_{opt}$ODN (the temperature at which the maximum rate of hybridization of target nucleic acid to ODNs occurs, under near stringent to stringent conditions (-20 to -5°C below the $T_d$)), complementary $^{32}$P-labeled ODN is hybridized (to the $C_0t_{1/2}$) to either covalently immobilized ODN sequences on the ODN-bead as described above, or in a sandwich assay format when RNA is used as the target nucleic acid. The hybridizations are performed over a 40°C range (+ or -20°C around the $T_d$ of the respective duplex in 5°C increments). The extent of hybridization is then measured as a function of temperature at the $C_0t_{1/2}$ of the respective hybridization.

Spectroscopic Thermal Denaturation

Thermal transitions determined in solution ($T_m$) are recorded at 260 nm using a Gilford System 2600 UV-VIS spectrophotometer equipped with a Gilford 2527 Thermo-programmer. ODNs (2 mM/strand) are dissolved in the respective hybridization or melting solutions. The ODN mixtures were heated to 85°C, then cooled to 10-15°C to allow hybridization. The samples were slowly heated to 85°C employing a temperature increase of 0.5°C/min. Absorbance versus time is recorded. and the first derivative is computed automatically. The $T_m$ values are determined using the first derivative maxima.

Oligonucleotides

The following oligonucleotides are used to measure the difference in $T_d$ between a wild type oligonucleotide and a mutant oligonucleotide. The wild type oligonucleotide represents fully and perfectly base-paired duplex and a mutant oligonucleotide represents a single base pair mismatch (generally in the middle of the oligonucleotide).

The sequence of the "capture" oligonucleotide is 5'-GTCATACTCCTGCTTGCTGATCCACATCTG-3'. The sequence of the wild type 30-mer is 5'-CAGATGGGTATCAGCAAGCAGGAGTATGAC-3', the sequence for the wild type 24-mer 5'-ATGGGTATCAGCAAGCAGGAGTAT-3', the sequence for the wild type 18-mer 5'-GGTATCAGCAAGCAGGAG-3'. The sequence of the mutant 30-mer is 5'-CAGATGGGTATCAGGAAGCAGGAGTATGAC-3', the sequence for the mutant 24-mer 5'-ATGGGTATCAGGAAGCAGGAGTAT-3', the sequence for the mutant 18-mer 5'-GGTATCAGGAAGCAGGAG-3'.

The helical coil transition of an oligonucleotide or nucleic acid duplex can be measured by essentially an adaptation of methods previously described by Martinson (*Biochemistry 12*:145-165, 1973) for the thermal elution of DNA or RNA duplexes or hybrids from hydroxylapatite. For the determination of the helical coil transition from a solid support, fluorescently-labeled oligonucleotide (ODN) was incubated in various hybridization solutions with a complementary ODN immobilized on ODN-beads. From 5 to 5000 ng of ODN were hybridized in 300-400 μl volumes at various temperatures (19-65°C) for 5-30 minutes with constant agitation. The beads were washed with 3 x 1 ml of the respective hybridization solution, and then once with the respective melting solution at the starting temperature of the melting process. The beads in 300-400 μl of the respective melting solution were then placed in a 0-15°C water bath. At 5 minute intervals, the temperature was raised 5°C, the solution decanted into a well of a microtiter plate, and fresh solution (5°C below the next increment) was added to the beads. The "melting" or duplex dissociation was conducted over a temperature range of 15°C to 95°C. Fluorescence was measured with a commercial fluorescence plate reader. To calculate the $T_d$, cumulative counts eluted at each temperature were plotted against temperature. The temperature at which 50% of the material had been dissociated from the bead was taken as the $T_d$. The helical coil transition is defined as the temperature at which a value of a equals 0.2 for a given oligonucleotide duplex (or nucleic acid duplex, containing or not containing a mismatch at any place in the duplex) to the temperature at which a value for a equals 0.8 for the same given oligonucleotide duplex (or nucleic acid duplex).

The following $T_d$s are obtained in the hybridizations described below:

Table 7

| Solution Type | Length of Probe | $T_d$ (Mutant) (°C) | $T_d$ (Wild Type) (°C) | $\Delta T_d$ (°C) | HCT (°C) |
|---|---|---|---|---|---|
| 2.5 m LiTCA | 30-mer | 27 | 33 | 6 | 13/14 |
| 2.5 m LiTCA | 24-mer | 25.5 | 32 | 6.5 | 13/14.5 |
| 2.5 m LiTCA | 18-mer | 24 | 31 | 7 | 9/14 |
| 2.0 m LiTCA | 30-mer | 42 | 47 | 5 | 13.5/16 |
| 2.0 m LiTCA | 24-mer | 38 | 44 | 6 | 14/15 |
| 2.0 m LiTCA | 18-mer | 37 | 43 | 6 | 14.5/16.5 |
| 3.0 m GuSCN | 30-mer | 37 | 42.5 | 5.5 | 13.5/17.5 |
| 3.0 m GuSCN | 24-mer | 34.5 | 41 | 6.6 | 12.5/17 |
| 3.0 m GuSCN | 18-mer | 33.5 | 40.5 | 7 | 14.5/15 |
| 3.0 m GuHCl | 30-mer | 55.5 | 60 | 4.5 | 16/21 |
| 3.0 m GuHCl | 24-mer | 52.5 | 58 | 5.5 | 15/20 |
| 3.0 m GuHCl | 18-mer | 50 | 57 | 7 | 18/20 |
| Rapid Hybe | 30-mer | 80 | 80 | 0 | na* |
| Rapid Hybe | 24-mer | 80 | 80 | 0 | na |
| Rapid Hybe | 18-mer | 68 | 70 | 2 | 18/23 |
| 5x SSC | 30-mer | 72.5 | 72.5 | 0 | 18/18 |
| 5x SSC | 24-mer | 69 | 70 | 1 | 18/18 |
| 5x SSC | 18-mer | 67 | 72 | 5 | 16/18 |
| Promega QY | 30-mer | 80 | 80 | 0 | na |
| Promega QY | 24-mer | 80 | 80 | 0 | na |
| Promega QY | 18-mer | 62 | 65 | 3 | 20/23 |

* na indicates not applicable or too large to accurately determine.

The data indicate that the hybotropic solutions (LiTCA, GuSCN and GuHCl) permit the detection of a single base-pair mismatch in a 24-mer and 30-mer probe whereas the detection of a single base-pair mismatch in standard hybridization solutions (Rapid Hybe, Promega QY or 5x SSC) is not possible.

A similar experiment is performed for the 24-mers described above in a series of hybridization solutions.

Table 8

| Hybridization Solution Type | Slope ([..], k) | HCT | $\Delta T_d$ |
|---|---|---|---|
| LiTCA, 3 M | 19 | 8 C | 7.5 C |
| GuSCN, 3 M | 13 | 10 | 6.0 |
| NaSCN, 3 M | 8.5 | 11 | 5.5 |
| NaClO$_4$, 3 M | 7 | 12 | 4.5 |
| KI, 3M | 5 | 15 | 3.0 |
| NaCl, 0.165 M | 4.5 | 17.5 | 1.5 |
| GuCl, 3 M | 3.5 | 18 | 1.2 |
| CsTFA, 2M | 2.5 | 18 | 1.2 |
| 30% formamide | ND | 20 | 1.5 |

$T_d$(Wt) is the $T_d$ of a perfectly base-paired oligonucleotide duplex and $T_d$(mt) is the $T_d$ of a oligonucleotide duplex containing a single mismatch. The values are for a 24-mer duplex of sequence described in Example 1. From the data presented in the table above, the stringency factor is directly proportional to the difference between a perfectly base paired duplex and a duplex containing a mismatch. That is, the stringency factor predicts the ability of given hybridization solution to discriminate mismatched duplexes.

EXAMPLE 3

EFFECT OF CONCENTRATION OF SALT OR HYBOTROPE ON HCT AND $T_D$.

The discrimination between mismatched oligonucleotides (mutant abbreviated as "mt") and perfected based-paired oligonucleotides (abbreviated as "wt") is not a function of concentration of a particular hybotrope but rather a function of hybotrope type. HCT is defined as the temperature range over which a duplex melts during a melting process under defined conditions. To calculate HCT, the temperature at which 80% of the duplexes are melted is subtracted from the temperature at which 20% melting is observed. Surprisingly, for the hybotropes LiTCA, GuSCN, GuHCl, NaClO$_4$ the HCT does not change over about the range of 0.5 M to about 6.0 M. The slope of the mt duplex is always observed to be greater than for wt duplexes (see Table 8). Another parameter which does not change as a function of concentration is the difference between the $T_d$ of the wt duplex and the mutant duplex ($\Delta T_d$). The $T_d$ of the mt and wt duplexes is observed to be strictly dependent on concentration in a precisely linear relation. In Table 9, the HCT and $T_d$ for mt and wt 30-mer duplexes and mt and wt 18-mers are presented.

The effect of concentration and hybotrope type on HCT, $\Delta T_d$, and $T_d$.

Table 9

| Hybotrope | Conc. | Oligo Length | HCT | $\Delta T_d$ | $T_d$ |
|---|---|---|---|---|---|
| GuSCN | 0.5 M | 24-mer wt | 12.5 C | | 70 C |
| GuSCN | 0.5 M | 24-mer mt | 10.0 C | 4.0 C | 74 |
| GuSCN | 1.0 M | 24-mer wt | 12.5 C | | 65 |
| GuSCN | 1.0 M | 24-mt mt | 10.0 C | 3.5 C | 68.5 |
| GuSCN | 2.0 M | 24-mer wt | 12.5 C | | 52 |
| GuSCN | 2.0 M | 24-mer mt | 10.0 C | 4.0 C | 56 |
| GuSCN | 2.5 M | 24-mer wt | 12.5 C | | 46.5 |
| GuSCN | 2.5 M | 24-mer mt | 12,5 C | 3.5 C | 50 |

The data from Table 9 is graphically represented in Figure 9.

Because $\Delta T_d$ does not change over a wide concentration range for the hybotropic solutions described above, a wide temperature range can be employed for conducting oligonucleotide-based assays *(i.e.,* 20 to 80°C). In addition, relatively low concentrations *(e.g.,* 0.5 M) of oligonucleotide can be employed in assays and polymerase based assays.

EXAMPLE 4

DETECTION OF A SINGLE BASE-PAIR MISMATCH ON A SOLID PHASE.

This example describes the detection of a single-base pair mismatch in an immobilized probe using complementary fluorescently labeled oligonucleotides. The set of probe oligonucleotides consists of one probe which forms perfect base-pairing and one oligonucleotide which contains the mismatch when hybridized. The two oligonucleotides are labeled with different fluorochromes, and after hybridization is allowed to occur at the $T_d$ of the mismatch, the ratio of hybridized fluorochromes is determined.

A "target" oligonucleotide (DM0501: 5'-TTGATTCCCAATTATGCGAAGGAG-3') was immobilized on a set of solid supports. ODN-beads (3/32nd inch diameter) were prepared as previously described (Van Ness et al., *Nucl. Acids Res. 19*:3345, 1991). The ODN-beads contained 0.01 to 1.2 mg/bead of covalently immobilized ODN. DMO578 is the complement to DMO501 (perfect complement). DMO1969 is the complement to DM0501 with a G--->T change at position 11. DMO1971 is the complement to DM0501 with a A--->T change at position 12. Each probe oligonucleotide was labeled with either BODIPY, TAMRA or Texas Red. Hybridization reactions were assembled in 3 M GuSCN, 0.01 M Tris pH 7.6, 5 mM EDTA at 50 ng/ml respective probe. Equal molar ratios of each probe type were used in each hybridization in the presence of 3 solid supports per tube. Hybridizations are performed at 42°C for 30 minutes with constant agitation. The beads were washed twice with 3 M GuSCN at 42°C and then with SDS/FW 5 times.

To denature the probe oligonucleotide. the solid supports are placed in 200 μl TE (TE is 0.01 M Tris, pH 7.0, 5 mM EDTA). The mixture is incubated for 10 minutes at 100°C. Fluorescence is measured in a black microtiter plate. The solution is removed from the incubation tubes (200 microliters) and placed in a black microtiter plate (Dynatek Laboratories. Chantilly, VA). The plates are then read directly using a Fluoroskan II fluorometer (Flow Laboratories, McLean, VA) using an excitation wavelength of 495 nm and monitoring emission at 520 nm for fluorescein, using an excitation wavelength of 591 nm and monitoring emission at 612 nm for Texas Red, and using an excitation wavelength of 570 nm and monitoring emission at 590 nm for lissamine or TAMRA.

The results are as follows:

Table 10

| Probe Mix | Fluorochrome ratio in hybridization mix | Fluorochrome ratio after denaturing |
|---|---|---|
| 578TR/578BD | 1.9/1 | 1.9/1 |
| 578TR/1969BD | 2.0/1 | 25/1 |
| 578TR/1971TA | 0.025/1 | 0.58/1 |
| 578BD/1971TA | 0.014/1 | 0.48/1 |

The results indicate that there is no effect of the fluorochrome on the hybridization as indicated in line 1 that Texas Red (TR) 578 oligonucleotide and 578-BD (BODIPY) competed evenly for hybridization to the immobilized target since the ratio of labels did not change after hybridization. There is an average of a 20-fold enrichment of perfectly based probes over the mismatched probes in GuSCN allowing certain detection of base-pair mismatches.

EXAMPLE 5

DETERMINATION OF THE HELICAL COIL TRANSITIONS IN PCR AND LOW MOLARITY HYBOTROPE SOLUTIONS.

The observation that $\Delta T_d$ does not change as a function of concentration of hybotrope has substantial utility for uses in DNA, RNA or nucleic acid amplifications based on primer extension by polymerases *(i.e.,* polymerase chain reaction). The observation that mismatched probes as long as 30-mer oligonucleotides can be distinguished on the basis of thermal melting in 0.5 M LiTCA, permits the possibility of a substantial improvement in priming efficiency in the PCRs. In its current configuration, the PCR buffer is optimized for the polymerase rather for specific priming.

Commercially available PCR buffers were examined with respect to the melting behavior of 18-mers, 24-mers and 30-mers in both the wild-type (wt) and mutant (mt) forms. In Table 11, the level of discrimination achieved in PCR buffer versus a low molarity concentration of hybotrope is presented.

Table 11:

| ΔTd for PCR Buffers and Low Molarity Hybotropes | | | | | |
|---|---|---|---|---|---|
| Hybotrope | Conc. | Oligo Length | HCT | $\Delta T_d$ | $T_d$ |
| PCR buffer | 1x | 24-mer wt | 15 C | | 61 C |
| PCR buffer | 1x | 24-mer mt | 14 C | 1C | 60 C |
| LiTCA 0.1 M | | 24-mer wt | 12 C | | 65.5 |
| LiTCA 0.1 M | | 24-mer wt | 8 C | 4 C | 61.5 |

Note that the HCT for standard PCR buffer is about 15°C whereas the HCT for 0.1 M LiTCA is about 12°C. The $\Delta T_d$ for the 1x PCR buffer is 1°C for the 24-mer whereas the $\Delta T_d$ in 0.1 M LiTCA is 4°C. Thus, priming specificity is significantly improved in a 0.1 M LiTCA versus 1X PCR buffer.

EXAMPLE 6

INTRODUCTION OF AN ABASIC SITE INTO AN OLIGONUCLEOTIDE TO INCREASE THE HCT OF THE OLIGONUCLEOTIDE AND IMPROVE PRIMING SPECIFICITY

It is shown in Example 3 that the introduction of an abasic site or mismatched site into an oligonucleotide primer will decrease the $T_d$ and HCT of the respective derived primer compared to a perfected based pair "sister" primer. Abasic sites in polynucleotides of oligonucleotides can be introduced by the chemical or enzymatic hydrolysis of the glycosidic bond. The resulting structure is apurinic or apyrimidinic which lacks the coding information and fails to base pair. The CE phosphoramidite of the tetrahydroduran derivative is commercially available (dSPACER, Glenn Research, Sterling, Virginia) as well as other spacer phosphoramidites (Glenn Research, Sterling, Virginia).

Effect of abasic substitution on the HCT of set of oligonucleotides is shown in the Table below.

Table 12

| Buffer Type | Oligo Type | HCT* | $T_d$* | Stringency Factor |
|---|---|---|---|---|
| 1X PCR buffer | normal | 18 | | |
| 1X PCR buffer | abasic (dSPACER) | 12 | | |
| 1X PCR buffer | abasic (C3 spacer) | 12 | | |
| 0.5 M TMATCA | normal | 14 | | |
| 0.5 M TMATCA | abasic (dSPACER) | 8 | | |
| 0.5 M TMATCA | abasic (C3 spacer) | 8 | | |
| 2.0 M LiTCA | normal | 12.5 | 44.5 | 4.97 |
| 2.0 M LiTCA | abasic (dSPACER) | 10 | 39 | 6.37 |
| 2.0 M LiTCA | abasic (C3 spacer) | 10 | 39 | 6.25 |
| 3.0 M GuSCN | normal | 16 | 35.5 | 3.85 |
| 3.0 M GuSCN | abasic (dSPACER) | 12.5 | 32 | 5.24 |
| 3.0 M GuSCN | abasic (C3 spacer) | 12.5 | 31 | 5.31 |
| * = °C | | | | |

The oligonucleotide is a 24-mer with the following sequence: 5'-hexylamine-TGTGGATCAGCA-spacer-GCAGGAGTATG-3' where the spacer is either the C3-spacer or dSPACER from Glenn Research (Sterling, VA).

EXAMPLE 7

DETECTION OF A SINGLE BASE-PAIR MISMATCH ON A SOLID PHASE USING ABASIC SUBSTITUTED OLIGONUCLEOTIDES

This example describes the hybridization of an oligonucleotide containing an abasic site to an immobilized oligonucleotide using fluorescent tags. The set of probe oligonucleotides consists of one probe which forms perfect base-pairing and one oligonucleotide which contains the an abasic site when hybridized. The two oligonucleotides are labeled with different fluorochromes, and after hybridization at the Td of the mismatch, the ratio of hybridized fluorochromes is determined.

A "target" oligonucleotide (DMO501: 5'-TTGATTCCCAATTATGCGAAGGAG-3') was immobilized on a set of solid supports. ODN-beads (3/32nd inch diameter) were prepared as previously described (Van Ness et al., Nuc. *Acids Res. 19*:3345, 1991). The ODN-beads contained 0.01 to 1.2 mg/bead of covalently immobilized ODN. DMO578 is the complement to DM0501 (perfect complement). DMO1969 is the complement to DM0501 with an abasic site at position 11. DMO1971 is the complement to DM0501 with an abasic site at position 12. Each probe oligonucleotide is labeled with either BODIPY, TAMRA or Texas Red. Hybridization reactions were assembled in 3 M GuSCN, 0.01 M Tris pH 7.6, 5 mM EDTA at 50 ng/ml respective probe. Equal molar ratios of each probe type were used in each hybridization in the presence of 3 solid supports per tube. Hybridizations were at 42°C for 30 minutes with constant agitation. The beads were washed twice with 3 M GuSCN at 42°C and then with SDS/FW 5 times.

To denature the probe oligonucleotide, the solid supports were placed in 200 µl TE (TE is 0.01 M Tris, pH 7.0, 5 mM EDTA). The mixture is incubated for 10 minutes at 100°C. Fluorescence is measured in a black microtiter plate. The solution is removed from the incubation tubes (200 microliters) and placed in a black microtiter plate (Dynatek Laboratories, Chantilly, VA). The plates are then read directly using a Fluoroskan II fluorometer (Flow Laboratories, McLean, VA) using an excitation wavelength of 495 nm and monitoring emission at 520 nm for fluorescein, using an excitation wavelength of 591 nm and monitoring emission at 612 nm for Texas Red. and using an excitation wavelength of 570 nm and monitoring emission at 590 nm for lissamine or TAMRA.

The results are as follows:

Table 13

| Probe Mix | Fluorochrome ratio in hybridization mix | Fluorochrome ratio after denaturing |
|---|---|---|
| 578TR/578BD | 2.1/1 | 2.1/1 |
| 578TR/1969BD | 1.8/1 | 25/1 |
| 578TR/1971TA | 0.024/1 | 0.6/1 |
| 578BD/1971TA | 0.015/1 | 0.36/1 |

The results indicate that there is no effect of the fluorochrome on the hybridization as indicated in line 1 that Texas Red (TR) 578 oligonucleotide and 578-BD (BODIPY) competed evenly for hybridization to the immobilized target since the ratio of labels did not change after hybridization. There is an average of a 20-fold enrichment of perfectly based probes over the abasic modified in GuSCN; allowing much higher levels of discrimination in hybridization reactions.

EXAMPLE 8

EFFECTS OF INTRODUCING A DEOXYNEBULARINE RESIDUE INTO AN OLIGONUCLEOTIDE PRIMER USED IN AMPLIFICATION

This example describes the use of oligonucleotide primers substituted with either abasic or deoxyNebularine residues to increase the specificity of priming in amplification reactions.

The primers used in this experiment are described by Rychlik (Rychlik, *Biotechniques, 18*:84-90, 1995). Primers may be synthesized or obtained as gelfiltration grade primers from Midland Certified Reagent Company (Midland Texas).

Amplification reactions are either *Taq* DNA polymerase-based (10 mM Tris-HCl pH 8.3, 1.5 mM MgCl$_2$, 50 mM KCl), or *Pfu* DNA polymerase based (20 mM Tris-HCl pH 8.75, 2.0 mM MgCl$_2$, 10 mM KCl, 10 mM (NH$_4$)$_2$SO$_4$, 0.1% Triton X-100, 0.1 mg/ml bovine serum albumin (BSA)). The total deoxynucleoside triphosphate (dNTPs) concentration in the reactions is 0.8 mM, the primer concentration is 200 nM (unless otherwise stated) and the template amount is 0.25 ng of bacteriophage lambda DNA per 25 µl reaction. The amplification cycles consist of a denaturation step at 94°C for 5 minutes followed by 30 cycles of: 94°C for 45 seconds, 52°C for 45 seconds, at 72°C for 30 seconds, followed by a single step of 72°C for 5 minutes. Amplified DNA fragments are electrophoresed along with DNA standards through a 2% agarose gel in 0.5 X TBE buffer (45 mM Tris-borate, pH 8.0, 0.1 mM EDTA) and visualized after staining with ethidium bromide. DNA is quantitated by densitometry. Each experiment is performed twice.

Two regions in the bacteriophage lambda DNA sequence (GenBank Accession #J02459) are chosen as the priming sites for amplification. The 5' primer has a stable GC-rich 3' end; the 3' primer is chosen so that a 381 bp product will result from the amplification. The primers used in this example are as follows:

Forward (5') primers:

H17: 5'-GAACGAAAACCCCCCGC-3'

H14: 5'-CTTCGAAAACCCCCCGC-3'

H11: 5'-CTTGCTAAACCCCCCGC-3'

AB1: 5'-GAACGA(dS)AACCCC(dS)CGC-3'

AB2: 5'-GAACGA(dS)AACCC(dS)CCGC-3'

AB3: 5'-GAACGA(dS)AACCCCCCG(dS)C-3'

DN1: 5'-GAACGA(dS)AACCCC(dN)CGC-3'

DN2: 5'-GAACGA(dNAACCC(dN)CCGC-3'

DN3: 5'-GAACGA(dN)AACCCCCCG(dN)C-3'

DN4: 5'-GAACG(dN)AAACCC(dN)CCGC-3'

DN5: 5'-GAACG(dN)AAACC(dN)CCCGC-3'

DN6: 5'-CTTCGAAAACCC(dN)CCGC-3'

Reverse (3') primer:

reverse: 5'-GATCGCCCCCAAAACACATA-3'

(dS) represents "dSPACER" residue and (dN) represents deoxyNebularine residue.

The forward primers are designed with their 5' ends variably mismatched to the target DNA. The H17 primer is a perfect match to the intended target, whereas the primer H14 is complementary only for the 14 nucleotides at the 3' end (the 3 nucleotides at the 5' end are mismatched). All of the primer pairs are used in separate amplification reactions, and the annealing temperature is varied from 25°C to 65°C. A set of typical results are presented in the following table. Similar results are obtained for both *Taq* and *Pfu* polymerases.

Table 14

| Primer Name | Number mismatches @ position in primer | Substitutions | Temp. Range (°C) that amplifications observed |
|---|---|---|---|
| H17 | none | none | 25 ---> 65 |
| H14 | 3 @ 5' | none | 25 ---> 65 |

Table 14   (continued)

| Primer Name | Number mismatches @ position in primer | Substitutions | Temp. Range (°C) that amplifications observed |
|---|---|---|---|
| H11 | 6 @ 5 | none | 25 ---> 50 |
| AB1 | 2, @ 7, 14 | dSpacer | no amplification |
| AB2 | 2, @ 7, 13 | dSpacer | no amplification |
| AB3 | 2, @ 7, 16 | dSpacer | no amplification |
| DN1 | 2, @ 7, 14 | dNeb. | 25 ---> 35 |
| DN2 | 2, @ 7, 13 | dNeb. | 25 ---> 35 |
| DN3 | 2, @ 7, 16 | dNeb. | 25 ---> 30 |
| DN4 | 2, @ 6, 13 | dNeb. | 25 ---> 35 |
| DN5 | 2, @ 6, 12 | dNeb. | 25 ---> 35 |
| DN6 | 2, 3 @ 5', 13 | dNeb. | 25 ---> 30 |

These results indicate that the dSpacer substitution prevents the *Taq* or *Pfu* DNA polymerase from "reading through" the abasic site. That is, when the polymerase encounters an abasic residue, chain extension is terminated. Therefore, the priming site is not conserved during the second strand synthesis, and amplification of the target nucleic acid is not achieved. However, the polymerases can read through deoxyNebularine residues present in the oligonucleotide primers. Most likely, but not verified, deoxythymidine is inserted as the complementary base to deoxyNebularine. However, the temperature range over which amplification is achieved is reduced compared to the temperature range for amplification using the H17 primer (from 25°C - 65°C down to 25°C to approximately 35°C). It is therefore apparent that the deoxyNebularine substituted primers can substantially increase the specificity of the PCR reaction. Priming was improved which led to the amplification of a specific amplicon.

In a second series of experiments, the primer pairs are used in separate amplification reactions utilizing an annealing temperature of 42°C. The results are presented in Figure 12. Similar results are obtained for both the Taq and Pfu polymerases. As expected, the H17, H14 and H11 primers all give rise to a 381 bp amplicon, despite the 3 base mismatches at the 5' end for the H14 primer and the 6 base mismatches at the 5' end for the H11 primer. As above, no amplification is observed using the AB1 primer containing abasic residues. In contrast, the DN1, DN2, and DN3 primers all give rise to a 381-bp amplicon. although no amplification is observed using the DN6 primer, probably due to the mismatch of 3 bases at the 5'-end of the primer and the deoxyNebularine substitution at the 3' end of the primer. Thus, deoxyNebularine substituted primer can greatly increase the specificity of priming in the polymerase chain reaction.

EXAMPLE 9

EFFECTS OF INTRODUCING A DEOXYNEBULARINE RESIDUE INTO AN OLIGONUCLEOTIDE ON THE HCT OF THE OLIGONUCLEOTIDE

In Example 3, the introduction of an abasic site or mismatched site into an oligonucleotide primer decreases the $T_d$ and HCT of the modified primer as compared to a perfectly based pair "sister" primer. The effect of deoxyNebularine substitutions on the HCT is also investigated.

DeoxyNebularine modified oligonucleotides can be synthesized by standard methods utilizing phosphoramidites. The CE phosphoramidite of the tetrahydroduran derivative, as well as other spacer phosphoramidites are commercially available (deoxyNebularine, Glenn Research, Sterling, Virginia). The oligonucleotide for the following experiments is synthesized as a 24-mer having the following sequence: 5'-hexylamine-TGTGGATCAGCA(dN)GCAGGAGTATG-3'.

The effect of the deoxyNebularine (dN) substitution on the HCT of a set of oligonucleotides is shown in the Table below.

Table 15

| Buffer Type | Oligo Type | $\Delta$BCT* | $T_d$* | Stringency Factor |
|---|---|---|---|---|
| 1X PCR buffer | normal | 18 | | |
| 1X PCR buffer | abasic | 12 | | |
| 1X PCR buffer | deoxyNebularine | 12 | | |
| 0.5 M TMATCA | normal | 14 | | |
| 0.5 M TMATCA | abasic | 8 | | |
| 0.5 M TMATCA | deoxyNebularine | 8 | | |
| 2.0 M LiTCA | normal | 12 | 44 | 5.0 |
| 2.0 M LiTCA | abasic | 10 | 39 | 6.3 |
| 2.0 M LiTCA | deoxyNebularine | 10 | 39 | 6.3 |
| 3.0 M GuSCN | normal | 16 | 35 | 3.9 |
| 3.0 M GuSCN | abasic | 12.5 | 32 | 5.2 |
| 3.0 M GuSCN | deoxyNebularine | 12.5 | 31 | 5.3 |

* = °C

The deoxynebularine substituted oligonucleotide showed the same decrease in the HCT as the abasic substituted oligonucleotide.

EXAMPLE 10

DETECTION OF A SINGLE BASE-PAIR MISMATCH ON A SOLID PHASE USING DEOXYNEBULARINE SUBSTITUTED OLIGONUCLEOTIDES

This example describes the hybridization of an oligonucleotide containing a deoxyNebularine site to an immobilized oligonucleotide (target). The set of probe oligonucleotides consists of one probe that is perfectly complementary to the target, and a second oligonucleotide that contains a deoxyNebularine site. The probe oligonucleotides are labeled with fluorescent tags to aid in detection of hybridization. For this data, the two oligonucleotides are labeled with different fluorochromes, and after hybridization at the $T_d$ of the mismatch, the ratio of hybridized fluorochromes is determined.

A target oligonucleotide, 5'-TTGATTCCCAATTATGCGAAGGAG-3' (DMO501), is immobilized on a solid support. Oligonucleotide containing beads (ODN-beads) that are 3/32nd inch diameter are prepared as previously described (Van Ness et al., *Nuc. Acids Res. 19.*3345, 1991). The ODN-beads contain from 0.01 to 1.2 mg/bead of covalently immobilized ODN. Probe oligonucleotides include DM0578, which is the perfect complement to DMO501. DMO1969. which is the complement to DM0501 but has a deoxyNebularine residue at position 11, DMO1971, which is the complement to DM0501 but has a deoxyNebularine site at position 12. Each probe oligonucleotide is labeled with either BODIPY, TAMRA or Texas Red. Hybridization reactions contain 50 ng/ml of each probe in a solution comprising 3 M GuSCN, 0.01 M Tris pH 7.6, and 5 mM EDTA. Equal molar ratios of each probe are used for each hybridization to 3 solid supports contained in a tube. Hybridizations are carried out at 42°C for 30 minutes with constant agitation. The beads are washed twice with 3 M GuSCN at 42°C followed by five washes of SDS/FW.

To denature the probe/target duplexes, the solid supports are placed in 200 µl TE (0.01 M Tris, pH 7.0, 5 mM EDTA) and incubated for 10 minutes at 100°C. The solution (200 µl) is removed from the incubation tubes and placed in a black microtiter plate (Dynatek Laboratories, Chantilly, VA) for measurement of fluorescence. The plates are then read directly in a Fluoroskan II fluorometer (Flow Laboratories, McLean, VA) using an excitation wavelength of 495 nm and monitoring emission at 520 nm for fluorescein, using an excitation wavelength of 591 nm and monitoring emission at 612 nm for Texas Red, and using an excitation wavelength of 570 nm and monitoring emission at 590 nm for lissamine or TAMRA.

The results are presented in the following table:

Table 16

| Probe Mix | Fluorochrome ratio in hybridization mix | Fluorochrome ratio after denaturing |
|---|---|---|
| 578TR/578BD | 5.6/1 | 5.6/1 |
| 578TR/1969BD | 2.0/1 | 36/1 |
| 578TR/1971TA | 0.018/1 | 0.7/1 |
| 578BD/1971TA | 0.022/1 | 0.48/1 |

The results indicate an average of 20-fold enrichment of perfectly based probes over the deoxyNebularine modified probes in GuSCN; allowing much higher levels of discrimination in hybridization reactions. This enrichment is not due to the presence of the fluorochrome, as the fluorochrome has no measurable effect on the hybridization. As indicated in line 1, Texas Red (TR) 578 oligonucleotide and 578-BD (BODIPY) competed equivalently for hybridization to the immobilized target as evidenced by the same ratio of labels before and after hybridization.

EXAMPLE 11

DETECTION OF A SINGLE BASE-PAIR MISMATCH ON A SOLID PHASE USING ABASIC SUBSTITUTED OLIGONUCLEOTIDES

This example describes the use of abasic substituted oligonucleotide probes to detect single base pair mismatches. As shown herein, an increase in efficiency is observed in detecting single base-pair mismatches using abasic substituted oligonucleotide probes as compared to standard probes.

Target oligonucleotides are covalently attached to membrane filters (Magna Graph nylon membrane filters, Micron Separations, Westboro, MA) (Van Ness et al., *Nuc. Acids Res. 19*:3345, 1991). The target oligonucleotides are based on the sequence: 5'-TGTGGATCAGCAAGCAGGAGTATC-3' and contain either a G→A. T→C, T→T, G→T, or T→G mismatch at positions 13 or 14 in the target oligonucleotides. After attachment of the oligonucleotides to the membrane. the sheet is blocked for 10 min with gentle mixing in a succinnic anhydride solution (2.5 g of succinnic anhydride dissolved in 25 ml m-pyrol mixed with 125 ml 0.1 M NaBorate pH 8.5). The sheets are then washed 5 times with a solution of 10 mM Tris, 5 mM EDTA (TE). The sheets are additionally blocked for 30 min with gentle mixing with a solution of 1% bovine serum albumin (Fraction 5, Sigma) and containing 100 μg/ml fragmented. single strand herring sperm DNA. The sheets were then washed 5 times in TE.

The following biotinylated probes

control probe: 5'-ACACCTAGTCGTTCGTCCTCATAC-3',

8S abasic probe: 5'-ACACCT(dS)GTCGTTCGTCCTCATAC-3', and

6S abasic probe: 5'-ACACCT(dS)GTCGTTCGTCCTC(DS)TAC-3'

are added to the sheet at a final concentration of 10 ng/ml in 1 ml of 3 M GuSCN, and the sheets are incubated at 28°C for 30 minutes. The sheets are then rinsed four times in 1xSSC/0.1% SDS for 1 minute each wash, followed by two rinses in Wash Solution (0.01 M Tris pH 7.2, 0.1 M NaCl, 0.005 M EDTA, 0.1% Tween 20).

The streptavidin/alkaline phosphatase conjugate (Vector, Burlingarne. CA) is diluted 1:10,000 in wash solution. The sheets are then incubated in this solution for I hour at room temperature with shaking. The sheets are subsequently rinsed four times with wash solution and once with detection buffer (0.1 M NaCl, 0.01 M Tris pH 8.5, 0.05 M $MgCl_2$) for 5 minutes. The alkaline phosphatase substrate is prepared by dissolving a BCIP/NBT tablet (Schleicher and Schuell, part #78349, Keene, NH) in 30ml $dH_2O$. The reaction is carried out for 0.5 to 4 hours at room temperature. The sheets are then rinsed with water and dried. A text scanner is used to detect signal.

As shown in Figure 13, hybridization to the control probe is observed for each target oligonucleotide, even for those that are mismatched. However, the 6S abasic-modified probe hybridized nearly exclusively to the perfect match target oligonucleotide. The 8S abasic-modified probe also hybridized preferentially to the perfect match target oligonucleotide. The density for each target is presented in the following table in relative intensity units:

Table 17

| Oligo | None | G/A | C/T | T/T | G/T | T/G |
|---|---|---|---|---|---|---|
| Control | 90 | 82 | 26 | 90 | 91 | 45 |
| 6S | 52 | 0 | 0 | 0 | 10 | 3 |
| 8S | 76 | 35 | 2 | 35 | 45 | 30 |

The following table presents the ratio of mismatch density to control density.

Table 18

| Oligo | None | G/A | C/T | T/T | G/T | T/G |
|---|---|---|---|---|---|---|
| Control | 1 | 0.91 | 0.28 | 1 | 1.01 | 0.5 |
| 6S | 1 | 0 | 0 | 0 | 0.19 | 0.06 |
| 8S | 1 | 0.46 | 0.03 | 0.46 | 0.59 | 0.39 |

EXAMPLE 12

HIGH THROUGHPUT ANALYSIS OF HELICAL COIL TRANSITIONS OF OLIGONUCLEOTIDES.

A capture oligonucleotide (36-mer) was covalently linked to nylon bead via a C6-amine tail as previously described (Van Ness et al., *Nuc. Acids Res. 19*:3345, 1991). Oligonucleotides (of various lengths as described in the text) were labeled via a C6 amine spacer with Texas Red (fluorescein, lissamine or TAMRA can also be used) and were hybridized to the capture oligonucleotide in a 1.5M guanidinium thiocyanate solution (other hybridization solutions as described in the text can also be used).

Specifically, the "signal" oligonucleotide was synthesized by Midland Certified Reagent Company (Midland, Texas) at 1µM scale. The oligo was diluted to 250 µL in TE buffer which was used as a stock solution. The signal oligo was further diluted for hybridization by removing 25 µL of the stock solution and mixing it into 975uL of 1.5M guanidinium thiocyanate solution (other hybridization solutions as described in the text can also be used). This working stock was aliquoted into a Cetus tube (100uL/tube). A nylon pin was immersed in the solution for 15 minutes at ambient temperature to allow the signal oligo to hybridize to the immobilized capture oligo. The beads were then washed to remove unhybridized signal oligonucleotide 1x with 0.01 M Tris pH 7.0, 5 mM EDTA, and 0.1 M NaCl; 2x with 0.01 M Tris pH 7.0, 5 mM EDTA, 0.1 M NaCl, and 0.1% SDS; lx with 0.01 M Tris pH 7.0, 5 mM EDTA, and 0.1 M NaCl (TEN: 0.01M Tris pH 7.5 , 1mM EDTA, 100mM NaCl; TENS: 0.01M Tris pH 7.5 , 1mM EDTA, 100mM NaCl, 0.1% SDS).

Test solutions were aliquoted into wells of a polycarbonate thermowell plate (Corning Costar Corp., Cambridge, MA) and the plate placed in an MJ thermal cycler (MJ Research Company, Watertown, MA). The beads were serially transferred between the wells of the plate; every 2.5 to 5 minutes the temperature increases by 5°C starting at 10°C and reaching 85 to 100°C at the final point. After the melting process was completed, the liquid in the polycarbonate thermowell plates was transferred to a black 96 well microtiter plate (Dynatek Laboratories, Chantilly, VA). The plates were then read directly using a Fluoroskan II fluorometer (Flow Laboratories, McLean, VA) using an excitation wavelength of 495 nm and monitoring emission at 520 nm for fluorescein, using an excitation wavelength of 591 nm and monitoring emission at 612 nm for Texas Red, and using an excitation wavelength of 570 nm and monitoring emission at 590 nm for lissamine or TAMRA. The level of fluorescence correlates with the amount of signal oligonucleotide that has melted from the capture oligo.

To calculate the $T_d$, cumulative counts eluted at each temperature were plotted against temperature. The temperature at which 50% of the material dissociates from the bead is the $T_d$. The data was exported into a spreadsheet and melt curves were generated for each solution. From these melt curves. $T_d$, $\Delta HCT$, and $\Delta T_d$ were calculated.

EXAMPLE 13

IDENTIFICATION OF HYBRIDIZATION SOLUTIONS WHICH EFFECTIVELY NEUTRALIZE THE G+C CONTENT OF NUCLEIC ACID DUPLEXES.

This example describes the identification and use of novel compounds that reduce or eliminate the effects of G+C content on the melting behaviour of nucleic acid duplexes.. Also, as shown herein, an increase in efficiency is observed in detecting single base-pair mismatches using modified oligonucleotide probes as compared to standard probes.

Solutions and Reagents

Filter wash (FW) is 0.09 M NaCl, 540 mM Tris pH 7.6, 25 mM EDTA. SDS/FW is FW with 0.1% sodium dodecyl sulfate (SDS). Hybridization solutions contain the text specified concentration of hybotrope of G+C neutralizing compound, 0.1 to 2% N-lauroylsarcosine (sarcosyl), 50 mM Tris pH 7.6 (in some cases) and 0.5 to 25 mM EDTA. Formamide hybridization solution contains 30% formamide, 0.09 M NaCl, 40 mM Tris-HCl pH 7.6, 5 mM EDTA and 0.1% SDS. GuSCN is purchased from Kodak (Rochester, NY). GuCl, lithium hydroxide, trichloroacetic acid, NaSCN, $NaClO_4$ and KI, are purchased from Sigma (St. Louis, MO). CsTFA is purchased from Pharmacia (Piscataway, NJ). The amine based compounds were purchased from Sigma (St. Louis, MO), Aldrich (Milwaukee, WI) or from Fluka (Ronkonkoma, NY)

Preparation of LiTCA, TMATCA and TEATCA and other Amine-based TCA, TFA and acetate salts.

LiTCA and TMATCA, and TEATCA are prepared by the dropwise titration of a 3 N solution of LiOH, TEAOH and TMAOH respectively, with trichloracetic acid (100% w/v, 6.1 N) to pH 7.0 on ice with constant stirring. The salt is evaporated to dryness under vacuum, washed once with ether and dried. The acetate, trichoroacetate, or trifluoroacetate salts of the amine containing compounds were synthesized by neutralizing the respective amines with acetic acid, trichloroacetic acid or with trifluoroacetate to pH 6.0 to pH 8.5, depending upon the application. The resulting salt solution was then diluted to the concentration desired as stated in the figures or tables in this example. In some cases the salt was then dissolved in water to a final concentration of 0.1 to 3.0 M. The resulting salt solution was in some cases then buffered with Tris-HCl, pH 7.0-8.5, and detergents, such as sarkosyl, are added to about 0.1%, and optionally EDTA is added to 0.5 to 5 mM. The oligonucleotide that was tethered to the bead was DMO-2060: 5'-hexylamine-GTC/ ATA/CTC/CTG/CTT/GCT/GAT/CCA/CAT/CTG-3' (oligonucleotide immobilized on the nylon bead); abd the probe oligonucleotides were: DMO-2055: 5'-TEXAS RED- TGT/GGA/TCA/GCA/AGC/AGG/AGT/ATG-3' (perfect complement); DMO-2058; 5'-TEXAS RED- TGT/GGA/TCA/GGAJAGC/AGG/AGT/ATG-3' (mismatch complement); and DMO-2058-dN: 5'-TEXAS RED-TGT/GGA/TCA/G(deoxynebularine)A/AGC/AGG/AGT/ATG-3' (deoxynebularine mismatch complement).

Table 19

| Stock Solution | Stock pH | 1 M $\Delta$Tm (27-83 GC) | 500mM $\Delta$Tm (27-83 GC) | 100mM $\Delta$Tm (27-83 GC) |
|---|---|---|---|---|
| 1-ethylpiperidine acetate | 7 | 0 | 1 | 6 |
| 1-ethylpiperidine trichloroacetate | 8.6 | -1 | 0 | 5 |
| 1-ethylpiperidine trifluoroacetate | 6.6 | -1 | 0 | 5 |
| 1-methylimidizole acetate | 6.6 | 8 | 7 | 14 |
| 1-methylpiperidine acetate | 7 | 4 | 14 | 7 |
| 1-methylpiperidine trichloroacetate | 8.4 | 3 | 4 | 8 |
| 1-methylpynrolidine acetate | 7 | -1 | -5 | 7 |
| 1-methylpyrrolidine trichloroacetate | 8.4 | 6 | 7 | 8 |
| 1-methylpyrrolidine trifluoroacetate | 7.2 | 2 | 4 | 8 |
| 2-methoxyethylamine acetate | 7 | 9 | -1 | 6 |
| 2-methaxyethylamine trifluoroacetate | 7.6 | 3 | 11 | 12 |
| 3-methoxypropylamine acetate | 6.8 | 9 | 3 | 6 |
| betaine in 1X TE | 7.7 | 9 | 13 | 11 |
| Bis(2-methoxyethyl)amine acetate | 6.2 | 4 | 5 | 4 |
| bis(2-methoxyethyl)amine trifluoroacetate | 7.6 | 3 | 5 | 9 |

Table 19  (continued)

| Stock Solution | Stock pH | 1 M $\Delta$Tm (27-83 GC) | 500mM $\Delta$Tm (27-83 GC) | 100mM $\Delta$Tm (27-83 GC) |
|---|---|---|---|---|
| Diallylamine acetate | 6.5 | 2 | 2 | 5 |
| diallylamine trifluoroacetate | 7.6 | 5 | 4 | 8 |
| dibutylamine acetate | 6.5 | 3 | 4 | 4 |
| Dicyclohexylamine Acetate | 6.7 | 3 | 4 | 5 |
| diisobutylamine acetate | 6.6 | 3 | 6 | 5 |
| diisopropylamine acetate | 6.9 | 1 | 2 | 4 |
| diisopropylamine trifluoroacetate | 6.9 | 6 | 2 | -1 |
| dipropylamine acetate | 6.5 | 2 | 4 | 4 |
| N,N,N',N'-tetraethylethylenediamine acetate | 7.3 | 0 | 3 | 2 |
| n,n-dimethylaminobutane acetate | 7 | 3 | 2 | 5 |
| n,n-dimethylaminobutane trichloroacetate | 8.2 | 5 | 4 | 9 |
| n,n-dimethylaminobutane trifluoroacetate | 6.2 | -1 | 7 | 0 |
| n,n-dimethylbutylamine acetate | 6.9 | 0 | 2 | 6 |
| n,n-dimethylcyclohexylamine acetate | 7.1 | 1 | 4 | 5 |
| n,n-dimethylcyclohexylamine trifluoroacetate | 7.3 | -5 | 13 | 12 |
| n,n-dimethylcyclohexylamine/TE/Sark | | 2 | 3 | 8 |
| n,n-dimethylheptylamine acetate | 6.5 | 3 | 5 | 5 |
| n,n-dimethylheptylamine acetate | 7.7 | 5 | 6 | 10 |
| n,n-dimethylhexylamine acetate | 6.6 | 3 | 3 | 3 |
| n,n-dimethylhexylamine acetate | 7.1 | 4 | 2 | 10 |
| n,n-dimethylisopropylamine acetate | 6.9 | 3 | 6 | 9 |
| n,n-dimethylisopropylamine trichloroacetate | 8.5 | 2 | 3 | 13 |
| n,n-dimethyloctylamine trifluoroacetate | 7 | 5 | 6 | -2 |
| n-ethylbutylamine acetate | 6 | 1 | 1 | 6 |
| n-ethylbutylamine trifluoroacetate | 6.1 | 6 | 6 | 8 |
| triethanolamine acetate | 6.5 | -4 | 4 | 12 |
| triethylamine acetate | 7 | 2 | 2 | 7 |
| triethylamine trichloroacetate | | 5 | 9 | 8 |
| tripropylamine acetate | 6.5 | -1 | 4 | 7 |
| tetraethylamonium acetate | | 0 | 8 | |
| tetra ethylamonium acetate 3M | | -3 | | |
| formamide 20%/TE/Sark | | 14 | | |
| 1X PCR Buffer | | 14 | | |
| 1X SSC | | 13 | | |

As shown in Table 19 numerous amine-based hybridization solutions (in the 100 mM concentration range, 500 mM concentration range and 1000 mM concentration range) have been identified which give rise to a ATd of 9 °C or less between oligonucleotide duplexes of G+C content of 27% to 83%. Novel hybridization solutions were prepared which demonstrate properties not previously described for a hybridization solution.

These hybridization solutions possess the property of neutralizing the differences in G+C and A+T base-pairing strength. Some of the solutions (most tripropylamine acetate, bis(2-methoxyethyl)amine trifluoroacetate, disopropylamine trifluoroacetate, n,n dimethylaminobutane trifluoroacetate at 100 mM; triethanolamine acetate, noteably n,n dimethylcyclohexylamine trifluoroacetate, n,n dimethylheptylamine acetate at 500 mM; noteably n,n dimethylcyclohexylamine trifluoroacetate, tripropylamine acetate, dibutylamine acetate, n,n dimethylheptylamine acetate, dimethylhexylamine acetate, dicyclohexylamine acetate at 1000 mM) simultaneously lowers the $T_d$ and $\Delta T_d$, Others such as increase $\Delta T_d$ (1-ethylpiperidine acetate, etc.) In the table below the characteristics of the novel hybridization solutions and hybotropes are described. The following $\Delta T_d$s as a function of G+C content were obtained from the melt curves described below: Novel hybridization solutions have also been identified which neutralize the effects of G+C content on the melting behaviour of nucleic acid duplexes. These solutions are in some cases hybotropes and in other cases can be used as PCR buffers or as hubridization solutions which minimize the effects of G+C content on nucleic acid duplexes. These new hybridization solutions, their properties, and their preparation are described in Example 12. Figure 14 is a graph showing the difference in $T_d$ between three duplexes, that vary in G+C content from 27% to 83%. The capture oligonucleotide is a 36-mer (DMO-GC36cap: 5'-hexylamine-GCA/GCC/TCG/CGG/AGG/CGG/ATG/ATC/GTC/ ATT/AGT/ATT-3') and three complementary oligos which are labelled with the fluorochrome are DMO-83GC: 5'-Texas Red- CCG/CCT/CCG/CGA/GGC/TGC-3'; DMO-50GC: 5'-Texas Red- AAT/GAC/GAT/CAT/CCG/CCT-3'; DMO-27GC: -Texas Red-AAT/ACT/AAT/GAC/GAT/CAT-3'. The temperature difference between any two $T_d$s at a = 0.5 is defined as the $\Delta T_d$. The percentage of single strand DNA (y-axis) is plotted versus temperature (°C; x-axis). The melting solution was 100 mM 2-methoxyethylamine trifluoroacetate. The maximum difference between the 3 melting curves in the $T_d$ was 6 C. The helical coil transition of the 27% G+C content was 21 C, 50% G+C was 33 C and for the 83% G+C duplex was 29 C. Note that the helical coil transitions (HCTs) of the 3 different G+C content oligonucleotides is different. This is in contrast to the case with diisobutylamine as shown in Figure 15. Figure 15 is a graph showing the difference in $T_d$ between three duplexes, that vary in G+C content from 27% to 83% (the same system as described in Figure 14. The temperature difference between any two $T_d$s at a = 0.5 is defined as the $\Delta T_d$. The percentage of single strand DNA (y-axis) is plotted versus temperature (°C; x-axis). The melting solution was 100 mM diisobutylamine acetate. The maximum difference between the 3 melting curves in the $T_d$ was 5 C. The helical coil transition of the 27% G+C content was 22 C, 50% G+C was 26 C and for the 83% G+C duplex was 25 C. The helical coil transitions for the three oligoncletide duplexes are very similar. This is the behaviour that is preferred for use in array hybridizations or polymerase chain reactions.

In Figure 16 the inability of GuSCN to neutralize G+C content is shown. Figure 16 is a graph showing the difference in $T_d$ between three duplexes, that vary in G+C content from 27% to 83% (the same capture and probe oligonucleotides as described in figure 14). The temperature difference between any two $T_d$s at a = 0.5 is defined as the $\Delta T_d$. The percentage of single strand DNA (y-axis) is plotted versus temperature (°C; x-axis). The melting solution was 2 M Guanidinium thiocyanate. The maximum difference between the 3 melting curves in the Td or Tm is 16 C. The helical coil transition of the 27% G+C content was 28 C, for the 50% G+C duplex was 30 C and for the 83% G+C duplex was 32 C. Similar results were obtained with lx PCR buffer (figure 17) and lx SSC buffer (Figure 18). There was also no neutralization of G+C content with 20% formamide (Figure 19). Figure 17 is a graph showing the difference in $T_d$ between three duplexes, that vary in G+C content from 27% to 83% (the same duplex system as described in Figure 14). The temperature difference between any two Tds at a = 0.5 is defined as the $\Delta T_d$. The percentage of single strand DNA (y-axis) is plotted versus temperature (°C; x-axis). The melting solution was lx PCR buffer. The maximum difference between the 3 melting curves in the $T_d$ was 14 C. The helical coil transition of the 27% G+C content was 17 C. for the 50% G+C duplex was 22 C and for the 83% G+C duplex was 23 C. Figure 18 is a graph showing the difference in $T_d$ between three duplexes, that vary in G+C content from 27% to 83%. The temperature difference between any two $T_d$s at a = 0.5 is defined as the $\Delta T_d$. The percentage of single strand DNA (y-axis) is plotted versus temperature (°C; x-axis). The melting solution was lx SSC. The maximum difference between the 3 melting curves in the $T_d$ is 13 C. The helical coil transition of the 27% G+C content was 20 C, for the 50% G+C duplex was 22 C and for the 83% G+C duplex was 23 C. Figure 19 is a graph showing the difference in $T_d$ between three duplexes, that vary in G+C content from 27% to 83%. The temperature difference between any two Tds at a = 0.5 is defined as the $\Delta T_d$. The percentage of single strand DNA (y-axis) is plotted versus temperature (°C; x-axis). The melting solution was 20% formamide, 10 mM Tris pH 7.6, and 5 mM EDTA with 0.1 % sarkosyl. The maximum difference between the 3 melting curves in the $T_d$ is 14 C. The helical coil transition of the 27% G+C content was 15 C, for the 50% G+C duplex was 16 C and for the 83% G+C duplex was 20 C.

In contrast to the situation in Figures 17, 18 and 19, Figure 20 shows the melting behaviour of the 3 different G+C oligonucleotide duplexes in 1 M dicyclohexylamine acetate. Figure 20 is a graph showing the difference in $T_d$

between three duplexes, that vary in G+C content from 27% to 83%. The temperature difference between any two $T_d$s at a = 0.5 is defined as the $\Delta T_d$. The percentage of single strand DNA (y-axis) is plotted versus temperature (°C; x-axis). The melting solution was 1 M dicyclohexylamine acetate. The maximum difference between the 3 melting curves in the $T_d$ was 3 C. The helical coil transition of the 27% G+C content was 13 C, for the 50% G+C duplex was 17 C and for the 83% G+C duplex was 19 C. This is an ideal profile for a hybotrope. In contrast the the narrow helical coil transition observed in Figure 20, a much wider HCT is observed with 500 mM n-ethylbutylamine acetate. Figure 21 is a graph showing the difference in $T_d$ between three duplexes, that vary in G+C content from 27% to 83%. The capture oligonucleotide is a 36-mer (DMO-GC36cap: 5'-hexylamine-GCA/GCC/TCG/CGG/AGG/CGG/ATG/ATC/GTC/ATT/ AGT/ ATT-3') and three complementary oligos which are labelled with the fluorochrome are DMO-83GC: 5'-Texas Red- CCG/ CCT/CCG/CGA/GGC/TGC-3'; DMO-50GC: 5'-Texas Red- AAT/GAC/GAT/CAT/CCG/CCT-3'; DMO-27GC: -Texas Red-AAT/ACT/AAT/GAC/GAT/CAT-3'. The temperature difference between any two $T_d$s at α = 0.5 is defined as the $\Delta T_d$. The percentage of single strand DNA (y-axis) is plotted versus temperature (°C; x-axis). The melting solution was 500 mM n-ethylbutylamine acetate. The maximum difference between the 3 melting curves in the $T_d$ was 1 C. The helical coil transition of the 27% G+C content was 22 C, for the 50% G+C duplex was 22 C and for the 83% G+C duplex was 26 C.

The ability of some of the G+C neutralizing buffer to act as hybrotropes is illustrated in Figure 22. Figure 22 is a graph showing the difference in $T_d$ between three duplexes, one that is perfectly based-paired and the other two that contains a mismatch or a deoxynebularine substitution. The temperature difference between any two $T_d$s at a = 0.5 is defined as the $\Delta T_d$. The percentage of single strand DNA (y-axis) is plotted versus temperature (°C; x-axis). DMO-2060: 5'-hexylamine-GTC/ATA/CTC/CTG/CTT/GCT/GAT/CCA/CAT/CTG-3' (oligonucleotide immobilized on the nylon bead.; DMO-2055: 5'-TEXAS RED-TGT/GGA/TCA/GCA/AGC/AGG/AGT/ATG-3' (perfect complement); DMO-2058; 5'-TEXAS RED- TGT/GGA/TCA/GGA/AGC/AGG/AGT/ATG-3' (mismatch complement); and DMO-2058-dN: 5'-TEXAS RED-TGT/GGA/TCA/G(deoxynebularine)A/AGC/AGG/AGT/ATG-3' (deoxynebularine mismatch complement). The melting solution was 1 M diisopropylamine acetate. The maximum difference between the 3 melting curves in the $T_d$ was 6 C. The helical coil transition (HCT) of the true mismatch was 14 C; the HCT for the deoxynebularine mismatch duplex was 14 C and the HCT for the perfectly based paired duplex was 16 C.

The same situation was observed for 1 M diisopropylamine acetate (Figure 22), 1 M n,n-dimethylcyclohexylamine acetate (Figure 23) and 1 M dicyclohexylamine acetate (Figure 24) and n,n-dimethylhexylamine acetate (Figure 25). Figure 23 is a graph showing the difference in $T_d$ between three duplexes, one that is perfectly based-paired and the other two that contains a mismatch or a deoxynebularine substitution. The temperature difference between any two $T_d$s at a = 0.5 is defined as the $\Delta T_d$. The percentage of single strand DNA (y-axis) is plotted versus temperature (°C; x-axis). DMO-2060: 5'-hexylamine-GTC/ATA/CTC/CTG/CTT/GCT/GAT/CCA/CAT/CTG-3' (oligonucleotide immobilized on the nylon bead.; DMO-2055: 5'-TEXAS RED-TGT/GGA/TCA/GCA/AGC/AGG/AGT/ATG-3' (perfect complement); DMO-2058: 5'-TEXAS RED- TGT/GGA/TCA/GGA/AGC/AGG/AGT/ATG-3' (mismatch complement); and DMO-2058-dN: 5'-TEXAS RED-TGT/GGA/TCA/G(deoxynebularine)A/AGC/AGG/AGT/ATG-3' (deoxynebularine mismatch complement). The melting solution was 1 M n,n-dicyclohexylamine acetate. The maximum difference between the 3 melting curves in the $T_d$ or $T_m$ is 4 C. The helical coil transition (HCT) of the true mismatch was 15 C: the HCT for the deoxynebularine mismatch duplex was 15 C and the HCT for the perfectly based paired duplex was 15 C.

Figure 24 is a graph showing the difference in $T_d$ between three duplexes, one that is perfectly based-paired and the other two that contains a mismatch or a deoxynebularine substitution. The temperature difference between any two $T_d$s at a = 0.5 is defined as the $\Delta T_d$. The percentage of single strand DNA (y-axis) is plotted versus temperature (°C; x-axis). DMO-2060: 5'-hexylamine-GTC/ATA/CTC/CTG/CTT/GCT/GAT/CCA/CAT/CTG-3' (oligonucleotide immobilized on the nylon bead.; DMO-2055: 5'-TEXAS RED-TGT/GGA/TCA/GCA/AGC/AGG/AGT/ATG-3' (perfect complement); DMO-2058; 5'-TEXAS RED- TGT/GGA/TCA/GGA/AGC/AGG/AGT/ATG-3' (mismatch complement); and DMO-2058-dN: 5'-TEXAS RED-TGT/GGA/TCA/G(deoxynebularine)A/AGC/AGG/AGT/ATG-3' (deoxynebularine mismatch complement). The melting solution was 1 M n,n-dicyclohexylamine acetate. The maximum difference between the 3 melting curves in the $T_d$ is 4 C. The helical coil transition (HCT) of the true mismatch was 17 C; the HCT for the deoxynebularine mismatch duplex was 17 C and the HCT for the perfectly based paired duplex was 15 C.

Figure 25 is a graph showing the difference in $T_d$ between three duplexes, one that is perfectly based-paired and the other two that contains a mismatch or a deoxynebularine substitution. The temperature difference between any two $T_d$s at α = 0.5 is defined as the $\Delta T_d$. The percentage of single strand DNA (y-axis) is plotted versus temperature (°C; x-axis). The melting solution was 100 mM n,n-dimethylhexylamine acetate. The maximum difference between the 3 melting curves in the $T_d$ is 9 C. The helical coil transition (HCT) of the true mismatch was 15 C; the HCT for the deoxynebularine mismatch duplex was 15 C and the HCT for the perfectly based paired duplex was 15 C.

It will be appreciated that, although specific embodiments of the invention have been described herein for purposes of illustration, various modifications may be made without departing from the spirit and scope of the invention. Accordingly, the invention is not limited except as by the appended claims.

## Claims

1. A composition comprising a nucleic acid, a salt, and an enzyme selected from polymerase and ligase, the salt comprising an anion and a cation, the anion selected from halogenated acetate, propionate and halogenated propionate, the cation selected from primary, secondary and tertiary ammonium comprising 1-36 carbon atoms.

2. A composition comprising a nucleic acid of 6-100 nucleotides, an enzyme selected from polymerase and ligase, and a salt, the salt comprising an anion and a cation, the anion selected from acetate, halogenated acetate, propionate, and halogenated propionate, the cation selected from primary, secondary and tertiary ammonium comprising 1-36 carbon atoms; the composition being at least one of non-flowing or free from organic solvent.

3. A composition comprising a nucleic acid, a salt, and an enzyme selected from polymerase and ligase, the nucleic acid immobilized on a solid support, the salt comprising an anion and a cation, the anion selected from acetate, halogenated acetate, propionate and halogenated propionate, the cation selected from primary, secondary and tertiary ammonium comprising 1-36 carbon atoms, and quaternary ammonium comprising 4-48 carbon atoms.

4. A composition according to any of claims 2 or 3 wherein the anion is acetate.

5. A composition according to any of claims 1-3 wherein the anion is trichloroacetate or trifluoroacetate.

6. A composition according to any of claims 1-5 wherein the cation is formed from atoms selected from 2-20 carbon atoms, 0-5 oxygen atoms and 1-5 nitrogen atoms.

7. A composition according to any of claims 1-5 wherein the cation has the structure $HN(R)_3$ wherein R is a $C_1$-$C_{12}$ hydrocarbyl and any two R groups may join together to form a cyclic structure with the nitrogen atom.

8. A composition according to any of claims 1-5 wherein the cation has the structure $N(H)_2(R)_2$ wherein R is a $C_1$-$C_{12}$ hydrocarbyl and the two R groups may join together to form a cyclic structure with the nitrogen atom.

9. A composition according to any of claims 7 or 8 wherein R is independently selected from the group consisting of $C_1$-$C_{12}$ alkyl, $C_3$-$C_{12}$ cycloalkyl and $C_7$-$C_{12}$ arylalkyl.

10. A composition according to any of claims 1-5 wherein the cation is selected from the group consisting of ethylbutylammonium, 1-methylimidizole, 1-methylpiperidine, 1-methylpyrrolidine, 3-methoxypropylamine, triethylamine, bis(2-methoxyethyl)amine, diallylamine, dibutylamine, diisobutylamine, N,N-dimethylaminobutane, N,N-dimethylclyclohexylamine, N,N-dimethylheptylamine, N,N-dimethylhexylamine, triethanolamine, 1-ethylpiperidine, dicyclohexylamine, diisopropylamine, dipropylamine, N,N-dimethylisopropylamine, N-ethylbutylamine, tetraethylamonium, tripropylamine, 2-methoxyethylamine, and N,N-dimethyloctylamine, and the anion is selected from the group consisting of trichloroacetate and trifluoroacetate.

11. A composition according to any of claims 1 and 3-10 wherein the nucleic acid comprises a chain of 6-100 nucleotides.

12. A composition according to any of claims 1, 2 and 4-11 wherein the nucleic acid is immobilized on a solid support.

13. A composition according to any of claims 1-12 wherein the nucleic acid forms an array on a solid support.

14. A composition according to claim 13 wherein the nucleic acid is in separated domains in an array of separated domains, where the number of domains in an array is selected from the ranges 10 to 50, 50 to 400, and 400 to 800.

15. A composition according to claim 14 wherein the domains are substantially circular with an average diameter of about 10 microns to 200 microns.

16. A composition according to any of claims 12-15 comprising a plurality of nucleic acid sequences.

17. A composition according to any of claims 3 and 12-16 wherein the solid support is selected from materials having a planar surface and comprising quartz, gold, nylon-6,6, nylon, polystyrene, glass, and silicon.

18. A composition according to any of claims 3 and 12-16 wherein the solid support is selected from a glass plate and a silicon wafer.

19. A composition according to any of claims 1-18 further comprising at least one of water, buffer, detergent or chelator.

20. A composition according to any of claims 1-19 wherein the salt is completely dissolved in water at a concentration of from 50mM to 6M at room temperature and/or the nucleic acid is present at a concentration of from $10^{-6}$ to $10^{-18}$ g/mL.

21. A composition according to any of claims 1-20 wherein the nucleic acid is DNA.

22. A salt selected from the group consisting of:

(a) an acetate salt of a cation of the formula $MN(CH_3)_2R_a$ wherein $R_a$ is a $C_4$-$C_7$hydrocarbyl;
(b) a halogenated acetate salt of a cation of the formula $HN(CH_3)_2R_b$ wherein $R_b$ is a $C_7$-$C_{12}$hydrocarbyl;
(c) acetate and halogenated acetate salts of a cation of the formula $H_2N(C_5$-$C_7cycloalkyl)R_c$ where $R_c$ is a $C_1$-$C_{12}$hydrocarbyl; and
(d) acetate and halogenated acetate salts of N-substituted piperidine, wherein the nitrogen atom of piperidine is substituted with $C_1$-$C_{12}$hydrocarbyl.

23. The salt of claim 23 wherein hydrocarbyl is selected from alkyl, alkenyl, alkynyl, cycloalkyl, cycloalkenyl, aryl, aralkyl, and alkylaryl.

24. A method of distinguishing between hybridization of a complementary nucleic acid target and a nucleic acid probe in which the probe and target are perfectly complementary and in which the probe and target have one or more base mismatches, comprising:

(a) mixing the nucleic acid target with the nucleic acid probe in a solution comprising a hybotrope;
(b) hybridizing at a discriminating temperature; and
(c) detecting probe hybridized to target,

thereby determining whether the nucleic acid probe and target are perfectly complementary or mismatched.

25. A method according to claim 24 wherein at least one of the nucleic acid target or nucleic acid probe is DNA.

26. A method according to claim 24 wherein at least one of the nucleic acid target or nucleic acid probe is RNA.

27. A method of increasing discrimination in a nucleic acid synthesis procedure, comprising:

(a) mixing a single-stranded nucleic acid target with an oligonucleotide primer in a solution comprising a hybotrope and a polymerase;
(b) annealing the primer to the target at a discriminating temperature; and
(c) synthesizing a complementary strand to the target to form a duplex.

28. A method according to claim 27 wherein the steps of (a), (b), and (c) are repeated multiple times.

29. A method according to any of claims 24-28 wherein the nucleic acid target is produced through a polymerase chain reaction.

30. A method according to any of claims 24-29 wherein the nucleic acid probe or nucleic acid primer is labeled with a radioactive molecule, fluorescent molecule, mass-spectrometry tag or enzyme.

31. A method according to any of claims 24-29 wherein the nucleic acid probe or nucleic acid primer is from 6 to 40 bases.

32. A method according to any of claims 24-31 wherein the nucleic acid target is from 6 to 40 bases.

33. A method according to any of claims 24-32 wherein the nucleic acid target is affixed to a solid support.

**34.** A method according to any of claims 24-33 wherein the hybotrope is selected from the group consisting of LiTCA, RbTCA, GuSCN, NaSCN, $NaClO_4$, KI, TMATCA TEATCA, TMATBA, TMTCA, TMTBA, TBATCA or TBATBA.

**35.** A method according to any of claims 24-33 wherein the hybotrope is a salt comprising an anion and a cation, wherein the anion is selected from acetate halogenated acetate, propionate and halogenated propionate, and the cation is selected from primary, secondary and tertiary ammonium comprising 1-36 carbon atoms, and quaternary ammonium comprising 4-48 carbon atoms.

**36.** A method according to any of claims 24-35 wherein the hybotrope is present at a molarity of from about 0.5 M to about 6 M.

Figure 1

Figure 2

Figure 3

Figure 4

Figure 5

Figure 6

Figure 7

Figure 8

Figure 9

Figure 10

Figure 11

Figure 12

| m | H17 | H14 | H11 | AB1 | dN1 | dN2 | dN3 | dN6 |

# Figure 13

Figure 14

Figure 15

Figure 16

Figure 17

Figure 18

Figure 19

Figure 20

Figure 21

Figure 22

Figure 23

Figure 24

Figure 25

Figure 26